(19) Europäisches Patentamt
European Patent Office
Office européen des brevets

(11) EP 1 651 653 B1

(12) **EUROPÄISCHE PATENTSCHRIFT**

(45) Veröffentlichungstag und Bekanntmachung des Hinweises auf die Patenterteilung:
**23.11.2011 Patentblatt 2011/47**

(21) Anmeldenummer: **04740985.9**

(22) Anmeldetag: **14.07.2004**

(51) Int Cl.:
***C07D 513/04*** *(2006.01)* ***A61K 31/415*** *(2006.01)*

(86) Internationale Anmeldenummer:
**PCT/EP2004/007767**

(87) Internationale Veröffentlichungsnummer:
**WO 2005/012312 (10.02.2005 Gazette 2005/06)**

(54) **NEUE CYANOTHIAZOLIDE, VERFAHREN ZU IHRER HERSTELLUNG UND IHRE VERWENDUNG ALS ARZNEIMITTEL**

NOVEL CYANO THIAZOLIDES, METHODS FOR THE PRODUCTION THEREOF, AND USE THEREOF AS A MEDICAMENT

NOUVELLES CYANOTHIAZOLIDES, LEUR PROCEDE DE PRODUCTION ET LEUR UTILISATION COMME MEDICAMENT

(84) Benannte Vertragsstaaten:
**AT BE BG CH CY CZ DE DK EE ES FI FR GB GR HU IE IT LI LU MC NL PL PT RO SE SI SK TR**

(30) Priorität: **25.07.2003 DE 10333935**

(43) Veröffentlichungstag der Anmeldung:
**03.05.2006 Patentblatt 2006/18**

(73) Patentinhaber: **Sanofi-Aventis Deutschland GmbH**
**65929 Frankfurt am Main (DE)**

(72) Erfinder:
- **WAGNER, Holger**
  **88400 Biberach an der Riss (DE)**
- **SCHOENAFINGER, Karl**
  **63755 Alzenau (DE)**
- **JAEHNE, Gerhard**
  **65929 Frankfurt (DE)**
- **GAUL, Holger**
  **65594 Runkel (DE)**
- **BUNING, Christian**
  **53175 Bonn (DE)**
- **TSCHANK, Georg**
  **55270 Essenheim (DE)**
- **WERNER, Ulrich**
  **56357 Miehlen (DE)**

(56) Entgegenhaltungen:
**WO-A-99/31507**

- **AICHER, THOMAS D.: J. MED. CHEM., Bd. 41, 1998, Seiten 4556-4566, XP002306475**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 651 653 B1

Printed by Jouve, 75001 PARIS (FR)

## Beschreibung

Die Erfindung betrifft substituierte Cyanothiazolide sowie deren physiologisch verträglichen Salze.

Es sind bereits strukturähnliche Verbindungen im Stand der Technik beschrieben (WO 99/31507) sowie deren Verwendung in einem Screening.

Der Erfindung lag die Aufgabe zugrunde, Verbindungen zur Verfügung zu stellen, die eine therapeutisch verwertbare, Blutzucker senkende Wirkung entfalten und insbesondere zur Behandlung von Diabetes geeignet sind.

Die Erfindung betrifft daher Verbindungen der Formel I,

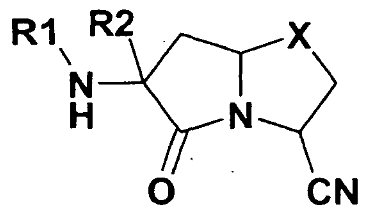

worin bedeuten

R1 H, $(C_1$-$C_{10})$-Alkyl, $(C_3$-$C_{10})$-Cycloalkyl, $(C_2$-$C_6)$-Alkenyl, $(C_2$-$C_6)$-Alkinyl, $(C_6$-$C_{10})$-Aryl, Heterozyklyl, wobei die Alkyl-, Cycloalkyl-, Alkenyl-, Alkinyl-, Aryl- und Heterozyklyl Reste ein- oder mehrfach substituiert sein können mit F, Cl, Br, I, CN, $NO_2$, OH, $-CF_3$, $(C_1$-$C_6)$-Alkyl, $(C_2$-$C_6)$-Alkenyl, $(C_2$-$C_6)$-Alkinyl, OR3, NR3R4, COR3, $CO_2R3$, CONR3R4, $(C_1$-$C_6)$-Alkylen-OR3, $(C_1$-$C_6)$-Alkylen-NR3R4, $(C_1$-$C_6)$-Alkylen-SR3, Alkylen-SOR3, Alkylen-$SO_2R3$, $(C_1$-$C_6)$-Alkylen-COR3, $(C_1$-$C_6)$-Alkylen-COOR3, $(C_1$-$C_6)$-Alkylen-CONR3R4, $(C_1$-$C_6)$-Alkylen-O-P(O)$(OR3)_2$, SR3, SOR3, $SO_2NR3R4$, $SO_2R3$, $(C_1$-$C_6)$-Alkylen- $(C_3$-$C_{10})$-Cycloalkyl, $(C_1$-$C_6)$-Alkylen-$(C_6$-$C_{10})$-Aryl, $(C_1$-$C_6)$-Alkylen-Heterozyklyl, $(C_3$-$C_{10})$-Cycloalkyl, $(C_6$-$C_{10})$-Aryl, das mit F, Cl, Br, I, CN, $NO_2$, OH, $-CF_3$, $(C_1$-$C_6)$-Alkyl, $(C_2$-$C_6)$-Alkenyl, $(C_2$-$C_6)$-Alkinyl, OR3, NR3R4, COR3, $CO_2R3$, CONR3R4 ein oder mehrfach substituiert sein kann, oder Heterozyklyl, das mit F, Cl, Br, I, CN, $NO_2$, OH, $-CF_3$, $(C_1$-$C_6)$-Alkyl, OR3, NR3R4, COR3, $CO_2R3$, CONR3R4 ein oder mehrfach substituiert sein kann;

R2 H, $(C_1$-$C_{10})$-Alkyl, $(C_3$-$C_{10})$-Cycloalkyl, $(C_2$-$C_6)$-Alkenyl, $(C_2$-$C_6)$-Alkinyl, $(C_6$-$C_{10})$-Aryl, Heterozyklyl, COR3, COOR3, CONR3R4, CN, wobei die Alkyl-, Cycloalkyl-, Alkenyl-, Alkinyl-, Aryl- und Heterozyklyl Reste ein- oder mehrfach substituiert sein können mit F, Cl, Br, I, CN, $NO_2$, SH, $SF_5$, OH, $(C_1$-$C_6)$-Alkyl, $-CF_3$, $(C_2$-$C_6)$-Alkenyl, $(C_2$-$C_6)$-Alkinyl, OR3, OP(O)$(OR3)_2$, NR3R4, NR3CONR3R4, COR3, OCOR3, OCOOR3, COOR3, CONR3R4, OCONR3R4, $(C_1$-$C_6)$-Alkylen-OR3, $(C_1$-$C_6)$-Alkylen-NR3R4, $(C_1$-$C_6)$-Alkylen-NR3$SO_2R4$, $(C_1$-$C_6)$-Alkylen-SR3, Alkylen-S(O)R3, Alkylen-S$(O)_2R3$, Alkylen-S$(O)_2NR3R4$, $(C_1$-$C_6)$-Alkylen-COR3, $(C_1$-$C_6)$-Alkylen-COOR3, $(C_1$-$C_6)$-Alkylen-CONR3R4, SR3, SOR3, $SO_2R3$, $SO_2NR3R4$, NR3$SO_2R4$, $(C_1$-$C_6)$-Alkylen-$(C_3$-$C_{10})$-Cycloalkyl, $(C_1$-$C_6)$-Alkylen-$(C_6$-$C_{10})$-Aryl, $(C_1$-$C_6)$-Alkylen-Heterozyklyl, $(C_3$-$C_{10})$-Cycloalkyl, $(C_6$-$C_{10})$-Aryl oder Heterozyklyl;

R3, R4 unabhängig voneinander H, $(C_1$-$C_6)$-Alkyl, $-CF_3$, $(C_3$-$C_{10})$-Cycloalkyl, $(C_6$-$C_{10})$-Aryl, Heterozyklyl, $(C_1$-$C_6)$-Alkylen-CONR5R6, CONR5R6, $(C_1$-$C_6)$-Alkylen-COOR5, COOR5, COR5, $(C_1$-$C_6)$-Alkylen-COR5, $(C_1$-$C_6)$-Alkylen-OR5, $(C_1$-$C_6)$-Alkylen-NR5R6, $(C_1$-$C_6)$-Alkylen-SR5, $(C_1$-$C_6)$-Alkylen-S(O)R5, $(C_1$-$C_6)$-Alkylen-S$(O)_2R5$, S(O)R5, S$(O)_2R5$, $(C_1$-$C_4)$-Alkylen-$(C_6$-$C_{10})$-Aryl oder $(C_1$-$C_4)$-Alkylen-Heterozyklyl;

R5, R6 unabhängig voneinander H, $(C_1$-$C_6)$-Alkyl, $(C_1$-$C_6)$-Alkylen-$(C_6$-$C_{10})$-Aryl, -$(C_6$-$C_{10})$-Aryl, Heterozyklyl, $(C_1$-$C_6)$-Alkylen-Heterozyklyl;

X S, SO, $SO_2$;

sowie deren physiologisch verträglichen Salze.

Bevorzugt sind Verbindungen der Formel I, worin ein oder mehrere Reste bedeuten

R1 H, $(C_1$-$C_{10})$-Alkyl, $(C_3$-$C_{10})$-Cycloalkyl, $(C_2$-$C_6)$-Alkenyl, $(C_2$-$C_6)$-Alkinyl, $(C_6$-$C_{10})$-Aryl, wobei die Alkyl-, Cycloalkyl-, Alkenyl-, Alkinyl- und Aryl- Reste ein- oder mehrfach substituiert sein können mit F, Cl, Br, I, CN, $NO_2$, OH, $-CF_3$, $(C_1$-$C_6)$-Alkyl, $(C_2$-$C_6)$-Alkenyl, $(C_2$-$C_6)$-Alkinyl, OR3, NR3R4, COR3, $CO_2R3$, CONR3R4, $(C_1$-$C_6)$-Alkylen-OR3, $(C_1$-$C_6)$-Alkylen-NR3R4, $(C_1$-$C_6)$-Alkylen-SR3, Alkylen-SOR3, Alkylen-$SO_2R3$, $(C_1$-$C_6)$-Alkylen-COR3, $(C_1$-$C_6)$-Alkylen-$CO_2R3$, $(C_1$-$C_6)$-Alkylen-CONR3R4, $(C_1$-$C_6)$-Alkylen-O-P(O)

$(OR3)_2$, SR3, SOR3, $SO_2NR3R4$, $SO_2R3$, $(C_1-C_6)$-Alkylen-$(C_3-C_{10})$-Cycloalkyl, $(C_1-C_6)$-Alkylen-$(C_6-C_{10})$-Aryl, $(C_1-C_6)$-Alkylen-Heterozyklyl, $(C_3-C_{10})$-Cycloalkyl oder $(C_6-C_{10})$-Aryl, das mit F, Cl, Br, I, CN, $NO_2$, OH, $-CF_3$, $(C_1-C_6)$-Alkyl, $(C_2-C_6)$-Alkenyl, $(C_2-C_6)$-Alkinyl, OR3, NR3R4, COR3, $CO_2R3$, CONR3R4 ein oder mehrfach substituiert sein kann;

R2 H, $(C_1-C_{10})$-Alkyl, $(C_3-C_{10})$-Cycloalkyl, $(C_2-C_6)$-Alkenyl, $(C_2-C_6)$-Alkinyl, $(C_6-C_{10})$-Aryl, Heterozyklyl, COR3, CO2R3, CONR3R4, CN, wobei die Alkyl-, Cycloalkyl-, Alkenyl-, Alkinyl-, Aryl- und Heterozyklyl Reste ein- oder mehrfach substituiert sein können mit F, Cl, Br, I, CN, $NO_2$, SH, $SF_5$, OH, $(C_1-C_6)$-Alkyl, $-CF_3$, $(C_2-C_6)$-Alkenyl, $(C_2-C_6)$-Alkinyl, OR3, $OP(O)(OR3)_2$, NR3R4, NR3CONR3R4, COR3, OCOR3, $CO_2R3$, CONR3R4, OCONR3R4, $(C_1-C_6)$-Alkylen-OR3, $(C_1-C_6)$-Alkylen-NR3R4, $(C_1-C_6)$-Alkylen-$NR3SO_2R4$, $(C_1-C_6)$-Alkylen-SR3, Alkylen-SOR3, Alkylen-$SO_2R3$, Alkylen-$SO_2NR3R4$, $(C_1-C_6)$-Alkylen-COR3, $(C_1-C_6)$-Alkylen-$CO_2R3$, $(C_1-C_6)$-Alkylen-CONR3R4, SR3, SOR3, $SO_2R3$, $SO_2NR3R4$, $NR3SO_2R4$, $(C_1-C_6)$-Alkylen-$(C_3-C_{10})$-Cycloalkyl, $(C_1-C_6)$-Alkylen-$(C_6-C_{10})$-A-ryl, $(C_1-C_6)$-Alkylen-Heterozyklyl, $(C_3-C_{10})$-Cycloalkyl, $(C_6-C_{10})$-Aryl, das mit F, Cl, Br, I, CN, $NO_2$, OH, $-CF_3$, $(C_1-C_6)$-Alkyl, $(C_2-C_6)$-Alkenyl, $(C_2-C_6)$-Alkinyl, OR3, NR3R4, COR3, $CO_2R3$, CONR3R4 ein oder mehrfach substituiert sein kann, oder Heterozyklyl, das mit F, Cl, Br, I, CN, $NO_2$, OH, $-CF_3$, $(C_1-C_6)$-Alkyl, OR3, NR3R4, COR3, $CO_2R3$, CONR3R4 ein oder mehrfach substituiert sein kann;

R3, R4 unabhängig voneinander H, $(C_1-C_6)$-Alkyl, $-CF_3$, $(C_3-C_{10})$-Cycloalkyl, $(C_6-C_{10})$-Aryl, Heterozyklyl, $(C_1-C_6)$-Alkylen-CONR5R6, $(C_1-C_6)$-Alkylen-$CO_2R5$, $(C_1-C_6)$-Alkylen-COR5, $(C_1-C_6)$-Alkylen-OR5, $(C_1-C_6)$-Alkylen-NR5R6, $(C_1-C_6)$-Alkylen-SR5, $(C_1-C_6)$-Alkylen-SOR5, $(C_1-C_6)$-Alkylen-$SO_2R5$, $(C_1-C_4)$-Alkylen-$(C_6-C_{10})$-Aryl oder $(C_1-C_4)$-Alkylen-Heterozyklyl;

R5, R6 unabhängig voneinander H, $(C_1-C_6)$-Alkyl, $(C_3-C_{10})$-Cycloalkyl, $(C_1-C_6)$-Alkylen-$(C_6-C_{10})$-Aryl, $-(C_6-C_{10})$-Aryl, Heterozyklyl, $(C_1-C_6)$-Alkylen-$(C_3-C_{10})$-Heterozyklyl;

X S;

sowie deren physiologisch verträglichen Salze.

Weiterhin bevorzugt sind Verbindungen der Formel I, worin ein oder mehrere Reste bedeuten

R1 H;

R2 H, $(C_1-C_{10})$-Alkyl, $(C_3-C_{10})$-Cycloalkyl, $(C_2-C_6)$-Alkenyl, $(C_2-C_6)$-Alkinyl, $(C_6-C_{10})$-Aryl, Heterozyklyl, COR3, $CO_2R3$, CONR3R4, CN, wobei die Alkyl-, Cycloalkyl-, Alkenyl-, Alkinyl-, Aryl- und Heterozyklyl Reste ein- oder mehrfach substituiert sein können mit F, Cl, Br, I, CN, $NO_2$, SH, $SF_5$, OH, $(C_1-C_6)$-Alkyl, $-CF_3$, $(C_2-C_6)$-Alkenyl, $(C_2-C_6)$-Alkinyl, OR3, OP(O)(OR3)2, NR3R4, NR3CONR3R4, COR3, OCOR3, $CO_2R3$, CONR3R4, OCONR3R4, $(C_1-C_6)$-Alkylen-OR3, $(C_1-C_6)$-Alkylen-NR3R4, $(C_1-C_6)$-Alkylen-$NR3SO_2R4$, $(C_1-C_6)$-Alkylen-SR3, Alkylen-SOR3, Alkylen-$SO_2R3$, Alkylen-$S(O)_2NR3R4$, $(C_1-C_6)$-Alkylen-COR3, $(C_1-C_6)$-Alkylen-$CO_2R3$, $(C_1-C_6)$-Alkylen-CONR3R4, SR3, SOR3, $SO_2R3$, $SO_2NR3R4$, $NR3SO_2R4$, $(C_1-C_6)$-Alkylen-$(C_3-C_{10})$- Cycloalkyl, $(C_1-C_6)$-Alkylen-$(C_6-C_{10})$-Aryl, $(C_1-C_6)$-Alkylen-Heterozyklyl, $(C_3-C_{10})$-Cycloalkyl, $(C_6-C_{10})$-Aryl, das mit F, Cl, Br, I, CN, $NO_2$, OH, $-CF_3$, $(C_1-C_6)$-Alkyl, $(C_2-C_6)$-Alkenyl, $(C_2-C_6)$-Alkinyl, OR3, NR3R4, COR3, $CO_2R3$, CONR3R4 ein oder mehrfach substituiert-sein-kann, oder Heterozyklyl, das mit F, Cl, Br, I, CN, $NO_2$, OH, $-CF_3$, $(C_1-C_6)$-Alkyl, OR3, NR3R4, COR3, $CO_2R3$, CONR3R4 ein oder mehrfach substituiert sein kann;

R3, R4 unabhängig voneinander H, $(C_1-C_6)$-Alkyl, $-CF_3$, $(C_3-C_{10})$-Cycloalkyl, $(C_6-C_{10})$-Aryl, Heterozyklyl, $(C_1-C_6)$-Alkylen-CONR5R6, $(C_1-C_6)$-Alkylen-$CO_2R5$, $(C_1-C_6)$-Alkylen-COR5, $(C_1-C_6)$-Alkylen-OR5, $(C_1-C_6)$-Alkylen-NR5R6, $(C_1)-C_6)$-Alkylen-SR5, $(C_1-C_6)$-Alkylen-SOR5, $(C_1-C_6)$-Alkylen-$SO_2R5$,$(C_1-C_4)$-Alkylen-$(C_6-C_{10})$-Aryl oder $(C_1-C_4)$-Alkylen-Heterozyklyl;

R5, R6 unabhängig voneinander H, $(C_1-C_6)$-Alkyl, $(C_3-C_{10})$-Cycloalkyl, $(C_1-C_6)$-Alkylen-$(C_6-C_{10})$-Aryl, $-(C_6-C_{10})$-Aryl, Heterozyklyl, $(C_1-C_6)$-Alkylen-$(C_3-C_{10})$-Heterozyklyl;

X S;

sowie deren physiologisch verträglichen Salze.

Weiterhin bevorzugt sind Verbindungen der Formel I, worin ein oder mehrere Reste bedeuten

R1 H;

R2 H, $(C_1-C_{10})$-Alkyl, $(C_3-C_{10})$-Cycloalkyl, $(C_2-C_6)$-Alkenyl, $(C_2-C_6)$-Alkinyl, $(C_6-C_{10})$-Aryl, ein Pyrrolidino-, Piperidino-, Hexamethylenimino-, Morpholino-, Piperazino-, Thiomorpholino- oder Homopiperazino-Rest; wobei die Alkyl-, Cycloalkyl-, Alkenyl-, Alkinyl-, Pyrrolidino-, Piperidino-, Hexamethylenimino-, Morpholino-, Piperazino-, Thiomorpholino-, Homopiperazino- und Aryl-Reste ) ein-oder mehrfach substituiert sein können mit F, Cl, Br, CN, $SF_5$, OH, $(C_1-C_6)$-Alkyl, $-CF_3$, $(C_2-C_6)$-Alkenyl, OR3, NR3R4, NR3CONR3R4, COR3, OCOR3, $CO_2R3$, CONR3R4, OCONR3R4, $(C_1-C_6)$-Alkylen-OR3, $(C_1-C_6)$-Alkylen-NR3R4, $(C_1-C_6)$-Alkylen-$NR3SO_2R4$, $(C_1-C_6)$-Alkylen-SR3, Alkylen-S(O)R3, Alkylen-$S(O)_2R3$, Alkylen-$S(O)_2NR3R4$, $(C_1-C_6)$-Alkylen-COR3, $(C_1-C_6)$-Alkylen-$CO_2R3$, $(C_1-C_6)$-Alkylen-CONR3R4, SR3, SOR3, $SO_2R3$, $SO_2NR3R4$, $NR3SO_2R4$, $(C_1-C_6)$-Alkylen-$(C_3-C_{10})$-Cycloalkyl, $(C_1-C_6)$-Alkylen-$(C_6-C_{10})$-Aryl, $(C_1-C_6)$-Alkylen-Heterozyklyl, $(C_3-C_{10})$-Cycloalkyl, $(C_6-C_{10})$-Aryl, das mit F, Cl, Br, I, CN, OH, $-CF_3$, $(C_1-C_6)$-Alkyl, OR3, NR3R4, COR3, CO2R3, CONR3R4 ein oder mehrfach substituiert sein kann, oder Heterozyklyl, das mit F, Cl, Br, CN, $NO_2$, OH, $-CF_3$, $(C_1-C_6)$-Alkyl, OR3, NR3R4, COR3, CO2R3, CONR3R4 ein oder mehrfach substituiert sein kann;

R3, R4 unabhängig voneinander H, $(C_1-C_6)$-Alkyl, $-CF_3$, $(C_3-C_{10})$-Cycloalkyl, $(C_6-C_{10})$-Aryl, Heterozyklyl, $(C_1-C_6)$-Alkylen-CONR5R6, $(C_1-C_6)$-Alkylen-COOR5, $(C_1-C_6)$-Alkylen-COR5, $(C_1-C_6)$-Alkylen-OR5, $(C_1-C_6)$-Alkylen-NR5R6, $(C_1-C_6)$-Alkylen-SR5, $(C_1-C_6)$-Alkylen-S(O)R5, $(C_1-C_6)$-Alkylen-$S(O)_2R5$, $(C_1-C_4)$-Alkylen-$(C_6-C_{10})$-Aryl oder $(C_1-C_4)$-Alkylen-Heterozyklyl;

R5, R6 unabhängig voneinander H, $(C_1-C_6)$-Alkyl, $(C_3-C_{10})$-Cycloalkyl, $(C_1-C_6)$-Alkylen-$(C_6-C_{10})$-Aryl, $-(C_6-C_{10})$-Aryl, Heterozyklyl, $(C_1-C_6)$-Alkylen-$(C_3-C_{10})$-Heterozyklyl;

X S;

sowie deren physiologisch verträglichen Salze.

Weiterhin bevorzugt sind Verbindungen der Formel I, worin ein oder mehrere Reste bedeuten

R1 H;

R2 $(C_1-C_{10})$-Alkyl, $(C_3-C_{10})$-Cycloalkyl, Phenyl, $(C_1-C_6)$-Alkylen-Phenyl, ein Pyrrolidino-, Piperidino-, Hexamethylenimino-, Morpholino-, Piperazino-, Thiomorpholino- oder Homopiperazino-Rest;

X S;

sowie deren physiologisch verträglichen Salze.

Die Erfindung betrifft Verbindungen der Formel I in Form all ihrer stereoisomeren Formen wie Racemate, racemischen und enantiomeren Mischungen und reinen Enantiomeren und Diastereomeren.

Besonders bevorzugt sind Verbindungen der Formel I,

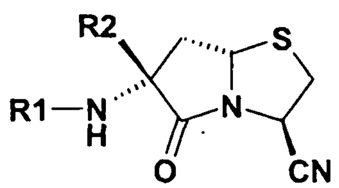

Ia

die die angegebene diastereomere Form Ia aufweisen.

Können Reste oder Substituenten mehrfach in den Verbindungen der Formel I auftreten, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Salze von Trometamol

(2-Amino-2-hydroximethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Unter einem Alkylrest wird eine geradkettige oder verzweigte Kohlenwasserstoffkette mit einem oder mehreren Kohlenstoffen verstanden, wie z.B. Methyl, Ethyl, Propyl, Butyl, Hexyl, Isopropyl, Isobutyl, Neopentyl, tert.-Butyl, Hexyl.

Die Alkylreste können ein oder mehrfach mit geeigneten Gruppen substituiert sein, wie z.B.: F, Cl, Br, I, $CF_3$, $NO_2$, $N_3$, CN, COOH, COO($C_1$-$C_6$)Alkyl, $CONH_2$, CONH($C_1$-$C_6$)Alkyl, CON[($C_1$-$C_6$)Alkyl]$_2$, Cycloalkyl, ($C_2$-$C_6$)-Alkenyl, ($C_2$-$C_6$)-Alkinyl, Aryl, Heterozyklyl, O-($C_1$-$C_6$)-Alkyl, O-COO-($C_1$-$C_6$)-Alkyl, O-CO-($C_1$-$C_6$)-Alkyl, O-CO-($C_1$-$C_6$)-Aryl, O-CO-($C_1$-$C_6$)-Heterozyklyl, $PO_3H_2$, P(O)(OAlkyl)$_2$, ($C_1$-$C_6$)-Alkylen-P(O)(OAlkyl)$_2$, O-P(O)(OH)$_2$, O-P(O)(OAlkyl)$_2$, $SO_3H$, $SO_2$-$NH_2$, $SO_2$NH($C_1$-$C_6$)-Alkyl, $SO_2$N[($C_1$-$C_6$)-Alkyl]$_2$, S-($C_1$-$C_6$)-Alkyl, S-($CH_2$)$_n$-Aryl, S-($CH_2$)$_n$-Heterozyklyl, SO-($C_1$-$C_6$)-Alkyl, SO-($CH_2$)$_n$-Aryl, SO-($CH_2$)$_n$-Heterozyklyl, $SO_2$(C)-$C_6$)-Alkyl, $SO_2$-($CH_2$)$_n$-Aryl, $SO_2$-($CH_2$)$_n$-Heterozyklyl , $SO_2$-NH($CH_2$)$_n$-Aryl, $SO_2$-NH($CH_2$)$_n$-Heterozyklyl, $SO_2$-N[(($C_1$-$C_6$)-Alkyl)($CH_2$)$_n$-Aryl], $SO_2$-N[(($C_1$-$C_6$)-Alkyl)($CH_2$)$_n$-Heterozyklyl], $SO_2$-N(($CH_2$)$_n$-Aryl)$_2$, , $SO_2$-N(($CH_2$)$_n$-(Heterozyklyl))$_2$, wobei n = 0 - 6 sein kann und der Aryl- oder Heterozyklyl- Rest bis zu dreifach mit F, Cl, Br, OH, $CF_3$, $SF_5$, $NO_2$, CN, $OCF_3$, O-($C_1$-$C_6$)-Alkyl, ($C_1$-$C_6$)-Alkyl, $NH_2$ substituiert sein kann;

C(NH)($NH_2$), $NH_2$, NH-($C_1$-$C_6$)-Alkyl, N(($C_1$-$C_6$)-Alkyl)$_2$, NH($C_1$-$C_7$)-Acyl, NH-CO-($C_1$-$C_6$)-Alkyl, NH-COO-($C_1$-$C_6$)-Alkyl, NH-CO-Aryl, NH-CO-Heterozyklyl, NH-COO-Aryl, NH-COO-Heterozyklyl, NH-CO-NH-($C_1$-$C_6$)-Alkyl), NH-CO-NH-Aryl, NH-CO-NH-Heterozyklyl, N[($C_1$-$C_6$)-Alkyl]-CO-($C_1$-$C_6$)-Alkyl, N[($C_1$-$C_6$)-Alkyl]-COO-($C_1$-$C_6$)-Alkyl, N[($C_1$-$C_6$)-Alkyl]-CO-Aryl, N[($C_1$-$C_6$)-Alkyl]-CO-Heterozyklyl, N[($C_1$-$C_6$)-Alkyl]-COO-Aryl, N[($C_1$-$C_6$)-Alkyl]-COO-Heterozyklyl, N[($C_1$-$C_6$)-Alkyl]-CO-NH-($C_1$-$C_6$)-Alkyl), N[($C_1$-$C_6$)-Alkyl]-CO-NH-Aryl, N[($C_1$-$C_6$)-Alkyl]-CO-NH-Heterozyklyl,N[($C_1$-$C_6$)-Alkyl]-CO-N(($C_1$-$C_6$)-Alkyl)$_2$,N[($C_1$-$C_6$)-Alkyl]-CO-N(($C_1$-$C_6$)-Alkyl)-Aryl,N[($C_1$-$C_6$)-Alkyl]-CO-N(($C_1$-$C_6$)-Alkyl)-Heterozyklyl, N[($C_1$-$C_6$)-Alkyl]-CO-N(Aryl)$_2$, N[($C_1$-$C_6$)-Alkyl]-CO-N(Heterozyklyl)$_2$, N(Aryl)-CO-($C_1$-$C_6$)-Alkyl, N (Heterozyklyl)-CO-($C_1$-$C_6$)-Alkyl, N(Aryl)-COO-($C_1$-$C_6$)-Alkyl, N(Heterozyklyl)-COO-($C_1$-$C_6$)-Alkyl, N(Aryl)-CO-Aryl, N (Heterozyklyl)-CO-Aryl, N(Aryl)-COO-Aryl, N(Heterozyklyl)-COO-Aryl, N(Aryl)-CO-NH-($C_1$-$C_6$)-Alkyl, N(Heterozyklyl)-CO-NH-($C_1$-$C_6$)-Alkyl, N(Aryl)-CO-NH-Aryl, N(Heterozyklyl)-CO-NH-Aryl, N(Aryl)-CO-N(($C_1$-$C_6$)-Alkyl)$_2$, N(Heterozyklyl)-CO-N(($C_1$-$C_6$)-Alkyl)$_2$, N(Aryl)-CO-N[($C_1$-$C_6$)-Alkyl]-Aryl, N(Heterozyklyl)-CO-N[($C_1$-$C_6$)-Alkyl]-Aryl, N(Aryl)-CO-N(Aryl)$_2$, N(Heterozyklyl)-CO-N(Aryl)$_2$, Aryl, O-($CH_2$)$_n$-Aryl, O-($CH_2$)$_n$-Heterozyklyl, wobei n = 0 - 6 sein kann, wobei der Aryl- oder Heterozyklyl- Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SF_5$, $SO_2$-$CH_3$, COOH, COO-($C_1$-$C_6$)-Alkyl, $CONH_2$.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl.

Die Alkenylreste können ein oder mehrfach mit geeigneten Gruppen substituiert sein, wie z.B.: F, Cl, Br, I, $CF_3$, $NO_2$, $N_3$, CN, COOH, COO($C_1$-$C_6$)Alkyl, $CONH_2$, CONH($C_1$-$C_6$)Alkyl, CON[($C_1$-$C_6$)Alkyl]$_2$, Cycloalkyl, ($C_1$-$C_{10}$)-Alkyl, ($C_2$-$C_6$)-Alkinyl, Aryl, Heterozyklyl, O-($C_1$-$C_6$)-Alkyl O-CO-($C_1$-$C_6$)-Alkyl, O-CO-($C_1$-$C_6$)-Aryl, O-CO-($C_1$-$C_6$)-Heterozyklyl,;

$PO_3H_2$, P(O)(OAlkyl)$_2$, ($C_1$-$C_6$)-Alkylen-P(O)(OAlkyl)$_2$, O-P(O)(OH)$_2$, O-P(O)(OAlkyl)$_2$, $SO_3H$, $SO_2$-$NH_2$, $SO_2$NH($C_1$-$C_6$)-Alkyl, $SO_2$N[($C_1$-$C_6$)-Alkyl]$_2$, S-($C_1$-$C_6$)-Alkyl, S-($CH_2$)$_n$-Aryl, S-($CH_2$)$_n$-Heterozyklyl, SO-($C_1$-$C_6$)-Alkyl, SO-($CH_2$)$_n$-Aryl, SO-($CH_2$)$_n$-Heterozyklyl, $SO_2$-($C_1$-$C_6$)-Alkyl, $SO_2$-($CH_2$)$_n$-Aryl, $SO_2$-($CH_2$)$_n$-Heterozyklyl, $SO_2$-NH ($CH_2$)$_n$-Aryl, $SO_2$-NH($CH_2$)$_n$-Heterozyklyl, $SO_2$-N(($C_1$-$C_6$)-Alkyl)($CH_2$)$_n$-Aryl, $SO_2$-N(($C_1$-$C_6$)-Alkyl)($CH_2$)$_n$-Heterozyklyl, $SO_2$-N(($CH_2$)$_n$-Aryl)$_2$, , $SO_2$-N(($CH_2$)$_n$-(Heterozyklyl)$_2$ wobei n = 0 - 6 sein kann und der Arylrest oder heterozyklische Rest bis zu zweifach mit F, Cl, Br, OH, $CF_3$, $SF_5$, $NO_2$, CN, $OCF_3$, O-($C_1$-$C_6$)-Alkyl, ($C_1$-$C_6$)-Alkyl, $NH_2$ substituiert sein kann;

C(NH)($NH_2$), $NH_2$, NH-($C_1$-$C_6$)-Alkyl, N(($C_1$-$C_6$)-Alkyl)$_2$, NH($C_1$-$C_7$)-Acyl, NH-CO-($C_1$-$C_6$)-Alkyl, NH-COO-($C_1$-$C_6$)-Alkyl, NH-CO-Aryl, NH-CO-Heterozyklyl, NH-COO-Aryl, NH-COO-Heterozyklyl, NH-CO-NH-($C_1$-$C_6$)-Alkyl), NH-CO-NH-Aryl, NH-CO-NH-Heterozyklyl, N[($C_1$-$C_6$)-Alkyl]-CO-($C_1$-$C_6$)-Alkyl, N[($C_1$-$C_6$)-Alkyl]-COO-($C_1$-$C_6$)-Alkyl, N[($C_1$-$C_6$)-Al-

kyl]-CO-Aryl, N[($C_1$-$C_6$)-Alkyl]-CO-Heterozyklyl, N[($C_1$-$C_6$)-Alkyl]-COO-Aryl, N[($C_1$-$C_6$)-Alkyl]-COO-Heterozyklyl, N[($C_1$-$C_6$)-Alkyl]-CO-NH-($C_1$-$C_6$)-Alkyl), N[($C_1$-$C_6$)-Alkyl]-CO-NH-Aryl, N[($C_1$-$C_6$)-Alkyl]-CO-NH-Heterozyklyl, N[($C_1$-$C_6$)-Alkyl]-CO-N(($C_1$-$C_6$)-Alkyl)$_2$,N[($C_1$-$C_6$)-Alkyl]-CO-N(($C_1$-$C_6$)-Alkyl)-Aryl,N[($C_1$-$C_6$)-Alkyl]-CO-N(($C_1$-$C_6$)-Alkyl)-Heterozyklyl, N[($C_1$-$C_6$)-Alkyl]-CO-N(Aryl)$_2$, N[($C_1$-$C_6$)-Alkyl]-CO-N(Heterozyklyl)$_2$, N(Aryl)-CO-($C_1$-$C_6$)-Alkyl, N(Heterozyklyl)-CO-($C_1$-$C_6$)-Alkyl, N(Aryl)-COO-($C_1$-$C_6$)-Alkyl, N(Heterozyklyl)-COO-($C_1$-$C_6$)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-($C_1$-$C_6$)-Alkyl, N(Helerozyklyl)-CO-NH-($C_1$-$C_6$)-Alkyl, N(Aryl)-CO-NH-Aryl, N(Heterozyklyl)-CO-NH-Aryl, N(Aryl)-CO-N(($C_1$-$C_6$)-Alkyl)$_2$, N(Heterozyklyl)-CO-N(($C_1$-$C_6$)-Alkyl)$_2$,N(Aryl)-CO-N[($C_1$-$C_6$)-Alkyl]-Aryl, N(Heterozyklyl)-CO-N[($C_1$-$C_6$)-Alkyl]-Aryl, N(Aryl)-CO-N(Aryl)$_2$, N(Heterozyklyl)-CO-N(Aryl)$_2$, Aryl, O-($CH_2$)$_n$-Aryl; O-(C-$H_2$)$_n$-Heterozyklyl, wobei n = 0 - 6 sein kann, wobei der Aryl- oder Heterozyklyl- Rest ein bis 3-fach substituiert sein kann mit F, Cl, Br, I, OH, $CF_3$, $NO_2$, CN, OCF3, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SF_5$, $SO_2$-$CH_3$, COOH, COO-($C_1$-$C_6$)-Alkyl, $CONH_2$.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Hexinyl.

Die Alkinylreste können ein oder mehrfach mit geeigneten Gruppen substituiert sein, wie z.B.: F, Cl, Br, I, $CF_3$, $NO_2$, $N_3$, CN, COOH, COO($C_1$-$C_6$)Alkyl, $CONH_2$, CONH($C_1$-$C_6$)Akyl, CON[($C_1$-$C_6$)Alkyl]$_2$, Cycloalkyl, ($C_2$-$C_6$)-Alkenyl, ($C_1$-$C_{10}$)-Alkyl, O-($C_1$-$C_6$)-Alkyl O-CO-($C_1$-$C_6$)-Alkyl, O-CO-($C_1$-$C_6$)-Aryl, O-CO-($C_1$-$C_6$)-Heterozyklyl;

$PO_3H_2$, P(O)(OAlkyl)2, ($C_1$-$C_6$)-Alkylen-P(O)(OAlkyl)$_2$, O-P(O)(OH)$_2$, O-P(O)(OAlkyl)$_2$, $SO_3H$, $SO_2$-$NH_2$, $SO_2$NH ($C_1$-$C_6$)-Alkyl, $SO_2$N[($C_1$-$C_6$)-Alkyl]$_2$, S-($C_1$-$C_6$)-Alkyl, S-($CH_2$)$_n$-Aryl, S-($CH_2$)$_n$-Heterozyklyl, SO-($C_1$-$C_6$)-Alkyl, SO-($CH_2$)$_n$-Aryl, SO-($CH_2$)$_n$-Heterozyklyl, $SO_2$-($C_1$-$C_6$)-Alkyl, $SO_2$-($CH_2$)$_n$-Aryl, $SO_2$-($CH_2$)$_n$-Heterozyklyl, $SO_2$-NH ($CH_2$)$_n$-Aryl, $SO_2$-NH($CH_2$)$_n$-Heterozyklyl, $SO_2$-N(($C_1$-$C_6$)-Alkyl)($CH_2$)$_n$-Aryl, $SO_2$-N(($C_1$-$C_6$)-Alkyl)($CH_2$)$_n$-Heterozyklyl, $SO_2$-N(($CH_2$)$_n$-Aryl)$_2$, , $SO_2$-N(($CH_2$)$_n$-(Heterozyklyl)$_2$ wobei n = 0 - 6 sein kann und der Arylrest oder Heterozyklische Rest bis zu zweifach mit F, Cl, Br, OH, $CF_3$, $SF_5$, $NO_2$, CN, $OCF_3$, O-($C_1$-$C_6$)-Alkyl, ($C_1$-$C_6$)-Alkyl, $NH_2$ substituiert sein kann;

C(NH)($NH_2$), $NH_2$, NH-($C_1$-$C_6$)-Alkyl, N(($C_1$-$C_6$)-Alkyl)$_2$, NH($C_1$-$C_7$)-Acyl, NH-CO-($C_1$-$C_6$)-Alkyl, NH-COO-($C_1$-$C_6$)-Alkyl, NH-CO-Aryl, NH-CO-Heterozyklyl, NH-COO-Aryl, NH-COO-Heterozyklyl, NH-CO-NH-($C_1$-$C_6$)-Alkyl), NH-CO-NH-Aryl, NH-CO-NH-Heterozyklyl, N[($C_1$-$C_6$)-Alkyl]-CO-($C_1$-$C_6$)-Alkyl, N[($C_1$-$C_6$)-Alkyl]-COO-($C_1$-$C_6$)-Alkyl, N[($C_1$-$C_6$)-Alkyl]-CO-Aryl, N[($C_1$-$C_6$)-Alkyl]-CO-Heterozyklyl, N[($C_1$-$C_6$)-Alkyl]-COO-Aryl, N[($C_1$-$C_6$)-Alkyl]-COO-Heterozyklyl, N[($C_1$-$C_6$)-Alkyl]-CO-NH-($C_1$-$C_6$)-Alkyl), N[($C_1$-$C_6$)-Alkyl]-CO-NH-Aryl, N[($C_1$-$C_6$)-Alkyl]-CO-NH-Heterozyklyl, N[($C_1$-$C_6$)-Alkyl]-CO-N(($C_1$-$C_6$)-Alkyl)$_2$,N[($C_1$-$C_6$)-Alkyl]-CO-N(($C_1$-$C_6$)-Alkyl)-Aryl,N[($C_1$-$C_6$)-Alkyl]-CO-N(($C_1$-$C_6$)-Alkyl)-Heterozyklyl, N[($C_1$-$C_6$)-Alkyl]-CO-N(Aryl)$_2$, N[($C_1$-$C_6$)-Alkyl]-CO-N(Heterozyklyl)$_2$, N(Aryl)-CO-($C_1$-$C_6$)-Alkyl, N(Heterozyklyl)-CO-($C_1$-$C_6$)-Alkyl, N(Aryl)-COO-($C_1$-$C_6$)-Alkyl, N(Heterozyklyl)-COO-($C_1$-$C_6$)-Alkyl, N(Aryl)-CO-Aryl, N(Heterozyklyl)-CO-Aryl, N(Aryl)-COO-Aryl, N(Heterozyklyl)-COO-Aryl, N(Aryl)=CO-NH=($C_1$-$C_6$)-Alkyl, N(Heterozyklyl)-CO-NH-($C_1$-$C_6$)-Alkyl, N(Aryl)-CO-NH-Aryl, N(Heterozyklyl)-CO-NH-Aryl, N(Aryl)-CO-N(($C_1$-$C_6$)-Alkyl)$_2$, N(Heterozyklyl)-CO-N(($C_1$-$C_6$)-Alkyl)$_2$, N(Aryl)-CO-N[($C_1$-$C_6$)-Alkyl]-Aryl, N(Heterozyklyl)-CO-N[($C_1$-$C_6$)-Alkyl]-Aryl, N(Aryl)-CO-N (Aryl)$_2$, N(Heterozyklyl)-CO-N(Aryl)$_2$, Aryl, O-($CH_2$)$_n$-Aryl, O-($CH_2$)$_n$-Heterozyklyl, wobei n = 0 - 6 sein kann, wobei der Aryl- oder Heterozyklyl- Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SF_5$, $SO_2$-$CH_3$, COOH, COO-($C_1$-$C_6$)-Alkyl, $CONH_2$.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen substituiert sein, wie z.B.: F, Cl, Br, I, $CF_3$, $NO_2$, $SF_5$, $N_3$, CN, COOH, COO($C_1$-$C_6$)Alkyl, $CONH_2$, CONH($C_1$-$C_6$)Alkyl, CON[($C_1$-$C_6$)Alkyl]$_2$, Cycloalkyl, ($C_1$-$C_{10}$)-Alkyl, ($C_2$-$C_6$)-Alkenyl, ($C_2$-$C_6$)-Alkinyl, O-($C_1$-$C_6$)-Alkyl O-CO-($C_1$-$C_6$)-Alkyl, O-CO-($C_1$-$C_6$)-Aryl, O-CO-($C_1$-$C_6$)-Heterozyklyl,;

$PO_3H_2$, P(O)(OAlkyl)$_2$, ($C_1$-$C_6$)-Alkylen-P(O)(OAlkyl)$_2$, O-P(O)(OH)$_2$, O-P(O)(OAlkyl)$_2$, $SO_3H$, $SO_2$-$NH_2$, $SO_2$NH ($C_1$-$C_6$)-Alkyl, $SO_2$N[($C_1$-$C_6$)-Alkyl]$_2$ , S-($C_1$-$C_6$)-Alkyl, S-($CH_2$)$_n$-Aryl, S-($CH_2$)$_n$-Heterozyklyl, SO-($C_1$-$C_6$)-Alkyl, SO-($CH_2$)$_n$-Aryl, SO-($CH_2$)$_n$-Heterozyklyl, $SO_2$-($C_1$-$C_6$)-Alkyl, $SO_2$-($CH_2$)$_n$-Aryl, $SO_2$-($CH_2$)$_n$-Heterozyklyl, $SO_2$-NH ($CH_2$)$_n$-Aryl, $SO_2$-NH($CH_2$)$_n$-Heterozyklyl, $SO_2$-N(($C_1$-$C_6$)-Alkyl)($CH_2$)$_n$-Aryl, $SO_2$-N(($C_1$-$C_6$)-Alkyl)($CH_2$)$_n$-Heterozyklyl, $SO_2$-N(($CH_2$)$_n$-Aryl)$_2$, , $SO_2$-N(($CH_2$)$_n$-(Heterozyklyl)$_2$ wobei n = 0 - 6 sein kann und der Arylrest oder Heterozyklische Rest bis zu zweifach mit F, Cl, Br, OH, $CF_3$, $NO_2$, $SF_5$, CN, $OCF_3$, O-($C_1$-$C_6$)-Alkyl, ($C_1$-$C_6$)-Alkyl, $NH_2$ substituiert sein kann;

C(NH)($NH_2$), $NH_2$, NH-($C_1$-$C_6$)-Alkyl, N(($C_1$-$C_6$)-Alkyl)$_2$, NH($C_1$-$C_7$)-Acyl,NH-CO-($C_1$-$C_6$)-Alkyl, NH-COO-($C_1$-$C_6$)-Alkyl, NH-CO-Aryl, NH-CO-Heterozyklyl, NH-COO-Aryl, NH-COO-Heterozyklyl, NH-CO-NH-($C_1$-$C_6$)-Alkyl), NH-CO-NH-Aryl, NH-CO-NH-Heterozyklyl, N[($C_1$-$C_6$)-Alkyl]-CO-($C_1$-$C_6$)-Alkyl, N[($C_1$-$C_6$)-Alkyl]-COO-($C_1$-$C_6$)-Alkyl, N[($C_1$-$C_6$)-Alkyl]-CO-Aryl, N[($C_1$-$C_6$)-Alkyl]-CO-Heterozyklyl, N[($C_1$-$C_6$)-Alkyl]-COO-Aryl, N[($C_1$-$C_6$)-Alkyl]-COO-Heterozyklyl, N[($C_1$-$C_6$)-Alkyl]-CO-NH-($C_1$-$C_6$)-Alkyl), N[($C_1$-$C_6$)-Alkyl]-CO-NH-Aryl, N[($C_1$-$C_6$)-Alkyl]-CO-NH-Heterozyklyl, N[($C_1$-$C_6$)-Alkyl]-CO-N(($C_1$-$C_6$)-Alkyl)$_2$,N[($C_1$-$C_6$)-Alkyl]-CO-N(($C_1$-C6)-Alkyl)-Aryl,N[($C_1$-$C_6$)-Alkyl]-CO-N(($C_1$-$C_6$)-Alkyl)-Heterozyklyl, N[($C_1$-C6)-Alkyl]-CO-N(Aryl)2, N[($C_1$-$C_6$)-Alkyl]-CO-N(Heterozyklyl)$_2$, N(Aryl)-CO-($C_1$-$C_6$)-Alkyl, N(Heterozyklyl)-CO-($C_1$-$C_6$)-Alkyl, N(Aryl)-COO-($C_1$-$C_6$)-Alkyl, N(Heterozyklyl)-COO-($C_1$-$C_6$)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-($C_1$-$C_6$)-Alkyl, N(Heterozyklyl)-CO-NH-($C_1$-$C_6$)-Alkyl, N(Aryl)-CO-NH-Aryl, N(Heterozyklyl)-CO-NH-Aryl, N(Aryl)-CO-N(($C_1$-$C_6$)-Alkyl)$_2$, N(Heterozyklyl)-CO-N(($C_1$-$C_6$)-Alkyl)$_2$, N(Aryl)-CO-N[($C_1$-$C_6$)-Alkyl]-Aryl, N(Heterozyklyl)-CO-N[($C_1$-$C_6$)-Alkyl]-Aryl, N(Aryl)-CO-N(Aryl)$_2$, N(Heterozyklyl)-CO-N(Aryl)$_2$, Aryl, O-($CH_2$)$_n$-Aryl, O-($CH_2$)$_n$-Heterozyklyl, wobei n = 0 - 6 sein kann, wobei der Aryl- oder Heterozyklyl- Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SF_5$, $SO_2$-$CH_3$, COOH, COO-($C_1$-$C_6$)-Alkyl, $CONH_2$.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.

Die Cycloalkylrestereste können ein oder mehrfach mit geeigneten Gruppen substituiert sein, wie z.B.: F, Cl, Br, I, $CF_3$, $NO_2$, $N_3$, CN, COOH, COO($C_1$-$C_6$)Alkyl, $CONH_2$, CONH($C_1$-$C_6$)Alkyl, CON[($C_1$-$C_6$)Alky]$_2$, Cycloalkyl, ($C_1$-$C_{10}$)-Alkyl, ($C_2$-$C_6$)-Alkenyl, ($C_2$-$C_6$)-Alkinyl, O-($C_1$-$C_6$)-Alkyl O-CO-($C_1$-$C_6$)-Alkyl, O-CO-($C_1$-$C_6$)-Aryl, O-CO-($C_1$-$C_6$)-Heterozyklyl,; $PO_3H_2$, P(O)(OAlkyl)$_2$, ($C_1$-$C_6$)-Alkylen-P(O)(OAkyl)$_2$, O-P(O)(OH)$_2$, O-P(O)(OAlkyl)$_2$, $SO_3H$, $SO_2$-$NH_2$, $SO_2$NH($C_1$-$C_6$)-Alkyl, $SO_2$N[($C_1$-$C_6$)-Alkyl]$_2$, S-($C_1$-$C_6$)-Alkyl, S-($CH_2$)$_n$-Aryl, S-($CH_2$)$_n$-Heterozyklyl, SO-($C_1$-$C_6$)-Alkyl, SO-($CH_2$)$_n$-Aryl, SO-($CH_2$)$_n$- Heterozyklyl, $SO_2$-($C_1$-$C_6$)-Alkyl, $SO_2$-($CH_2$)$_n$-Aryl, $SO_2$-($CH_2$)$_n$-Heterozyklyl , $SO_2$-NH($CH_2$)$_n$-Aryl, $SO_2$-NH($CH_2$)$_n$-Heterozyklyl, $SO_2$-N(($C_1$-$C_6$)-Alkyl)($CH_2$)$_n$-Aryl, $SO_2$-N(($C_1$-$C_6$)-Alkyl)($CH_2$)$_n$-Heterozyklyl, $SO_2$-N(($CH_2$)$_n$-Aryl)$_2$, , $SO_2$-N(($CH_2$)$_n$-(Heterozyklyl)$_2$ wobei n = 0 - 6 sein kann und der Arylrest oder Heterozyklische Rest bis zu zweifach mit F, Cl, Br, OH, $CF_3$, $SF_5$, $NO_2$, CN, $OCF_3$, O-($C_1$-$C_6$)-Alkyl, ($C_1$-$C_6$)-Alkyl, $NH_2$ substituiert sein kann;

C(NH)($NH_2$), $NH_2$, NH-($C_1$-$C_6$)-Alkyl, N(($C_1$-$C_6$)-Alkyl)$_2$, NH($C_1$-$C_7$)-Acyl, NH-CO-($C_1$-$C_6$)-Alkyl, NH-COO-($C_1$-$C_6$)-Alkyl, NH-CO-Aryl, NH-CO-Heterozyklyl, NH-COO-Aryl, NH-COO-Heterozyklyl, NH-CO-NH-($C_1$-$C_6$)-Alkyl), NH-CO-NH-Aryl, NH-CO-NH-Heterozyklyl, N[($C_1$-$C_6$)-Alkyl]-CO-($C_1$-$C_6$)-Alkyl, N[($C_1$-$C_6$)-Alkyl]-COO-($C_1$-$C_6$)-Alkyl, N[($C_1$-$C_6$)-Alkyl]-CO-Aryl, N[($C_1$-$C_6$)-Alkyl]-CO-Heterozyklyl, N[($C_1$-$C_6$)-Alkyl]-COO-Aryl, N[($C_1$-$C_6$)-Alkyl]-COO-Heterozyklyl, N[($C_1$-$C_6$)-Alkyl]-CO-NH-($C_1$-$C_6$)-Alkyl), N[($C_1$-$C_6$)-Alkyl]-CO-NH-Aryl, N[($C_1$-$C_6$)-Alkyl]-CO-NH-Heterozyklyl, N[($C_1$-$C_6$)-Alkyl]-CO-N(($C_1$-$C_6$)-Alkyl)$_2$,N[($C_1$-$C_6$)-Alkyl]-CO-N(($C_1$-$C_6$)-Alkyl)-Aryl,N[($C_1$-$C_6$)-Alkyl]-CO-N(($C_1$-$C_6$)-Alkyl)-Heterozyklyl, N[($C_1$-$C_6$)-Alkyl]-CO-N(Aryl)$_2$, N[($C_1$-$C_6$)-Alkyl]-CO-N(Heterozyklyl)$_2$, N(Aryl)-CO-($C_1$-$C_6$)-Alkyl, N(Heterozyklyl)-CO-($C_1$-$C_6$)-Alkyl, N(Aryl)-COO-($C_1$-$C_6$)-Alkyl, N(Heterozyklyl)-COO-($C_1$-$C_6$)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-($C_1$-$C_6$)-Alkyl, N(Heterozyklyl)-CO-NH-($C_1$-$C_6$)-Alkyl, N(Aryl)-CO-NH-Aryl, N(Heterozyklyl)-CO-NH-Aryl, N(Aryl)-CO-N(($C_1$-$C_6$)-Alkyl)$_2$, N(Heterozyklyl)-CO-N(($C_1$-$C_6$)-Alkyl)$_2$, N(Aryl)-CO-N[($C_1$-$C_6$)-Alkyl]-Aryl, N(Heterozyklyl)-CO-N[($C_1$-$C_6$)-Alkyl]-Aryl, N(Aryl)-CO-N(Aryl)$_2$, N(Heterozyklyl)-CO-N(Aryl)$_2$, Aryl, O-($CH_2$)$_n$-Aryl, O-($CH_2$)$_n$-Heterozyklyl, wobei n = 0 - 6 sein kann, wobei der Aryl- oder Heterozyklyl- Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SF_5$, $SO_2$-$CH_3$, COOH, COO-($C_1$-$C_6$)-Alkyl, $CONH_2$.

Unter Heterozyklyl bzw. heterozyklischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterozyklische Rest mit Benzolkernen kondensiert ist.

Geeignete Heterozyklyl- bzw. "heterozyklische Reste" sind Acridinyl, Benzimidazolyl, Benzofuryl, Benzothienyl, Benzothiophenyl, Benzoxazolyl, Benzthiazolyl, Benztriazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyrazolidinyl, Pyrazolinyl, Pyrazolyl, Pyridazinyl, Pyridooxazole, Pyridoimidazole, Pyridothiazol, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl, Aziridinyl, Azetininyl, Azepanyl, Azocanyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-Pyridyl.

Umfasst sind weiterhin ein oder mehrfach benzoannelierte Derivate dieser Heterozyklen.

Die heterozyklischen Ringe bzw. Heterozyklische Reste können ein oder mehrfach mit geeigneten Gruppen substituiert sein, wie z.B: F, Cl, Br, I, $CF_3$, $NO_2$, $N_3$, CN, COOH, COO($C_1$-$C_6$)Alkyl, $CONH_2$, CONH($C_1$-$C_6$)Alkyl, CON[($C_1$-$C_6$)Alkyl]$_2$, Cycloalkyl, ($C_1$-$C_{10}$)-Alkyl, ($C_2$-$C_6$)-Alkenyl, ($C_2$-$C_6$)-Alkinyl, O-($C_1$-$C_6$)-Alkyl O-CO-($C_1$-$C_6$)-Alkyl, O-CO-($C_1$-$C_6$)-Aryl, O-CO-($C_1$-$C_6$)-Heterozyklyl;

$PO_3H_2$, P(O)(OAlkyl)$_2$, ($C_1$-$C_6$)-Alkylen-P(O)(OAlkyl)$_2$, O-P(O)(OH)$_2$, O-P(O)(OAlkyl)$_2$, $SO_3H$, $SO_2$-$NH_2$, $SO_2$NH

$(C_1-C_6)$-Alkyl, $SO_2N[(C_1-C_6)$-Alkyl$]_2$, S-$(C_1-C_6)$-Alkyl, S-$(CH_2)_n$-Aryl, S-$(CH_2)_n$-Heterozyklyl, SO-$(C_1-C_6)$-Alkyl, SO-$(CH_2)_n$-Aryl, SO-$(CH_2)_n$-Heterozyklyl, $SO_2$-$(C_1-C_6)$-Alkyl, $SO_2$-$(CH_2)$-Aryl, $SO_2$-$(CH_2)_n$-Heterozyklyl, $SO_2$-NH $(CH_2)_n$-Aryl, $SO_2$-NH$(CH_2)_n$-Heterozyklyl, $SO_2$-N$(C_1-C_6)$-Alkyl)$(CH_2)_n$-Aryl, $SO_2$-N$(C_1-C_6)$-Alkyl)$(CH_2)_n$-Heterozyklyl, $SO_2$-N($(CH_2)_n$-Aryl$)_2$, , $SO_2$-N($(CH_2)_n$-(Heterozyklyl$)_2$ wobei n = 0 - 6 sein kann und der Arylrest oder heterozyklische Rest bis zu zweifach mit F, Cl, Br, OH, $CF_3$, $SF_5$, $NO_2$, CN, $OCF_3$, O-$(C_1-C_6)$-Alkyl, $(C_1-C_6)$-Alkyl, $NH_2$ substituiert sein kann;

C(NH)$(NH_2)$, $NH_2$, NH-$(C_1-C_6)$-Alkyl, N($(C_1-C_6)$-Alkyl$)_2$, NH$(C_1-C_7)$-Acyl, NH-CO-$(C_1-C_6)$-Alkyl, NH-COO-$(C_1-C_6)$-Alkyl, NH-CO-Aryl, NH-CO-Heterozyklyl, NH-COO-Aryl, NH-COO-Heterozyklyl, NH-CO-NH-$(C_1-C_6)$-Alkyl), NH-CO-NH-Aryl, NH-CO-NH-Heterozyklyl, N[$(C_1-C_6)$-Alkyl]-CO-$(C_1-C_6)$-Alkyl, N[$(C_1-C_6)$-Alkyl]-COO-$(C_1-C_6)$-Alkyl, N[$(C_1-C_6)$-Alkyl]-CO-Aryl, N[$(C_1-C_6)$-Alkyl]-CO-Heterozyklyl, N[$(C_1-C_6)$-Alkyl]-COO-Aryl, N[$(C_1-C_6)$-Alkyl]-COO-Heterozyklyl, N[$(C_1-C_6)$-Alkyl]-CO-NH-$(C_1-C_6)$-Alkyl), N[$(C_1-C_6)$-Alkyl]-CO-NH-Aryl, N[$(C_1-C_6)$-Alkyl]-CO-NH-Heterozyklyl, N[$(C_1-C_6)$-Alkyl]-CO-N($(C_1-C_6)$-Alkyl$)_2$,N[$(C_1-C_6)$-Alkyl]-CO-N($(C_1-C_6)$-Alkyl)-Aryl,N[$(C_1-C_6)$-Alkyl]-CO-N($(C_1-C_6)$-Alkyl)-Heterozyklyl, N[$(C_1-C_6)$-Alkyl]-CO-N(Aryl$)_2$, N[$(C_1-C_6)$-Alkyl]-CO-N(Heterozyklyl$)_2$, N(Aryl)-CO-$(C_1-C_6)$-Alkyl, N(Heterozyklyl)-CO-$(C_1-C_6)$-Alkyl, N(Aryl)-COO-$(C_1-C_6)$-Alkyl, N(Heterozyklyl)-COO-$(C_1-C_6)$-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-$(C_1-C_6)$-Alkyl, N(Heterozyklyl)-CO-NH-$(C_1-C_6)$-Alkyl, N(Aryl)-CO-NH-Aryl, N(Heterozyklyl)-CO-NH-Aryl, N(Aryl)-CO-N($(C_1-C_6)$-Alkyl$)_2$, N(Heterozyklyl)-CO-N($(C_1-C_6)$-Alkyl$)_2$, N(Aryl)-CO-N[$(C_1-C_6)$-Alkyl]-Aryl, N(Heterozyklyl)-CO-N[$(C_1-C_6)$-Alkyl]-Aryl, N(Aryl)-CO-N (Aryl$)_2$, N(Heterozyklyl)-CO-N(Aryl$)_2$, Aryl, O-$(CH_2)_n$-Aryl, O-$(CH_2)_n$-Heterozyklyl, wobei n = 0 - 6 sein kann, wobei der Aryl- oder Heterozyklyl- Rest ein bis 3-fach substituiert sein kann mit F, Cl, Br, I, OH, $CF_3$, $NO_2$, CN, $OCF_3$, $SF_5$, O-$(C_1-C_6)$-Alkyl, $(C_1-C_6)$-Alkyl, $NH_2$, NH$(C_1-C_6)$-Alkyl, N($(C_1-C_6)$-Alkyl$)_2$, $SF_5$, $SO_2$-$CH_3$, COOH, COO-$(C_1-C_6)$-Alkyl, $CONH_2$.

Die Verbindungen der Formel **1** lassen sich nach an sich bekannten Methoden herstellen. So kann man ausgehend vom kommerziell erhältlichen D-Allyl-Glycin **2** durch Schutz der Aminogruppe mit gängigen Schutzgruppen wie Boc zu **3**, Oxidation der Allylgruppe mit Ozon oder Osmiumtetroxid/Natriumperiodat zum zyklischen Aldehydderivat **4**, Reaktion mit Cysteinderivaten wie beispielsweise dem Methylester zu **5**, Zyklisierung mit gängigen, Säure aktivierenden, Ragentien wie DCC oder Chlormethylpyridinium-iodid, Ammonolyse des erhaltenen bizyklischen Thiazolidinesters **6** mit methanolischer Ammoniaklösung, Umwandlung des erhaltenen Amids **7** in das Nitril **8** und Derivatisierung des Amins nach gängigen Methoden wie Alkylierung mit Alkylhalogeniden oder der reduktiven Alkylierung mit Aldehyden oder Ketonen zu den Nitrilen **9**. Andererseits ist es möglich durch Einsatz von L-Allyl-glycin als kommerziell erhältliche Ausgangsverbindung stereoisomere Formen der Derivate **8** und **9** herzustellen. Die diastereoisomeren Gemische **5**, die bei der Zyklisierung mit Cysteinderivaten nach gängigen Methoden erhalten werden, können nach bekannten Methoden wie beispielsweise durch Säulenchromatografie oder durch Umkristallisation aus einem geeigneten Lösemittel in die einzelnen Diastereomeren aufgetrennt werden. Die stereoisomeren Formen können aber auch auf einer späteren Stufe der Synthese nach gängigen Methoden getrennt und gereinigt werden.

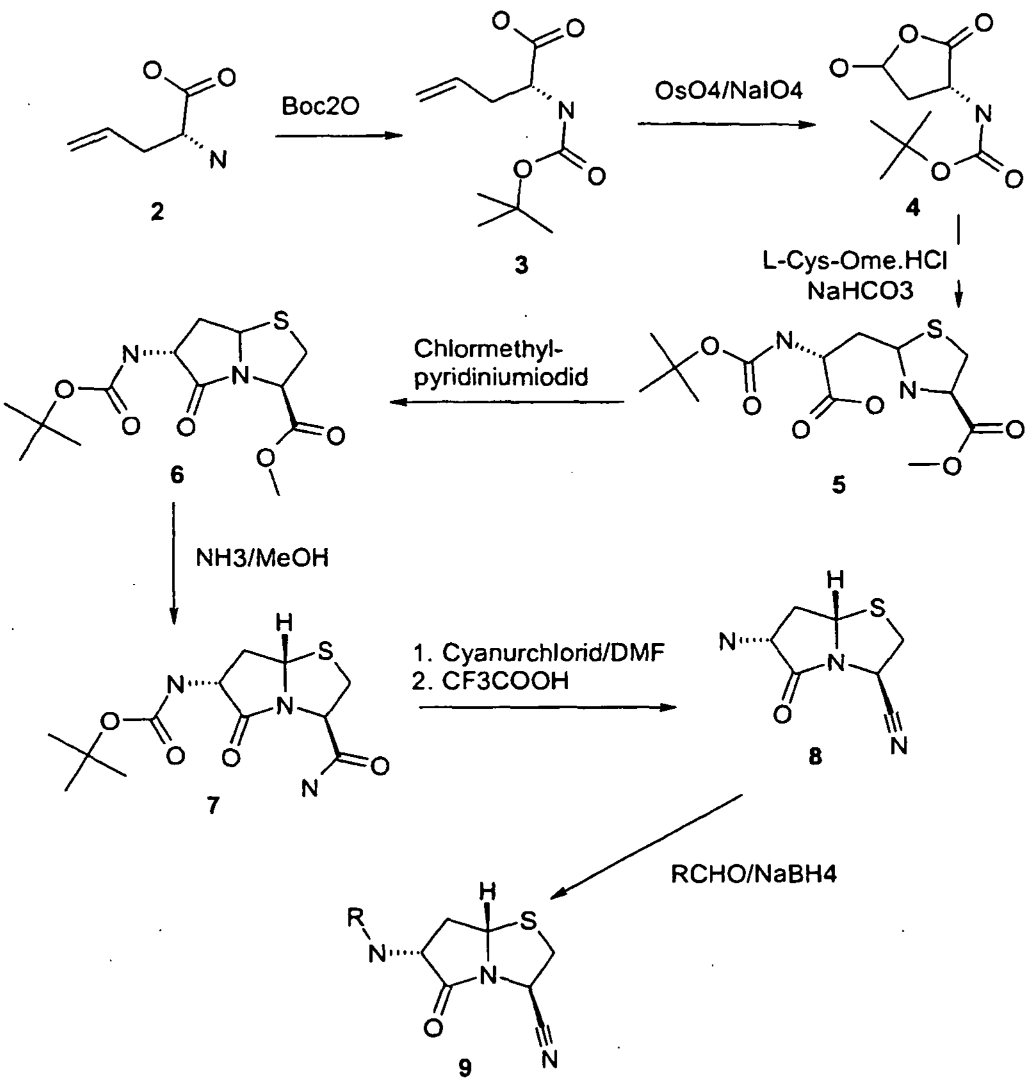


2 3 4 5 6 7 8 9

Eine weitere Methode zur Herstellung der erfindungsgemäßen Verbindungen der Formel 1 besteht in der an sich bekannten Umwandlung der kommerziell erhältlichen Säuren 10, in denen R2 nicht Wasserstoff sein kann, zu den Oxazolidonen 11 durch Einwirkung von Pivalinaldehyd und Chlorameisensäureallylester. Aus 11 können dann durch Reaktion mit starken Basen wie beispielsweise Kaliumbistrimethylsilylamid und Alkylierung mit Allylbromid mit nachfolgender Entfernung der Schutzgruppen und Ringöffnung unter geeigeten Bedingungen die Aminosäuren **12** hergestellt werden (Heterocycles 34 (5), 1992 903-906), aus denen nach Einführung von N-Schutzgruppen wie beispielsweise der BOC-Gruppe zu **13** und Veresterung zu **14** beispielsweise mit Diazomethan oder Trimethylsilyldiazomethan durch Oxidation mit Osmiumtetroxid und Natriumperiodat die Aldehyde **15** erhältlich sind. Durch Erhitzen dieser Aldhyde mit L-Cystein in Pyridin können die bizyklischen Derivate **16** und aus ihnen nach bekannten Methoden durch Veresterung die Stoffe **17**, und durch Ammonolyse die Amide **18** erhalten werden. Umwandlung des Amids **18** zum Nitril **19** mit beispielweise Trifluoressigsäureanhydrid oder Cyanurchlorid und Abspaltung der Schutzgruppe nach bekannten Verfahren führt zu den erfindungsgemäßen Stoffen **20**, die gegebenenfalls als Salze oder freie Basen isoliert werden können.

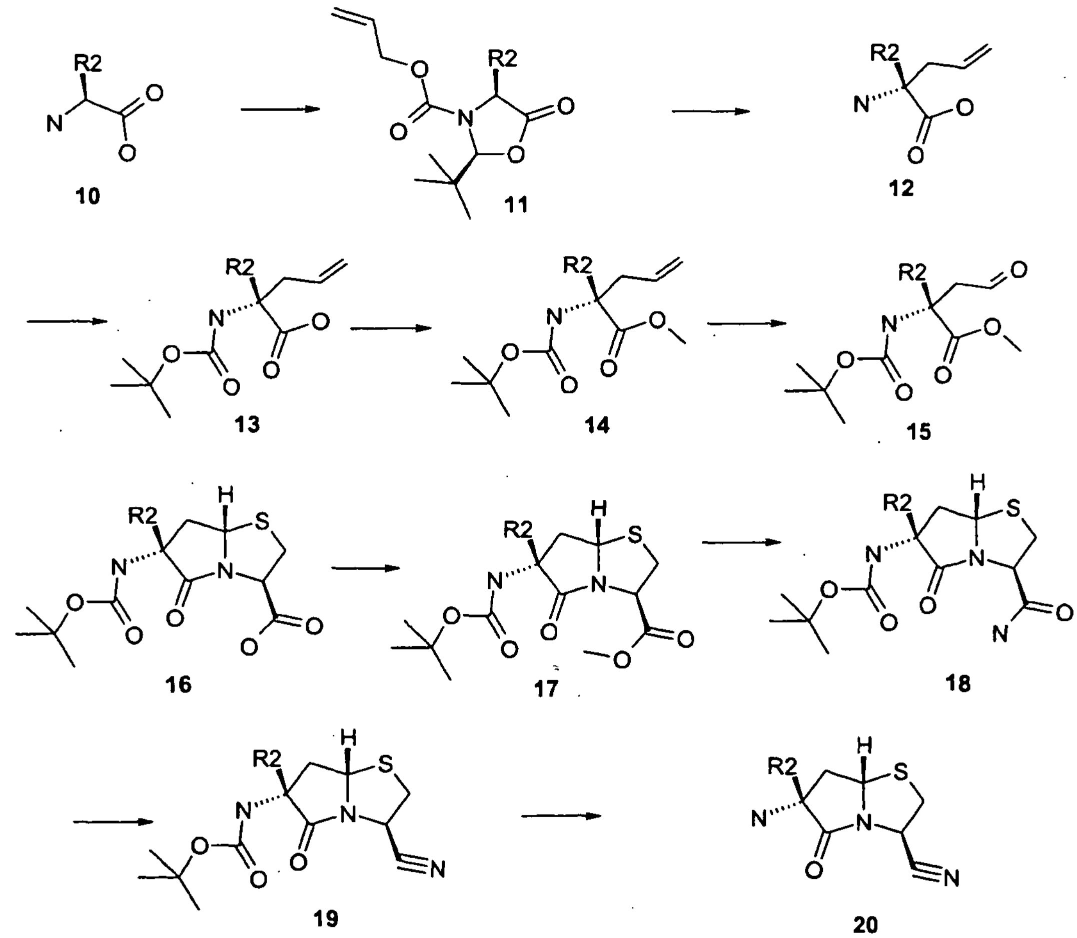

10 11 12

13 14 15

16 17 18

19 20

Eine weitere Möglichkeit zur Erzeugung von Verbindungen der allgemeinen Formel **1** besteht darin, daß die kommerziell erhältliche Verbindung **21** durch Deprotonierung mit einer starken Base (z. B. Kaliumhexamethyldisilazid (KHMDS)) und Alkylierung mit einem Allylderivat (z. B. Allylbromid) in die Verbindung **22** überführt wird (Synlett 1992, 249-251). Anschließend wird wieder mit einer starken Base (z. B. Natriumhexamethyldisilazid (NaHMDS) in Anwesenheit von 15-Krone-5) deprotoniert und dann mit einem Alkylierungsmittel R2X (X = Abgangsgruppe, z. B. Cl, Br, I, Mesyl, Tosyl, Triflat) zur Verbindung **23** umgesetzt. Spaltung mit Alkalimetallen (z. B. Natrium) in flüssigem Ammoniak ergibt Verbindung **24**. Durch Oxidation mit Osmiumtetroxid/Natriumperiodat oder Ozon erhält man dann das zyklische Aldehydderivat **25**. Daran schließt sich eine Umsetzung mit L-Cysteinderivaten (z. B. L-Cysteinmethylester Hydrochlorid) in Anwesenheit einer Base (z. B. Triethylamin oder Hünig Base) an, wobei ein Thiazolidinintermediat erhalten wird, welches in Anwesenheit eines Säure aktivierenden Reagenz wie zum Beispiel eines Carbodiimids (z. B. N'-(3-Dimethylaminopropyl)-N-ethylcarbodiimid Hydrochlorid) zu den beiden diastereomeren Thiazolididen **26** und **27** cyclisiert. Nach Trennung der Diastereomeren mit geeigneten Methoden wie durch Umkristallisation oder chromatografische Methoden wird Verbindung **26** mit Ammoniak in das Amid **28** überführt. Umsetzung zum Nitril **29** nach gängigen Methoden (z. B. Trifluoressigsäureanhydrid und Triethylamin, oder Cyanurchlorid) und Abspaltung der Boc-Gruppe unter sauren Bedingungen (z. B. Trifluoressigsäure in Anwesenheit von Thioanisol) ergibt die Verbindung **30**, welche dann wie oben erwähnt zu den Stoffen **31** umgesetzt werden kann.

Diastereomere dieser Verbindungen können entsprechend ausgehend vom anderen Enantiomeren der Ausgangsverbindung **21** hergestellt werden oder durch weitereumsetzung der Diastereomeren **27** zu den der Formel **30** und **31** analogen stereoisomeren Derivaten.

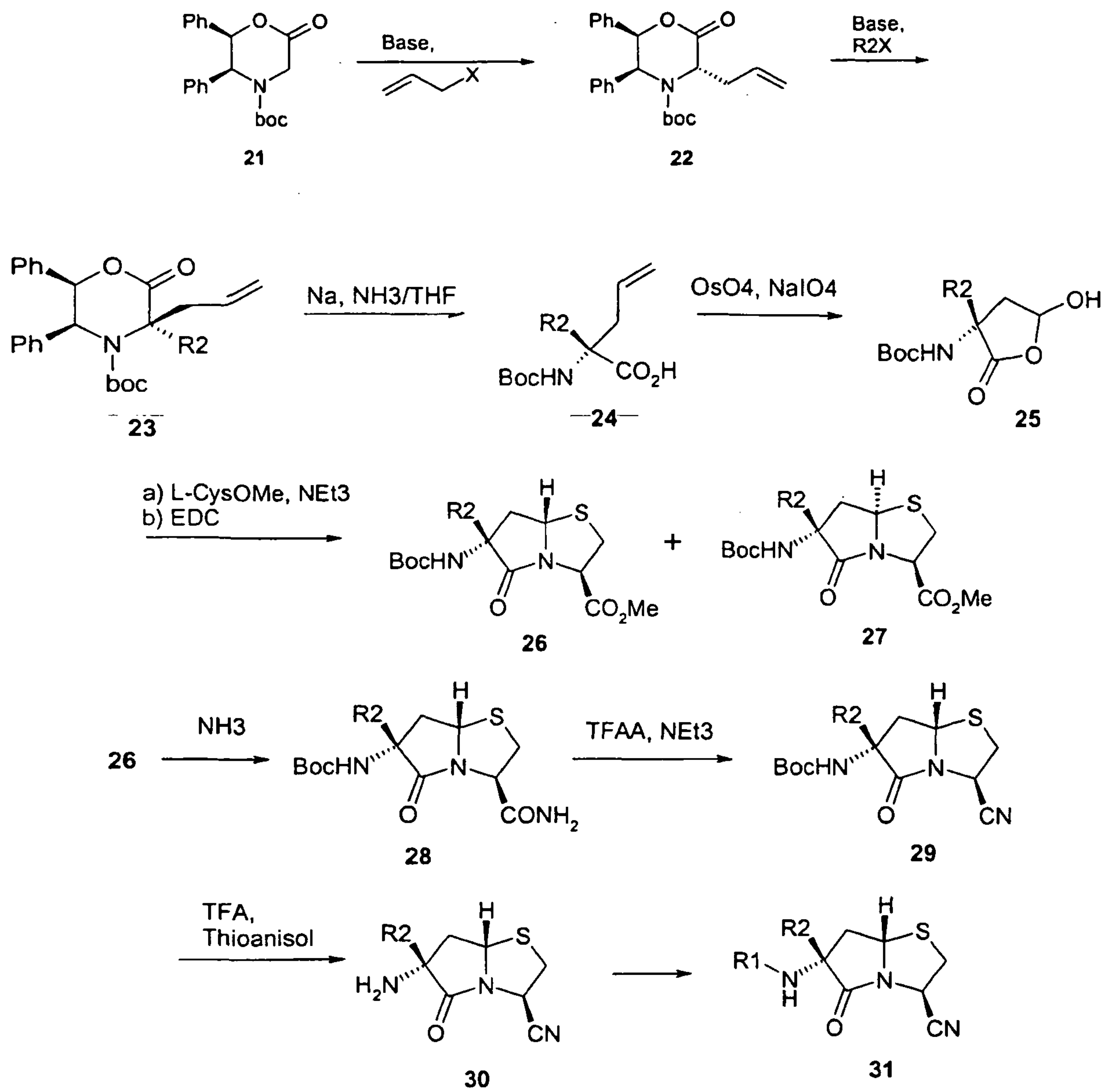


21 22 23 24 25 26 27 28 29 30 31

Eine weitere Herstellungsmethode der erfindungsgemäßen Stoffe der Formel **1** startet ausgehend vom kommerziell erhältlichen Ester **32**. Deprotonierung von **32** mit geeigneten Basen wie NaHMDS und Alkylierung mit Allylbromid ergibt **33**, das durch Verseifung beispielsweise mit Natronlauge und durch Oxidation beispielsweise mit Ozon oder Osmiumtetroxid/Natriumperiodat in das cyclische Aldehydderivat **34** überführt werden kann. Kondensation mit L-Cysteinmethylester-hydrochlorid zum Thiazolidin und anschließende Zyklisierung mit Säure aktivierenden Mitteln wie zum Beispiel 2-Chlor-1-methylpyridiniumiodid in Anwesenheit von Triethylamin ergibt das trizyklische Thiazolidid **35**, das sich neben anderen Diastereomeren dieser Konstitution durch gängige Trennverfahren wie der Säulenchromatografie isolieren lässt.. Mit Ammoniak wird dann in bekannter Weise das Amid 36 erhalten, welches mit Trifluoressigsäureanhydrid in Gegenwart von geeigneten Basen wie Triethylamin in das Nitril **37** überführt wird. Abspaltung der Schutzgruppen mit sauren Reagentien wie Trifluoressigsäure ergibt dann die Verbindung **38**, welche nach Umwandlung in den geschützten Aldehyd **39** (Sg = Schutzgruppe) durch reduktive Aminierung (Nu = Rest eines sekundären Amins) zu **40** und weiter wie oben erwähnt zu den Stoffen der Formel **41** umgesetzt werden kann.

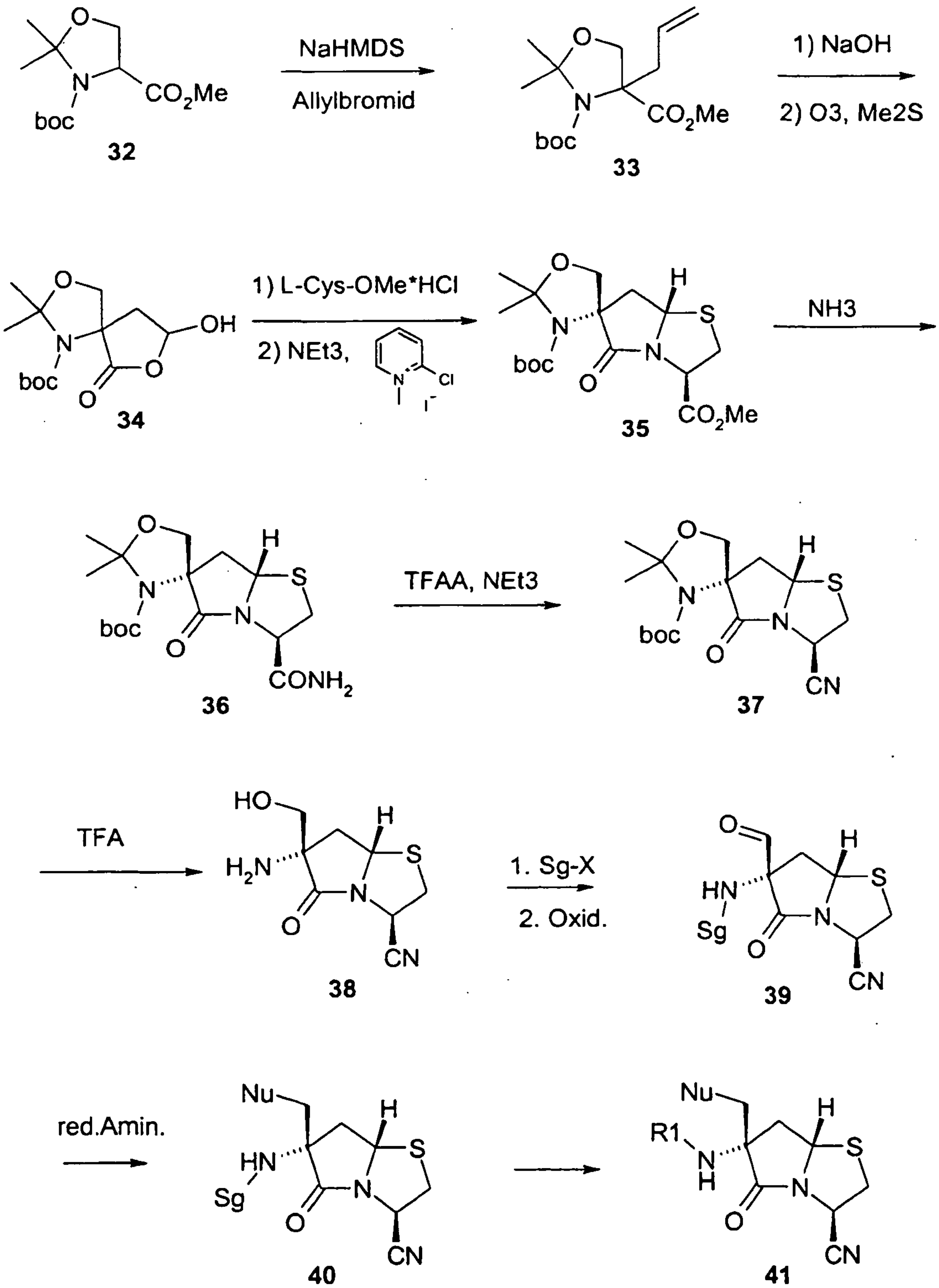


32 33 34 35 36 37 38 39 40 41

Eine weitere Möglichkeit Verbindungen der Formel **41** herzustellen, besteht darin, dass zunächst Verbindung **42** wie unten gezeigt durch metallische Reduktion aus **23** (R2 = $CH_2$-OBn) erzeugt wird. Doppelte Schützung von Hydroxigruppe und Carbonsäure mit tert.-Butyldimethylsilylchlorid (TBSCl) und Imidazol, sowie anschließende Hydrolyse des Silylesters ergibt Verbindung **43**. Mittels Umsetzung mit Osmiumtetroxid und Natriumperiodat wird das cyclische Aldehydderivat **44** erhalten. Durch Kondensation mit Cysteinderivaten (z. B. L-Cysteinmethylester Hydrochlorid) und anschließender Cyclisierung mit Säure aktivierenden Mitteln wie z. B. 1-(3-Dimethylaminopropyl)-3-ethyl-carbodiimid Hydrochlorid (EDC) werden die diastereomeren Bicyclen **45** und **46** erhalten. Diese können mit gängigen Methoden, zum Beispiel chromatografisch, getrennt werden. Abspaltung der Silylgruppe von **45** nach gängigen Methoden (z. B. Tetrabutylammoniumfluorid in THF) ergibt **47**. Oxidation der Alkoholfunktion nach bekannten Verfahren (z. B. Dess-Martin-Periodinan) und reduktive Aminierung (Nu = Rest eines primären oder sekundären Amins) erzeugt dann die Verbindungen **48**. Mit Ammoniak wird daraus das Amid hergestellt, welches mit Säure aktivierenden Reagentien (z. B. Trifluoressigsäureanhydrid in Gegenwart von Triethylamin) in das Nitril **49** überführt wird. Abspaltung der Boc-Schutzgruppe mit sauren Reagentien (z. B. Trifluoressigsäure) ergibt dann die Verbindungen **41**.

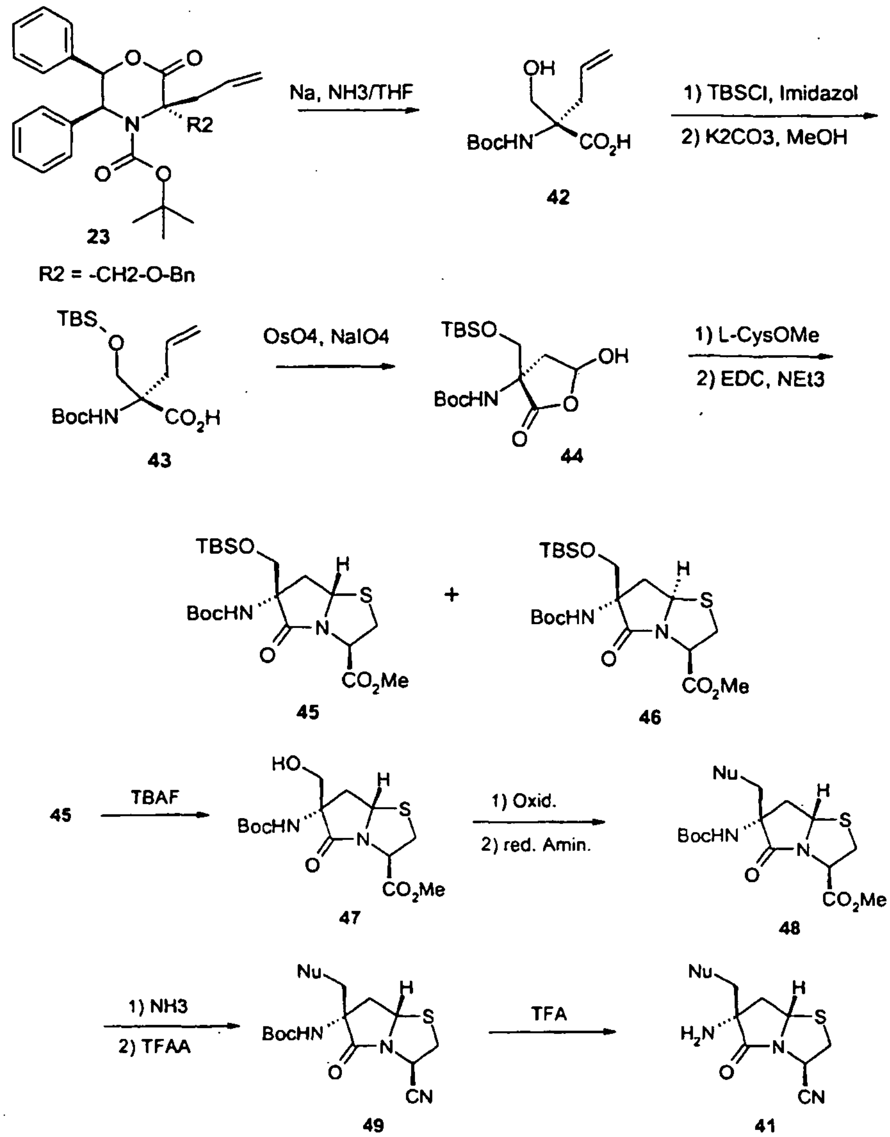


23, 42, 43, 44, 45, 46, 45, 47, 48, 49, 41

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Kilogramm Körpergewicht pro Tag, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95

Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxipropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindung(en) der Formel (I) können auch in Kombination mit weiteren Wirkstoffen verabreicht werden.

Als weitere Wirkstoffe für die Kombinationspräparate sind geeignet:

Alle Antidiabetika, die in der Roten Liste 2003, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus® (siehe www.lantus.com) oder HMR 1964, schnell wirkende Insuline (siehe US 6,221,633), GLP-1-Derivate wie z.B. diejenigen, die in WO 97/26265, WO 99/03861, WO 01/04156, WO 00/34331, WO00/34332, WO91/11457 und US 6,380,357 offenbart wurden, sowie oral wirksame hypoglykämische Wirkstoffe.

Die oral wirksamen hypoglykämischen Wirkstoffe umfassen vorzugsweise Sulphonylfhamstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, oder Verbindungen wie in WO 02/50027 oder WO 04/007455 beschrieben, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US00/11833, PCT/US00/11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht. Bei einer Ausführungsform der Erfindung werden die Verbindungen der Formel I in Kombination mit einem Squalen Synthetase Inhibitor, wie z.B. BMS-188494, verabreicht.

Bei einer Aüsführungsförm der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylhamstoff, wie z.B. Tolbutamid, Glibenclamid, Glipizid oder Glimepirid verabreicht. Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I in Kombination mit einem $\alpha$-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylhamstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]- amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), Cannabinoid 1 Rezeptor Antagonisten (z.B. Rimonabant oder Verbindungen, wie in WO 02/28346 beschrieben), H3-Agonisten (3-Cyclohexyl-1-(4,4-dimethyl-1,4,6,7-tetrahydroimidazo[4,5-c]pyridin-5-yl)-propan-1- on Oxalsäuresalz (WO 00 / 63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, $\beta$3-Agonisten (z.B. 1-(4-Chloro-3-methanesulfonylmethyl-phenyl)-2-[2-(2,3-dimethyl-1H-indol-6-yloxy)- ethylamino]-ethanol; hydrochloride (WO 01/83451)), MSH (Melanocyt-stimulierendes Hormon)-Agonisten, CCK-A Agonisten (z.B. (2-[4-(4-Chloro-2,5-dimethoxyphenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2- carboxylic acid tert-butylester (WO 01/85695)), TRH-Agonisten (siehe z.B. EP 0 462 884) entkoppelnde Protein 2-oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),

DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-$\beta$-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff ein Blutdrucksenker, wie z.B. ein ACE-Hemmer.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform ist der weitere Wirkstoff Rimonabant.

Bei einer Ausführungsform werden die Verbindungen der Formel I in Kombination mit Ballaststoffen, vorzugsweise unlöslichen Ballaststoffen (siehe z.B. Carob/ Caromax® (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax® kann in einer Zubereitung erfolgen, oder durch getrennte Gabe von Verbindungen der Formel I und Caromax®. Caromax® kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

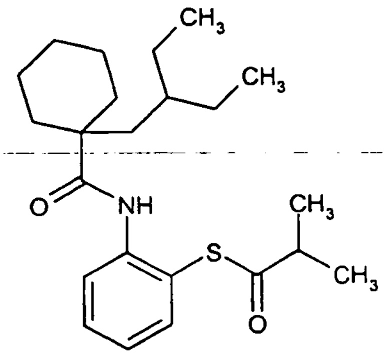

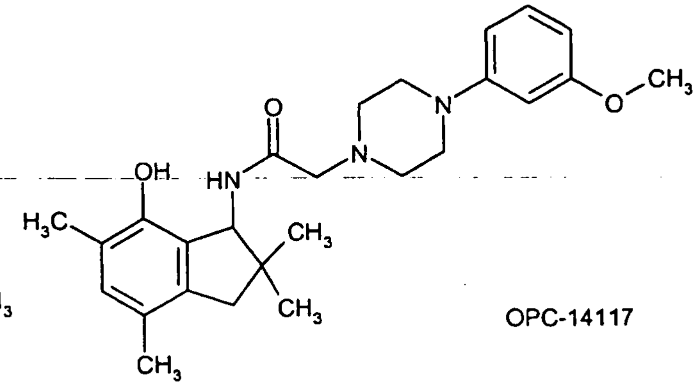

OPC-14117

JTT-705

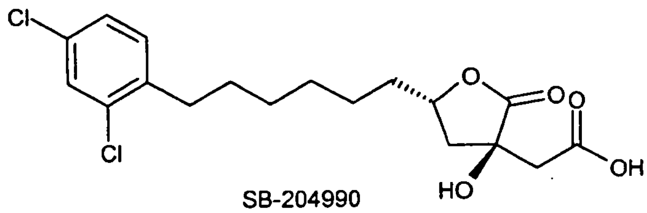

SB-204990

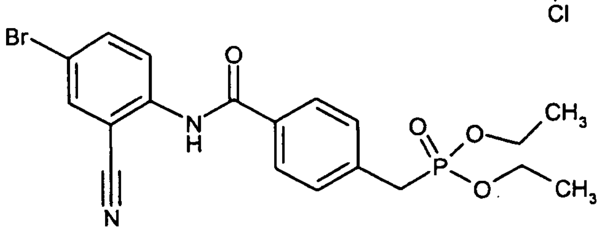

NO-1886

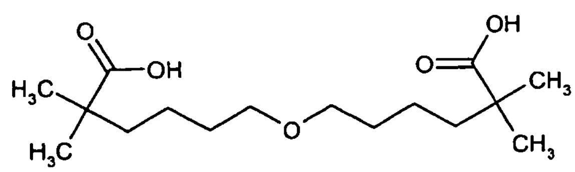

CI-1027

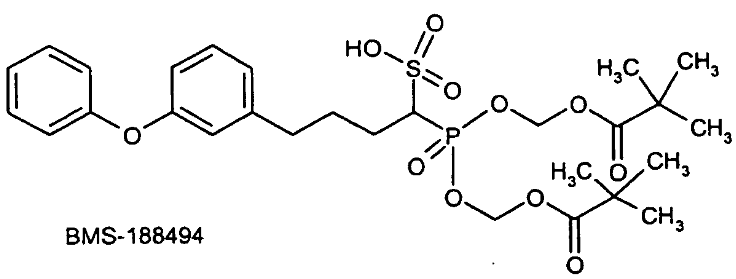

BMS-188494

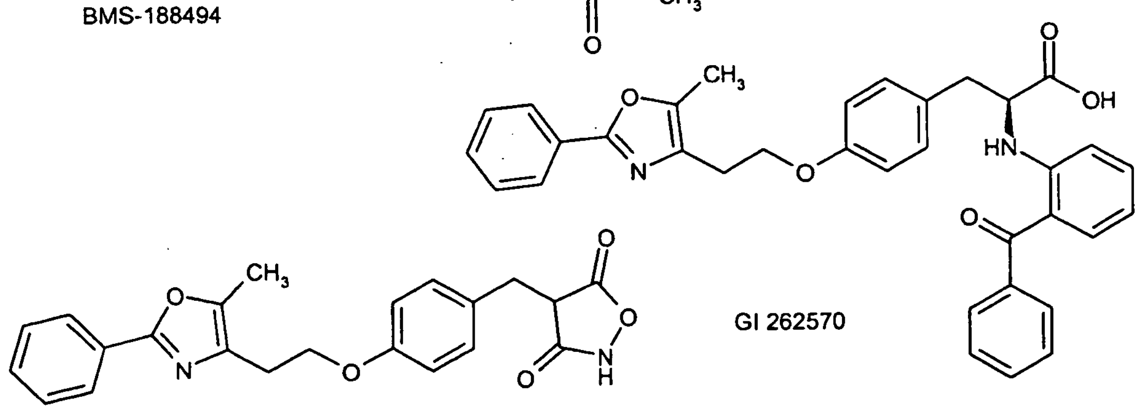

GI 262570

JTT-501

Die nachfolgend in der Tabelle I aufgeführten theoretischen Beispiele dienen zur

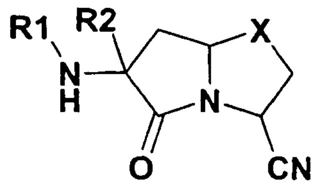

Erläuterung der Erfindung. Sie können analog zu den Ausführungsbeispielen hergestellt werden.

Formel I

Tabelle I

| | **R1** | **R2** | **X** |
|---|---|---|---|
| a | H | Me | S |
| b | H | i-Pr | S |
| c | H | Me | SO |
| d | H | Me | $SO_2$ |
| e | H | c-Hexyl | S |
| f | Me | H | S |
| g | i-Pr | H | S |
| h | i-Pr | H | SO |
| I | i-Pr | Me | S |
| j | 1-Adamantyl | H | S |
| k | 1-Hydroxi-adamant-3-yl | H | S |
| l | Ph | H | S |
| m | 2-Pyridyl | H | S |
| n | $-(CH_2)_2$-NH-2-pyridyl | H | S |
| o | 1,1-Dimethyl-2-phenyl | H | S |
| p | 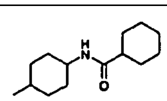 | H | S |
| q | 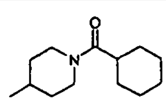 | H | S |
| r | Me | $-CH_2-CH(CH_2)_2$ | S |
| s | Et | 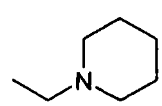 | S |
| t | $-CH_2-CH_2-OH$ | $-CH_2-CH(CH_3)_2$ | S |
| u | -CH2-CH2-OCH3 | 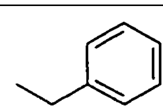 | S |
| v | $-CH_2-CH_2-N(CH_3)_2$ | $-CH_2-CH(CH_3)_2$ | S |

(fortgesetzt)

| | R1 | R2 | X |
|---|---|---|---|
| w | 2-Pyridyl | Me | S |
| x | 2-Thienyl | 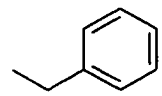 | S |
| y | $-CH_2-CH(CH_3)_2$ | $-CH_2-CH(CH_3)_2$ | S |
| aa | c-pentyl | $CH_2-CH_2-CH_3$ | S |
| ba | H | $CH_2-CH=C(CH_3)_3$ | S |
| ca | H | $CH_3$ | S |
| da | H | $CH_2-CH_3$ | S |
| ea | H | $CH_2OH$ | S |
| fa | H | $CH_2-O-CH_3$ | S |
| ga | H | $CH_2-O-CH_2-CH_3$ | S |
| ha | H | $CH_2-S-CH_3$ | S |
| ia | H | $CH_2-CH_2-N(CH_2-CH_3)_2$ | S |
| ja | H | $CH_2-C(CH_3)_3$ | S |
| la | H | $C(CH_3)_3$ | S |
| ma | H | $CH(CH_3)(Ph)$ | S |
| na | H | $CH_2$-S-Ph | S |
| oa | H | $CH_2-O-(C_6H_4-4-Cl)$ | S |
| pa | H | $CH_2-CH_2-CH(CH_3)_2$ | S |
| qa | H | CH2-$N(CH_3)_2$ | S |
| ra | H | $CH_2-N(CH_2-CH_3)_2$ | S |
| sa | H | 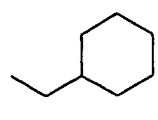 | S |
| ta | H | 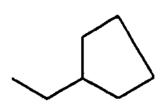 | S |
| ua | H | 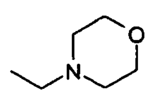 | S |
| va | H | 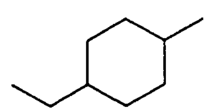 | S |
| wa | H | 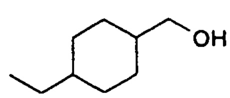 | S |

(fortgesetzt)

| | R1 | R2 | X |
|---|---|---|---|
| xa | H | 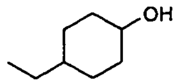 | S |
| ya | H | 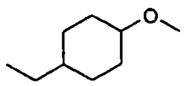 | S |
| za | H | 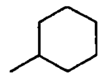 | S |
| ab | H | 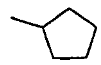 | S |
| bb | H | 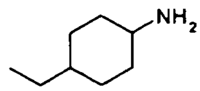 | S |
| cb | H | 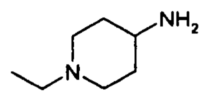 | S |
| db | H | 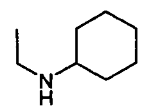 | S |
| eb | H | 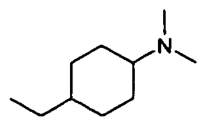 | S |
| fb | H | 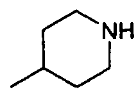 | SO |
| gb | H | 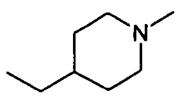 | S |
| hb | H | 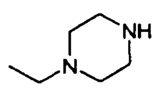 | S |
| ib | H | 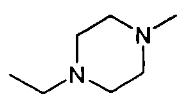 | S |

(fortgesetzt)

| | R1 | R2 | X |
|---|---|---|---|
| jb | H | 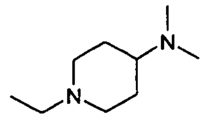 | S |
| kb | H | 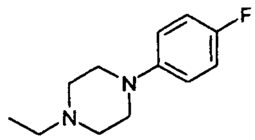 | S |
| lb | H | 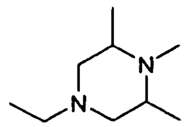 | S |
| mb | H | 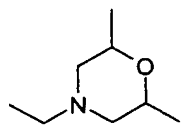 | S |
| nb | H | 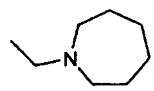 | S |
| ob | H | 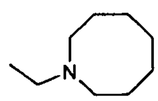 | S |
| pb | H | 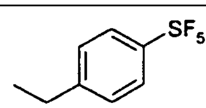 | S |
| qb | H | 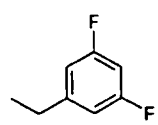 | S |
| rb | H | 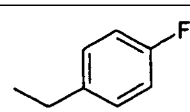 | S |
| sb | H | 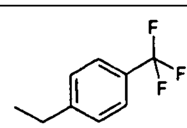 | S |
| tb | H | 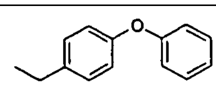 | S |

(fortgesetzt)

| | R1 | R2 | X |
|---|---|---|---|
| ub | H | 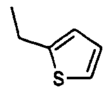 | S |
| vb | H | 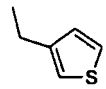 | S |
| wb | H | 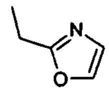 | S |
| xb | H | 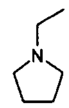 | S |
| yb | H | 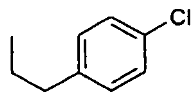 | S |
| zb | H | 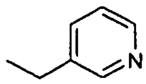 | S |
| ac | H | 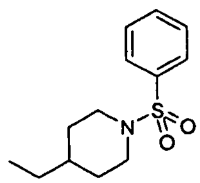 | S |
| bc | H | 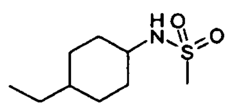 | S |
| cc | H | 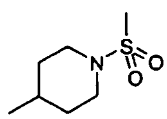 | S |
| cd | H | $-CH_2-CH(CH_3)_2$ | SO |
| ce | H | 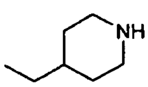 | SO |
| cf | H | 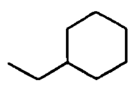 | SO |
| cg | H | $-CH_2-CH(CH_3)_2$ | SO2 |

Die Verbindungen der Formel I zeichnen sich durch günstige Wirkungen auf den Lipid-und Kohlenhydratstoffwechsel aus, sie senken insbesondere den Blutzuckerspiegel und sind zur Behandlung von Typ 2 Diabetes, von Insulinresistenz, von Dyslipidämien und des metabolischen Syndroms / Syndrom X geeignet. Weiterhin sind die Verbindungen zur Prophylaxe und Behandlung von arteriosklerotischen Erscheinungen geeignet. Die Verbindungen können allein oder in Kombination mit weiteren Blutzucker senkenden Wirkstoffen eingesetzt werden. Die Verbindungen wirken als DPP-IV Inhibitoren und eignen sich auch zur Behandlung von Störungen des Empfindens und anderer psychiatrischen Indikationen, wie zum Beispiel Depressionen, Angstzuständen, Angstneurosen, Schizophrenie sowie zur Behandlung von Störungen assoziiert mit dem zirkadianen Rhythmus, zur Gewichtsreduktion bei Säugetieren, zur Behandlung von Immunstörungen, und zur Behandlung von Drogenmissbrauch.

Weiterhin eignen sie sich zur Behandlung von Krebs, Arthritis, Osteoarthritis, Osteoporose, Schlafstörungen, Schlaf Apnoe, weiblicher und männlicher Sexualstörungen, Entzündungen, Akne, Pigmentierung der Haut, Störungen des Steroidstoffwechsels, Hautkrankheiten, Psoriasis, Mykosen, neurodegenerativer Krankheiten, Multiple Sklerose und Alzheimer-Krankheit.

Die Wirksamkeit der Verbindungen wurde wie folgt getestet:

Aktivitätstest

Messung der DPP-IV Aktivität:

Material:

DPP-N aus Schweineniere (Sigma, München)
H-Ala-Pro-AFC (Bachem, Weil am Rhein)

Testbedingungen:

DPP-IV (1 mU/ml, Endkonzentratio)
H-Ala-Pro-AFC (15μM Endkonzentration) in Tris/HCl (40 mM, pH 7.4), Gesamtvolumen 0,2 ml

Die Reaktion wurde bei Raumtemperatur für unterschiedliche Zeiträume (typischerweise 10 min) durchgerührt und am Ende der Reaktion durch Zugabe von 20μl $ZnCl_2$ (1 M) gestoppt. Der Umsatz von H-Ala-Pro-AFCwurde fluorimetrisch durch Messung der Emission bei 535nm nach Anregung bei 405 nm bestimmt. Im Falle der Zugabe von Inhibitoren wurde das zugegebene Puffervolumen so angepasst, dass ein Gesamtvolumen der Testmischung von 200μl eingehalten wurde.

IC50 Werte für Inhibitoren wurden durch Variation der Inhibitorkonzentrationen bei der angegebenen Substratkonzentration von 15μM bestimmt. Ki und Km Werte wurden durch enstsprechende Variation von Substrat- und Inhibitorkonzentration wie beschrieben (Dixon, M. and Webb, E.C.(1979) Enzymes , third edition, pp. 47-206, Academic Press) ermittelt. Die Werte für Km, IC50 and Ki wurden mit einem kommerziell erhältlichen Software-Paket errechnet (Leatherbarrow, R.J. (1992) GraFit Version 3.0, Erithacus Software Ltd. Staines, U.K.).

Die Messungen ergaben folgende Werte:

| Ausführungsbeispiel Nr. | IC-50 | Bemerkungen |
|---|---|---|
| 1) | 48 nM | Trifluoressigsäuresalz |
| 2) | 47 nM | Trifluoressigsäuresalz |
| 6) | 2 μM | Trifluoressigsäuresalz |
| 7) | 18 nM | Trifluoressigsäuresalz |
| 8) | 400 nM | Trifluoressigsäuresalz |
| 9) | 110 nM | Trifluoressigsäuresalz |
| 10) | 300 nM | Bistrifluoressigsäuresalz |
| 11) | 500 nM | Bistrifluoressigsäuresalz |

Die Ausführungsbeispiele 1 bis 11 wurden wie folgt hergestellt:

**1) (3R,6R,7aS)-6-Amino-6-benzyl-5-oxo-hexahydro-pyrrolo[2,1-b]thiazol-3-carbonitrile-trifluoracetat**

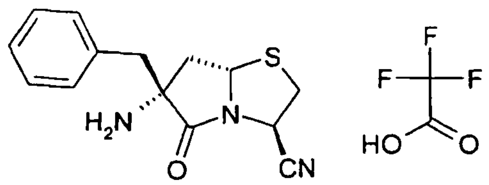

1a) (2S,4S)-4-Benzyl-2-tert-butyl-5-oxo-oxazolidin-3-carbonsäureallylester

Die Mischung bestehend aus 5 g (S)-Phenylalanin und 30,5 ml 1 molarer Natronlauge wird 1 Stde bei RT gerührt und dann im Vakuum bei 60 ˚C zur Trockne eingedampft. Nach Zugabe von 50 ml Toluol wird erneut bei 40˚C zur Trockne eingedampft und der Rückstand in 100 ml n-Pentan suspendiert, mit 7,7 ml Trimethylacetaldehyd versetzt und 15 Stdn am Wasserabscheider unter Rückfluß im Ölbad gerührt. Die flüchtigen Anteile werden im Vakuum bei 40˚C entfernt, der Rückstand je zweimal mit 40 ml Toluol versetzt und im Vakuum zur Trockne einrotiert. Dann wird der Rückstand in 100 ml Methylenchlorid suspendiert, im Eisbad gerührt und mit 3,53 ml Allylchloroformat versetzt. Die Mischung wurde ohne Erneuerung des Eisbads bei allmählichem Erreichen der Raumtemperatur 4 Tage gerührt. Das Produktgemisch wurde zwischen 100 ml Methylenchlorid und 75 ml gesättigter Kochsalzlösung verteilt, der unlösliche Feststoff durch Absaugen entfernt, die organische Phase 3 mal mit gesättigter Natriumbicarbonatlösung (je 30 ml) und dann noch einmal mit gesättigter Kochsalzlösung gewaschen, über Natriumsulfat getrocknet und im Vakuum eingeengt. Der ölige Rückstand wurde säulenchromatografisch (Kieselgel, LM:Heptan.Essigester = 45:5) gereinigt.

Ausbeute: 3,4 g; Fp.: Öl; M+H: 274

1b) (2S,4S)-4-Allyl-4-benzyl-2-tert-butyl-5-oxo-oxazolidin-3-carbonsäureallylester

3,16 g (2S,4S)-4-Benzyl-2-tert-butyl-5-oxo-oxazolidin-3-carbonsäureallylester in 20 ml Tetrahydrofuran werden unter Argon im Trockeneisbad auf-65 ˚C abgekühlt und mit 20,9 ml einer 0,5 molaren Lösung von Kalium-bis-trimethylsilylamid in Toluol tropfenweise versetzt und 30 Minuten bei dieser Temperatur gerührt. Dann werden 0,861 ml Allylbromid zugetropft und die Mischung unter allmählichem Erwärmen auf Raumtemperatur gerührt. Nach dem Stehenlassen über Nacht wurden 15 ml gesättigte Ammoniumchloridlösung zugesetzt und das Produkt mit Essigester extrahiert. Nach dem Waschen der Essigesterlösung mit gesättigter Kochsalzlösung wurde im Vakuum eingedampft und das Produkt säulenchromatografisch gereinigt (Kieselgel, LM: Essigester:Heptan = 5:45).

Ausbeute: 2,0 g; Fp. Öl; M+H: 358

1c) (S)-2-Amino-2-benzyl-pent-4-en-carbonsäure

Die Mischung aus 2 g (2S,4S)-4-Allyl-4-benzyl-2-tert-butyl-5-oxo-oxazolidin-3-carbonsäureallylester, 60 ml Tetrahydrofuran, 4,88 ml Morpholin und 300 mg Tetrakistriphenylphosphin-palladium wird 20 Minuten bei Raumtemperatur gerührt und dann im Vakuum eingeengt. Der Rückstand wird in einer Mischung aus 6 ml Eisessig und 20 ml Wasser 30 Minuten gerührt. Nach dem Abziehen der flüchtigen Anteile im Vakuum bei 40˚C wird das Produkt säulenchromatografisch (Kieselgel, LM: Methylenchlorid : bMethanol = 9:1) gereinigt.

Ausbeute: 680 mg Fp.: 221,2°C M+H: 206

1d) (S)-2-Benzyl-2-tert-butoxycarbonylamino-pent-4-en-carbonsäure

Zur Lösung von 450 mg (S)-2-Amino-2-benzyl-pent-4-en-carbonsäure in 8 ml Dioxan und 6 ml Wasser werden

2,2 ml 1N Natronlauge zugefügt und die Mischung anschließend mit 1,6 g Di-tert.Butyldicarbonat und 350 mg Pottasche versetzt und 7 Stunden bei 40˚C gerührt. Dann wird das Dioxan bei Raumtemperaur im Vakuum abgezogen und die wässrige Phase mit 10 %iger Zitronensäure auf pH = 3-4 gestellt, und mit Essigester (10 ml) extrahiert. Die organische Phase wird über Natriumsulfat getrocknet und eingeengt und der ölige Rückstand roh für die weiteren Umsetzungen verwendet.

Ausbeute: 490 mg Fp.: Öl M+H: 306

1e) (S)-2-Benzyl-2-tert-butoxycarbonylamino-pent-4-en-carbonsäure-methylester

Die Lösung von 490 mg (S)-2-Benzyl-2-tert-butoxycarbonylamino-pent-4-en-carbonsäure in 8 ml Methanol wird portionsweise bei Raumtemperatur unter Umrühren mit insgesamt 5 ml einer 2molaren Lösung von Trimethylsilyldiazomethan in Hexan versetzt. nach beendeter Umsetzung wird der Überschuß an Trimethylsilyldiazomethan durch Zutropfen von Eisessig vernichtet und die flüchtigen Anteile am Rotationsverdampfer bei 40 ˚C abgezogen. Der Rückstand wird über eine Säule (Kieselgel, LM: Methylenchlorid : Methanol = 95:5) gereinigt.

Ausbeute: 410 mg Fp.: Öl M+H: 320

1f) (R)-2-Benzyl-2-tert-butoxycarbonylamino-4-oxo-buttersäure-methylester

Die Lösung von 410 mg (S)-2-Benzyl-2-tert-butoxycarbonylamino-pent-4-en-carbonsäuremethylester in 15 ml Tetrahydrofuran und 5 ml Wasser wird unter Stickstoff mit 1,1 ml einer 2,5%igen Lösung von Osmiumtetroxid in tert.Butanol und danach portionsweise mit 686 mg Natriumperiodat versetzt und über Nacht bei Raumtemperatur gerührt. Die flüchtigen Anteile werden im Vakuum entfernt und der rückstand mit 26 ml 1N Natriumbicarbonatlösung aufgenommen und das Produkt mit Diethylether extrahiert. Nach dem Trocknen und Einengen verbleibt ein Öl.

Ausbeute: 400 mg Fp.: Öl M+H: 322

1g) (3R,6R,7aS)-6-Benzyl-6-tert-butoxycarbonylamino-5-oxo-hexahydro-pyrrolo[2,1-b]thiazol-3-carbonsäure

Die Mischung bestehend aus 400 mg (R)-2-Benzyl-2-tert-butoxycarbonylamino-4-oxobuttersäure-methylester, 5 ml Pyridin und 166 mg L-Cystein wird 4 Stunden unter rückfluß erhitzt. Nach dem Einengen im Vakuum bei 50˚C wird das Produkt durch Säulenchromatografie (Kieselgel, LM: Methylenchlorid : Methanol : Eisessig = 90:10:1) gereinigt.

Ausbeute: 375 mg Fp.: Öl M+H: 393

1h) (3R,6R,7aS)-6-Benzyl-6-tert-butoxycarbonylamino-5-oxo-hexahydro-pyrrolo[2,1-b]thiazol-3- carbonsäuremethylester

Die Lösung von 250 mg (3R,6R,7aS)-6-Benzyl-6-tert-butoxycarbonylamino-5-oxohexahydro-pyrrolo[2,1-b] thiazol-3-carbonsäure in 8 ml Methanol wird portionsweise bei Raumtemperatur unter Umrühren mit insgesamt 1,5 ml einer 2molaren Lösung von Trimethylsilyldiazomethan in Hexan versetzt. Nach beendeter Umsetzung wird der Überschuß an Trimethylsilyldiazomethan durch Zutropfen von Eisessig vernichtet und die flüchtigen Anteile am Rotationsverdampfer bei 40 ˚C abgezogen. Der Rückstand wird über eine Säule (Kieselgel, LM: Essigester : nHeptan = 1 : 1,5) gereinigt.

Ausbeute: 250 mg Fp.: Öl M+H: 407

1i) (3R,6R,7aS)-6-Benzyl-6-tert-butoxycarbonylamino-5-oxo-hexahydro-pyrrolo[2,1-b]thiazol-3- carbonamid

Die Mischung aus 170 mg (3R,6R,7aS)-6-Benzyl-6-tert-butoxycarbonylamino-5-oxohexahydro-pyrrolo[2,1-b] thiazol-3- carbonsäuremethylester und 10 ml einer 7N $NH_3$-Lösung in Methanol wird über Nacht bei Raumtemperatur stehen gelassen. Die flüchtigen Anteile werden im Vakuum abgezogen und der Rückstand mit tert.Butylmethylether verrührt und der Feststoff abgesaugt und bei 40 ˚C im Vakuum getrocknet.

Ausbeute: 130 mg Fp.: 76,5°C M+H: 392

1j) (3R,6R,7aS)-(6-Benzyl-3-cyano-5-oxo-hexahydro-pyrrolo[2,1-b]thiazol-6-yl)-carbaminsäure-tert-butylester

100 mg (3R,6R,7aS)-6-Benzyl-6-tert-butoxycarbonylamino-5-oxo-hexahydro-pyrrolo[2,1-b]thiazol-3- carbonamid werden in 3 ml Dimethylformamid gelöst und mit 28,25 mg Cyanurchlorid bei Raumtemperatur zur Reaktion gebracht. Nach 2 Stunden wurden die flüchtigen Anteile bei 40°C im Vakuum abgezogen und der Rückstand über eine Säule (Kieselgel, LM: Diisopropylether: Methylenchlorid = 100 : 10) gereinigt.

Ausbeute: 50 mg Fp.: Öl M+H: 374

1k) (3R,6R,7aS)-6-Amino-6-benzyl-5-oxo-hexahydro-pyrrolo[2,1-b]thiazol-3-carbonitriltrifluoracetat

Die Mischung bestehend aus 50 mg (3R,6R,7aS)-(6-Benzyl-3-cyano-5-oxo-hexahydropyrrolo[2,1-b]thiazol-6-yl)-carbaminsäure-tert-butylester, 0,1 ml Thioanisol und 1 ml Trifluoressigsäure wird 15 Minuten im Eisbad gerührt. Nach Erreichen der Raumtemperatur wurde im Vakuum bei 40 °C eingeengt und der Rückstand mit Diisopropylether verrührt und das feste Produkt abgesaugt.

Ausbeute: 35 mg Fp.: 209°C M+H: 274

**2) (3R,6R,7aS)-6-Amino-5-oxo-hexahydro-pyrrolo[2,1-b]thiazol-3-carbonitriltrifluoracetat**

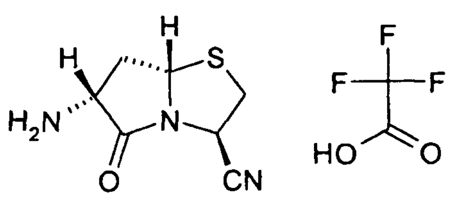

2a) (R)-(5-Hydroxi-2-oxo-tetrahydro-furan-3-yl)-carbaminsäure-tert-butylester

Zur Mischung bestehend aus 5,3 g (R)-N-BOC-Allyl-glycin, 80 ml Tetrahydrofuran, 300 ml Wasser und 16 ml einer 2,5%igen Lösung von Osmiumtetroxid in tert.Butanol werden portionsweise insgesamt 13,4 g Natriumperiodat zugefügt. Nach dem Umrühren über Nacht bei Raumtemperatur wird der Niederschlag abgesaugt und das Filtrat im Vakuum eingeengt. Der Rückstand wird in 500 ml 1N Natriumbikarbonatlösung aufgenommen, mit 2 Portionen a 250 ml Diethylether ausgeschüttelt. Die wässrige Phase wurde mit 2N Salzsäure auf pH = 2 gestellt und nochmals mit Ether extrahiert. Die vereinigten organischen Phasen wurden über Natriumsulfat getrocknet und zur Trockne im Vakuum bei Raumtemperatur eingeengt.

Der Rückstand wurde mit nHeptan verrührt, der feste Anteil an Produkt abgesaugt und das Filtrat eingeengt, wobei ein etwas verunreinigtes öliges Produkt erhalten wurde.

Ausbeute: 1,58 g Fp.: 112,4°C M+H: 218
Ausbeute: 1,2 g Fp.: Öl M+H: 218

2b) (2RS, 4R)-2-((R)-2-tert-Butoxycarbonylamino-2-carboxy-ethyl)-thiazolidin-4-carbonsäure-methylester

Die Mischung von 2,7 g (R)-(5-Hydroxi-2-oxo-tetrahydro-furan-3-yl)-carbaminsäure-tert-butylester, 46 ml Ethanol und 46 ml Wasser wird mit Natriumbikarbonat auf pH = 5 eingestellt, mit 2,2 g L-Cysteinmethylester-hydrochlorid versetzt und 3 Stunden bei Raumtemperatur gerührt. Die flüchtigen Lösemittel wurden im Vakuum bei 40°C abgezogen, die wässrige Phase hat einen pH von etwa 7 und wird mit Essigester ausgeschüttelt, diese organische Phase wird verworfen Die restliche wässrige Lösung wird durch Zugabe von Eisessig auf pH = 5 gestellt und das Produkt mit je 2 Portionen a 20 ml Essigester extrahiert. Die organische Phase wird über Natriumsulfat getrocknet und im Vakuum

eingeengt.

Ausbeute: 1,2 g Fp.: Harz M+H: 335

Aus der wässrigen Phase lassen sich weitere 2,4 g verunreinigtes Produkt durch Einengen und Verrühren mit Methylenchlorid gewinnen.

2c) (3R,6R,7aS)-6-tert-Butoxycarbonylamino-5-oxo-hexahydro-pyrrolo[2,1-b]thiazol-3-carbonsäure-methylester und

2d) (3R, 6R, 7aR)-6-tert-Butoxycarbonylamino-5-oxo-hexahydro-pyrrolo[2,1-b]thiazol-3-carbonsäure-methylester

Die Mischung bestehend aus 3,6 g (2RS, 4R)-2-((2R)-2-tert-Butoxycarbonylamino-2-carboxy-ethyl)-thiazolidin-4-carbonsäure-methylester, 62 ml Methylenchlorid, 3,3 g 2-Chlormethylpyridiniumiodid und 3,6 ml Triethylamin wird 2 Tage bei Raumtemperatur stehen gelassen. Diese Mischung wird mit 50 ml 1N Zitronensäurelösung und anschließend mit 30 ml 1N Natriumbikarbonatlösung gewaschen, über Natriumsulfat getrocknet und im Vakuum eingeengt. Aus dem öligen Rückstand werden durch Säulenchromatografie (Kieselgel, LM: Essigester : nHeptan = 1 : 1) zwei Produkte erhalten.
(3R,6R,7aS)-Isomer, unpolare Verbindung 2c:

Ausbeute: 1080 mg Fp.: Harz M+H: 317

(3R,6R,7aR)-Isomer, polare Verbindung 2d:

Ausbeute: 820 mg Fp.: Harz M+H: 317

2e) (3R,6R,7aS)-(3-Carbamoyl-5-oxo-hexahydro-pyrrolo[2,1-b]thiazol-6-yl)-carbaminsäure-tert-butylester

850 mg von (3R,6R,8S) 6-tert-Butoxycarbonylamino-5-oxo-hexahydro-pyrrolo[2,1-b]thiazol-3-carbonsäure-methylester (2c) wurden in 30 ml 7 N $NH_3$-Lösung in Methanol gelöst und über Nacht bei Raumtemperatur stehen gelassen. Nach dem Einengen im Vakuum bei 40 ˚C wurde das Produkt durch Verrühren in tert.Butyl-methylether und Absaugen gereinigt, und im Vakuum getrocknet.

Ausbeute: 250 mg Fp.: 189,4˚C M+H: 302

2f) (3R,6R,7aR)-(3-Carbamoyl-5-oxo-hexahydro-pyrrolo[2,1-b]thiazol-6-yl)-carbaminsäure-tert-butylester

wurde analog zur Synthese von 2e) ausgehend von 540 mg (3R,6R,8R) 6-tert-Butoxycarbonylamino-5-oxo-hexahydro-pyrrolo[2,1-b]thiazol-3-carbonsäure-methylester (2d) erhalten.

Ausbeute: 250 mg Fp.: Harz M+H: 317

2g) (3R,6R,7aS)-(3-Cyano-5-oxo-hexahydro-pyrrolo[2,1-b]thiazol-6-yl)-carbaminsäure-tert-butylester

Die Lösung von 538 mg (3R,6R,7aS)-(3-Carbamoyl-5-oxo-hexahydro-pyrrolo[2,1-b]thiazol-6-yl)-carbaminsäure-tert-butylester in 35 ml Tetrahydrofuran wurde im Eisbad abgekühlt, mit 0,75 ml Triethylamin und 0,37 ml Trifluoressigsäureanhydrid versetzt und über Nacht bei Raumtemperatur gerührt. Die flüchtigen Anteile wurden im Vakuum bei 30 ˚C abgezogen und der ölige Rückstand säulenchromatografisch (Kieselgel, LM: Essigester : nHeptan = 1 : 1) gereinigt.

Ausbeute: 320 mg Fp.: 191,2˚C M+H: 284

2h) (3R, 6R, 7aR)-(3-Cyano-5-oxo-hexahydro-pyrrolo[2,1-b]thiazol-6-yl)-carbaminsäure-tert-butylester

wurde analog zur Synthese von 2g) ausgehend von 238 mg (3R,6R,7aR)-(3-Carbamoyl-5-oxo-hexahydro-pyrrolo[2,1-b]thiazol-6-yl)-carbaminsäure-tert-butylester erhalten.

Ausbeute: 130 mg Fp.: Öl M+H: 284

2i) (3R,6R,7aS)-(6-Amino-5-oxo-hexahydro-pyrrolo[2,1-b]thiazol-3-carbonitril-trifluoracetat

Die Lösung von 300 mg (3R,6R,7aS)-(3-Cyano-5-oxo-hexahydro-pyrrolo[2,1-b]thiazol-6-yl)-carbaminsäure-tert-butylester in 10 ml Methylenchlorid wird im Eisbad gekühlt, mit 1,5 ml Trifluoressigsäure versetzt und 2 Stunden bei allmählichem Erreichen der Raumtemperatur gerührt. Die flüchtigen Anteile wurden im Vakuum bei 40˚C entfernt und der Rückstand mit Diisopropylether verrührt und der Feststoff abgesaugt.

Ausbeute: 280 mg Fp.: 180,3˚C M+H: 184

**3) (3R, 6R, 7aR)-(6-Amino-5-oxo-hexahydro-pyrrolo[2,1-b]thiazol-3-carbonitriltrifluoracetat**

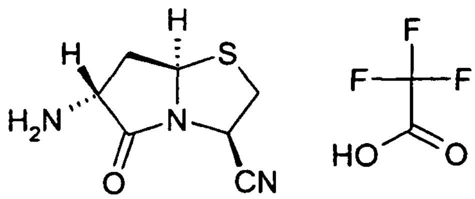

wurde analog zur Synthese von 2i) ausgehend von 130 mg (3R, 6R, 7aR)-(3-Cyano-5-oxohexahydro-pyrrolo [2,1-b]thiazol-6-yl)-carbaminsäure-tert-butylester erhalten.

Ausbeute: 105 mg Fp.: 173,5˚C M+H: 184

**4) (3R,6R,7aS)-5-Oxo-6-(3-pbenyl-propylamino)-hexahydro-pyrrolo[2,1-b]thiazol-3-carbonitril**

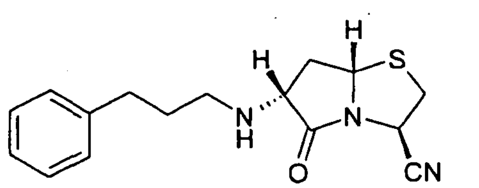

30 mg (3R,6R,7aS)-(6-Amino-5-oxo-hexahydro-pyrrolo[2,1-b]thiazol-3-carbonitriltrifluoracetat, 0,0132 ml 3-Phenyl-propionaldehyd und 0,115 ml Eisessig wurden in 1,5 ml Methylenchlorid 20 minuten auf 40˚C erwärmt. Dann wurde auf Raumtemperatur abgekühlt, 8,3 mg Natriumacetat, 0,5 ml Dimethoxiethan und 27,8 mg Natriumtriacetoxiborhydrid zugefügt und über Nacht bei RT gerührt. Nach dem Einengen der Mischung wurde der Rückstand säulenchromatografisch (Kieselgel, LM: Essigester : nHeptan = 8:1) gereinigt.

Ausbeute: 13 mg Fp.: Öl M+H: 302

**5) (3R,6R,7aS)-6-Benzylamino-5-oxo-hexahydro-pyrrolo[2,1-b]thiazol-3-carbonitriltrifluoracetat**

20 mg (3R,6R,7aS)-6-Amino-5-oxo-hexahydro-pyrrolo[2,1-b]thiazol-3-carbonitriltrifluoracetat wurden in 1 ml 1,2-Dichlorethan gelöst. Nacheinander gab man 2 µl Essigsäure, 7 µl Benzaldehyd und 29 mg Natriumtriacetoxyborhydrid zu. Man erwärmte für 2 h auf 45 ˚C. Danach wurde das Lösungsmittel im Vakuum entfernt. Der Rückstand wurde durch präparative HPLC gereinigt (Merck RP18, Acetonitril/Wasser (0,5 % Trifluoressigsäure), linearer Gradient (0->80 % Acetonitril)).

Ausbeute: 5 mg (3R,6R,(S)-6-Benzylamino-5-oxo-hexahydro-pyrrolo[2,1-b]thiazol-3-carbonitril-trifluoracetat
MS: 274 (M+H)

**6) (3R,6R,7aS)-6-Cyclopentylamino-5-oxo-hexahydro-pyrrolo[2,1-b]thiazol-3-carbonitril-trifluoracetat**

20 mg (3R,6R,7aS)-6-Amino-5-oxo-hexahydro-pyrrolo[2,1-b]thiazol-3-carbonitriltrifluoracetat wurden in 1 ml 1,2-Dichlorethan gelöst. Nacheinander gab man 2 µl Essigsäure, 6 µl Cyclopentanon und 29 mg Natriumtriacetoxyborhydrid zu. Man erwärmte für 2 h auf 45 ˚C. Danach wurde das Lösungsmittel im Vakuum entfernt. Der Rückstand wurde durch präparative HPLC gereinigt (Merck RP18, Acetonitril/Wasser (0,5 % Trifluoressigsäure), linearer Gradient (0->80 % Acetonitril)).
Ausbeute: 15 mg 6-Cyclopentylamino-5-oxo-hexahydro-pyrrolo[2,1-b]thiazol-3-carbonitriltrifluoracetat
MS: 252 (M+H)

**7) (3R,6R,7aS)-6-Amino-6-isobutyl-5-oxo-hexahydro-pyrrolo[2,1-b]thiazol-3-carbonitril-trifluoracetat**

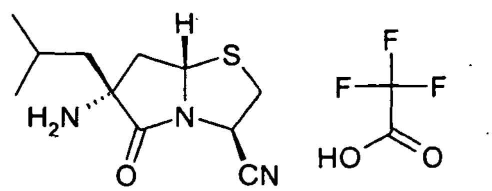

7a) (3S,5S,6R)-3-Allyl-2-oxo-5,6-diphenyl-morpholin-4-carbonsäure-tert-butylester

10 g (5S,6R)-2-Oxo-5,6-diphenyl-morpholin-4-carbonsäure-tert-butylester wurden in 200 ml THF gelöst und auf-78˚C abgekühlt. Man tropfte 63 ml einer 0,5 M Lösung von KHMDS in Toluol zu, ließ 10 Min. nachrühren und tropfte dann 3,7 ml Allylbromid zu. Innerhalb von 3 h ließ man von -78˚C auf -20˚C erwärmen und beendete die Reaktion dann durch Zugabe von 300 ml einer gesättigten Ammoniumchlorid-Lösung. Man gab 200 ml Essigester hinzu und trennte die Phasen, Die wäßrige Phase wurde 3 mal mit je 100 ml Essigester extrahiert und die vereinigten organischen Phasen wurden mit $MgSO_4$ getrocknet. Die Lösungsmittel wurden im Vakuum entfernt und er Rückstand aus Diisopropylether ausgerührt.
Ausbeute: 7,3 g (35,5S,6R)-3-Allyl-2-oxo-5,6-diphenyl-morpholin-4-carbonsäure-tert-butylester
MS: 338 (M+H-tBu)

7b) (3R,5S,6R)-3-Allyl-3-isobutyl-2-oxo-5,6-diphenyl-morpholin-4-carbonsäure-tert-butylester

1 g (3R,5S,6R)-3-Allyl-2-oxo-5,6-diphenyl-morpholin-4-carbonsäure-tert-butylester wurde in 20 ml THF gelöst. Man gab 3 ml 15-Krone-5 hinzu und kühlte auf-78˚C ab. Danach tropfte man 1,4 ml einer 2 M Lösung von NaHMDS in THF zu. Man ließ 10 Min. rühren und tropfte dann 300 µl Isobutyliodid zu. Innerhalb von 6 h ließ man auf 0˚C erwärmen und beendete dann die Reaktion durch Zugabe von 50 ml gesättigter Ammoniumchlorid-Lösung. Die wäßr.Phase wurde 3 mal mit je 50 ml Essigester extrahiert und die vereinigten organischen Phasen wurden mit $MgSO_4$ getrocknet. Die Lösungsmittel wurden im Vakuum entfernt und der Rückstand über Kieselgel mit Heptan/Essigester 10:1 chromatografiert. Ausbeute: 620 mg (3R,5S,6R)-3-Allyl-3-isobutyl-2-oxo-5,6-diphenyl-morpholin-4-carbonsäure-tert-butylester
MS: 394 (M+H-tBu)

7c) (R)-2-tert-Butoxycarbonylamino-2-isobutyl-pent-4-ensäure

60 ml Ammoniak wurden bei -78˚C einkondensiert. Anschließend wurden 624 mg Natrium in 2 Portionen im Abstand von 10 Min. zugegeben. Man ließ 20 Min. nachrühren. Dazu tropfte man bei - 60˚C eine Lösung von (3R,5S, 6R)-3-Allyl-3-isobutyl-2-oxo-5,6-diphenylmorpholin-4-carbonsäure tert-butylester 1,22 g und 1,6 ml Ethanol in 20 ml THF. Man ließ 1,5 h bei -45˚C rühren und beendete die Reaktion durch Zugabe von soviel festem Ammoniumchlorid, bis die blaue Farbe verschwindet. Danach lässt man das Ammoniak abdampfen und gibt zum Rückstand 50 ml Wasser. Das THF wird im Vakuum entfernt. Die wäßrige mit 1 N HCl auf pH 2 eingestellt und 3 mal mit je 60 ml Essigester extrahiert. Die vereinigten organischen Phasen werden mit $MgSO_4$ getrocknet. Die Lösungsmittel werden im Vakuum entfernt und der Rückstand durch Chromatografie an Kieselgel gereinigt. Ausbeute: 508 mg (R)-2-tert-Butoxycarbonylamino-2-isobutyl-pent-4-ensäure
MS: 172 (M+H-Boc)

7d) (3R,6R,7aS)-6-tert-Butoxycarbonylamino-6-isobutyl-5-oxo-hexahydro-pyrrolo[2,1-b]thiazol-3-carbonsäure-methylester und

7e) (3R,6R,7aR)-6-tert-Butoxycarbonylamino-6-isobutyl-5-oxo-hexahydro-pyrrolo[2,1-b]thiazol-3-carbonsäure-methylester

500 mg (R)-2-tert-Butoxycarbonylamino-2-isobutyl-pent-4-ensäure wurden in 20 ml THF und 5 ml Wasser gelöst. Dazu tropfte man eine Lösung von 24 mg Osmiumtetroxid in 2 ml THF, ließ 5 Min. nachrühren und gab dann 985 mg Natriumperiodat hinzu. Man ließ 3 h rühren und filtrierte dann über Kieselgur. Der Filterkuchen wurde 2 mal mit je 10 ml THF gewaschen. Das THF wurde im Vakuum entfernt und der Rückstand zwischen 60 ml Essigester und 30 ml Wasser verteilt. Der pH-Wert wurde mit 1 N HCl Lösung auf 2 eingestellt. Die Phasen wurden getrennt und die wäßr. Phase 2 mal mit je 20 ml Essigester extrahiert. Die vereinigten organischen Phasen wurden mit $MgSO_4$ getrocknet und die Lösungsmittel im Vakuum entfernt. Der Rückstand wurde an Kieselgel mit 5 % Methanol in Dichlormethan chromatographiert. Dabei wurden 410 mg des zyklischen Aldehydderivats erhalten, die in 10 ml Dichlormethan gelöst wurden. Unter Rühren gab man 230 μl Triethylamin und 258 mg L-Cysteinmethylester-hydrochlorid hinzu. Man ließ 2,5 h bei Raumtemperatur rühren und gab dann 317 mg 1-(3-Dimethylaminopropyl)-3-ethyl-carbodiimid-hydrochlorid zu. Nach 14 stündigem Rühren bei Raumtemperatur wurde die Reaktion mit 80 ml Dichlormethan verdünnt und 2 mal mit je 80 ml Wasser gewaschen. Die organische Phase wurde mit $MgSO_4$ getrocknet und die Lösungsmittel wurden im Vakuum entfernt. Die Diastereomeren Verbindungen (3R,6R;7aS)-6-tert-Butoxycarbonylamino-6-isobutyl-5-oxo-hexahydro-pyrrolo[2,1-b]thiazol-3-carbonsäuremethylester und 3R,6R,7R-6-tert-Butoxycarbonylamino-6-isobutyl-5-oxo-hexahydro-pyrrolo[2,1-b]thiazol-3-carbonsäure-methylester wurden chromatografisch an Kieselgel getrennt.
Ausbeute: 75 mg 7d) (3R,6R,7aS)-6-tert-Butoxycarbonylamino-6-isobutyl-5-oxohexahydro-pyrrolo[2,1-b]thiazol-3-carbonsäure-methylester
MS: 373 (M+H)
und
304 mg 7e) (3R,6R,7aR)-6-tert-Butoxycarbonylamino-6-isobutyl-5-oxo-hexahydropyrrolo[2,1-b]thiazol-3-carbonsäure-methylester
MS: 373 (M+H)

7f) (3R,6R,7aS)-6-tert-Butoxycarbonylamino-6-isobutyl-5-oxo-hexahydro-pyrrolo[2,1-b]thiazol-3-carbonsäureamid

71 mg (3R,6R,7aS)-6-tert-Butoxycarbonylamino-6-isobutyl-5-oxo-hexahydro-pyrrolo[2,1-b]thiazol-3-carbonsäure-methylester wurden in 10 ml Ammoniak gesättigtem Methanol gelöst und auf 0°C gekühlt. Man ließ langsam auf Raumtemperatur kommen und 14 h rühren. Die Lösungsmittel wurden im Vakuum entfernt. Das Rohprodukt wurde ohne weitere Reinigung in der nächsten Umsetzung eingesetzt.
MS: 302 (M+H-tBu)

7g) (3R,6R,7aS)-(3-Cyano-6-isobutyl-5-oxo-hexahydro-pyrrolo[2,1-b]thiazol-6-yl)-carbaminsäure-tert-butylester

Das Rohprodukt aus 2e wurde in 5 ml THF gelöst. Dazu gab man 63 μl Triethylamin und 29 μl Trifluoressigsäureanhydrid. Man ließ 3 h bei Raumtemperatur rühren und verteilte dann zwischen 60 ml Wasser und 60 ml Essigester. Die wäßr.Phase wurde 2 mal mit je 50 ml Essigester extrahiert und die vereinigten organischen Phasen wurden mit $MgSO_4$ getrocknet. Die Lösungsmittel wurden im Vakuum entfernt und der Rückstand durch Chromatografie an Kieselgel gereinigt.
Ausbeute: 57 mg (3R,6R,7aS)-(3-Cyano-6-isobutyl-5-oxo-hexahydro-pyrrolo[2,1-b]thiazol-6-yl)-carbaminsäure-tert-butylester
MS: 284 (M+H-tBu)

7h) (3R,6R,7aS)-6-Amino-6-isobutyl-5-oxo-hexahydro-pyrrolo[2,1-b]thiazol-3-carbonitril-trifluoracetat

53 mg (3R,6R,7aS)-(3-Cyano-6-isobutyl-5-oxo-hexahydro-pyrrolo[2,1-b]thiazol-6-yl)-carbaminsäure-tert-butylester wurden bei 0°C für 30 Min. mit 3 ml Trifluoressigsäure und 600 μl Thioanisol umgesetzt. Danach wurden die Lösungsmittel im Vakuum entfernt und der Rückstand in Diethylether verrührt, abgesaugt und getrocknet.
Ausbeute: 39 mg (3R,6R,7aS)-6-Amino-6-isobutyl-5-oxo-hexahydro-pyrrolo[2,1-b]thiazol-3-carbonitril-trifluoracetat
MS: 240 (M+H)

7i) (3R,6R,7aR)-6-Amino-6-isobutyl-5-oxo-hexahydro-pyrrolo[2,1-b]thiazol-3-carbonitril-trifluoracetat

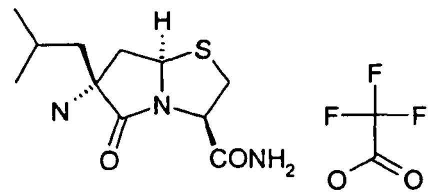

wurde analog den Beispielen 7f), 7g) und 7h) ausgehend vom Diastereomeren 7e) erhalten. MS: 240 (M+H)

**8) (3R,6S,7aS)-6-Amino-6-hydroximethyl-5-oxo-hexahydro-pyrrolo[2,1-b]triazol-3-carbonitril-trifluoracetat**

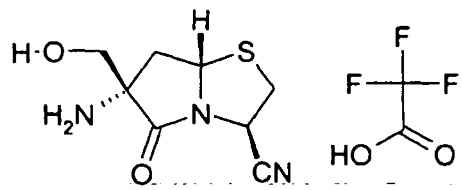

8a) 4-Allyl-2,2-dimethyl-oxazolidin-3,4-dicarbonsäure-3-tert-butylester-4-methylester

10 g 2,2-Dimethyl-oxazolidin-3,4-dicarbonsäure-3-tert-butylester-4-methylester wurden in 270 ml THF gelöst. Man kühlte auf -78˚C und tropfte dann 100 ml einer 0,5 M Lösung von KHMDS in Toluol zu. Nach Rühren für 30 Min. bei -78˚C wurden 5.4 ml Allylbromid zugetropft. Man ließ langsam auf Raumtemperatur kommen und 14 h rühren. Danach wurde die Reaktion durch Zugabe von 400 ml gesättigter Ammoniumchlorid Lösung beendet. Das THF wurde im Vakuum entfernt und die wäßr. Phase 3 mal mit je 150 ml Essigester extrahiert. Die vereinigten organische Phasen wurden 1 mal mit 300 ml gesättigter Natriumchlorid Lösung gewaschen und mit $MgSO_4$ getrocknet. Die Lösungsmittel wurden im Vakuum entfernt und der Rückstand an Kieselgel chromatografiert.
Ausbeute: 5,58 g 4-Allyl-2,2-dimethyl-oxazolidin-3,4-dicarbonsäure-3-tert-butylester 4-methyl ester
MS: 322 (M+Na)

8b) 8-Hydroxi-2,2-dimethyl-6-oxo-3,7-dioxa-1-aza-spiro[4.4]nonan-1-carbonsäure-tert-butylester

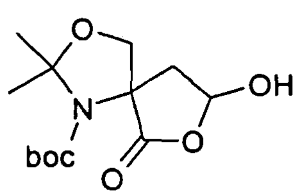

1.7 g 4-Allyl-2,2-dimethyl-oxazolidin-3,4-dicarbonsäure-3-tert-butylester-4-methylester wurden in 20 ml Methanol gelöst und dazu 8 ml 1 N NaOH gegeben. Man erhitzte für 3 h auf 60˚C ließ abkühlen und entfernte das Methanol im Vakuum. Der pH-Wert der wäßr. Phase wurde mit 1 N HCl auf 2 eingestellt und es wurde 3 mal mit je 50 ml Dichlormethan extrahiert. Die vereinigten organische Phasen wurden mit $MgSO_4$ getrocknet und das Lösungsmittel im Vakuum entfernt. Der Rückstand wurde in 80 ml Dichlormethan und 10 ml Methanol aufgenommen. Man kühlte auf -78˚C und leitete so lange Ozon durch die Lösung, bis diese eine leicht blaue Farbe annahm. Danach wurde Argon durchgeleitet, bis die blaue Farbe vollständig verschwunden war. Man gab 3 ml Dimethylsulfid zu und ließ über Nacht auf Raumtemperatur kommen. Danach wurden die Lösungsmittel im Vakuum entfernt. Das so erhaltene Rohprodukt

wurde direkt in der nächsten Umsetzung eingesetzt.

8c) Acetonid von (3R,6S,7aS)-6-(tert. butyloxycarbonylamino)-6-hydroximethyl-5-oxo-hexahydro-pyrrolo[2,1-b)thiazol-3-carbonsäureamid

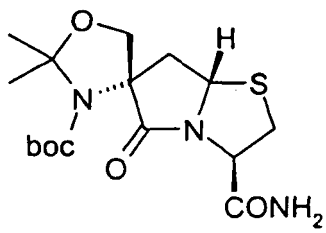

Das Rohprodukt aus 8b wurde in 40 ml Ethanol gelöst. Man kühlte auf 0˚C und gab unter Argon 500 mg Natriumhydrogencarbonat in 5 ml Wasser sowie 1 g L-Cysteinmethylester Hydrochlorid zu. Der pH-Wert der Lösung wurde mit 1% Natriumhydrogencarbonat Lösung in Wasser auf 6,5 eingestellt. Man ließ langsam auf Raumtemperatur kommen und 14 h rühren. Danach engt man das Volumen auf etwa die Hälfte im Vakuum ein. Der pH-Wert wurde mit 1 N HCl auf 6 eingestellt und die wäßr. Phase mit 70 ml Essigester extrahiert. Dieser Vorgang wurde dreimal wiederholt. Die vereinigten organische Phasen wurden mit $MgSO_4$ getrocknet. Danach wurden die Lösungsmittel im Vakuum entfernt. Der Rückstand wurde in 400 ml 1,2-Dichlorethan aufgenommen und 1,7 g 2-Chlor-1-methylpyridiniumiodid sowie 1,9 ml Triethylamin zugegeben. Man erwärmte für 4 h auf 50˚C. Danach wird 1 mal mit 150 ml 1 N HCl und 150 ml gesättigter Natriumhydrogencarbonat Lösung gewaschen. Die organische Phase wird mit $MgSO_4$ getrocknet. Die Lösungsmittel werden im Vakuum und der Rückstand in 60 ml Ammoniak gesättigtem Methanol aufgenommen. Man ließ 18 h rühren, entfernte die Lösungsmittel im Vakuum und chromatografierte den Rückstand an Kieselgel.
Ausbeute: 230 mg der Verbindung obiger Formel
MS: 316 (M+H-tBu)

8d) Acetonid von (3R,6S,7aS)-6-(tert.-butyloxycarbonylamino)-6-hydroximethyl-5-oxohexahydro-pyrrolo[2,1-b)thiazol-3-carbonitril

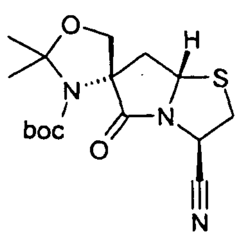

200 mg der Verbindung 8c wurden in 5 ml THF gelöst. Man gab nacheinander 188 µl Triethylamin und 114 µl Trifluoressigsäureanhydrid zu. Danach ließ man 6 h bei Raumtemperatur rühren. Die Lösung wurde zwischen 60 ml gesättigter Natriumhydrogencarbonat Lösung und 60 ml Essigester vetreilt. Die wäßr. Phase wurde 2 mal mit je 30 ml Essigester extrahiert und die vereinigten organischen Phasen wurden mit $MgSO_4$ getrocknet. Die Lösungsmittel wurden im Vakuum entfernt und der Rückstand durch Chromatografie an Kieselgel gereinigt.
Ausbeute: 90 mg des Acetonids obiger Formel
MS: 240

8e) (3R,6S,7aS)-6-Amino-6-hydroximethyl-5-oxo-hexahydro-pyrrolo[2,1-b]thiazol-3-carbonitril-trifluoracetat

5 mg der Verbindung 8d wurden in 2 ml Dichlormethan gelöst und auf 0˚C gekühlt. Man gab 300 µl TFA zu und ließ 4 h bei 0˚C rühren. Danach wurden die Lösungsmittel im Vakuum entfernt und der Rückstand aus Diethylether ausgerührt.
Ausbeute: 5 mg

MS: 214 (M+H)

**9) (3R,6S,7aS)-6-Amino-5-oxo-6-piperidin-1-yl-methyl)-hexahydro-pyrrolo[2,1-b]thiazol-3-carbonitril-bi-strifluoracetat**

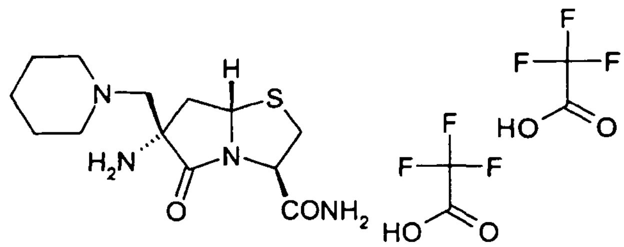

9a) (3S,5S,6R)-3-Allyl-3-benzyloxymethyl-2-oxo-5,6-diphenyl-morpholin-4-carbonsäure-tert-butylester

5,7 g 3S,5S,6R-3-Allyl-2-oxo-5,6-diphenyl-morpholin-4-carbonsäure-tert-butylester wurden in 150 ml THF gelöst. Man gab 8,6 ml 15-Krone-5 hinzu und kühlte auf -78°C ab. Danach tropfte man 8 ml einer 2 M Lösung von NaHMDS in THF zu. Man ließ 10 Min. rühren und tropfte dann 6,7 ml Benzyloxymethylchlorid zu. Innerhalb von 6 h ließ man auf 0°C erwärmen und beendete dann die Reaktion durch Zugabe von 200 ml gesättigter Ammoniumchlorid-Lösung. Die wäßr. Phase wurde 3 mal mit je 150 ml Essigester extrahiert und die vereinigten organischen Phasen wurden mit $MgSO_4$ getrocknet. Die Lösungsmittel wurden im Vakuum entfernt und der Rückstand über Kieselgel mit Heptan/Essigester 10:1 chromatografiert.
Ausbeute: 620 mg (3S,5S,6R)-3-Allyl-3-benzyloxymethyl-2-oxo-5,6-diphenyl-morpholin-4-carbonsäure-tert-butylester
MS: 458 (M+H-tBu)

9b) (S)-2-tert-Butoxycarbonylamino-2-hydroximethyl-pent-4-ensäure

450 ml Ammoniak wurden bei -78°C einkondensiert. Man gab 4,4 g Natrium in 4 Portionen im Abstand von je 10 Min. zu und ließ 20 Min nachrühren. Dazu tropfte man bei - 60°C eine Lösung von 9,8 g (3S,5S,6R)-3-Allyl-3-benzyloxymethyl-2-oxo-5,6-diphenylmorpholin-4-carbonsäure-tert-butylester und 11,2 ml Ethanol in 150 ml THF. Man ließ 1,5 h bei -45°C rühren und beendete die Reaktion durch Zugabe von soviel festem Ammoniumchlorid, bis die blaue Farbe verschwand. Danach ließ man das Ammoniak abdampfen und gab zum Rückstand 400 ml Wasser. Das THF wurde im Vakuum entfernt. Die wäßr. wurde mit 1 N HCl auf pH 2 eingestellt und 3 mal mit je 200 ml Essigester extrahiert. Die vereinigten organischen Phasen wurden mit $MgSO_4$ getrocknet. Die Lösungsmittel wurden im Vakuum entfernt und der Rückstand durch Chromatografie an Kieselgel gereinigt.
Ausbeute: 2,6 g (S)-2-tert-Butoxycarbonylamino-2-hydroximethyl-pent-4-ensäure
MS: 146 (M+H-Boc)

9c) (S)-2-tert-Butoxycarbonylamino-2-(tert-butyl-dimethyl-silanyloxymethyl)-pent-4-ensäure

1,3 g (S)-2-tert-Butoxycarbonylamino-2-hydroximethyl-pent-4-ensäure wurden in 10 ml Dimethylformamid gelöst. Dazu gab man 1,44 g Imidazol und 1,7 g tert-Butyl-dimethylsilylchlorid. Man ließ 14 h bei Raumtemperatur rühren und verteilte dann zwischen 150 ml Wasser und 200 ml Diethylether. Die wäßr. Phase wurde 2 mal mit je 100 ml Diethylether extrahiert und die vereinigten organische Phasen mit $MgSO_4$ getrocknet. Die Lösungsmittel wurden im Vakuum entfernt und der Rückstand in 20 ml THF und 60 ml Methanol aufgenommen. Man gab 11 ml einer 1 M Kaliumcarbonat Lösung hinzu und ließ 2 h bei Raumtemperatur rühren. Danach wurden 150 ml einer gesättigten Natriumchlorid Lösung zugegeben und der pH-Wert der Lösung mit 1 M HCl Lösung auf 2 eingestellt. Die wäßr. Phase wurde 3 mal mit je 100 ml Essigester extrahiert und die vereinigten organische Phasen wurden mit $MgSO_4$ getrocknet. Die Lösungsmittel wurden im Vakuum entfernt und das Rohprodukt ohne weitere Reinigung in der nächsten Umsetzung eingesetzt. Ausbeute: 1.8 g (S)-2-tert-Butoxycarbonylamino-2-(tert-butyl-dimethyl-silanyloxymethyl)-pent-4-ensäure
MS: 260 (M+H-Boc)

9d) (3R,65,7aS)-6-tert-Butoxycarbonylamino-6-(tert-butyl-dimethyl-silanyloxymethyl)-5-oxo-hexahydro-pyrrolo[2,1-b] thiazol-3-carbonsäure-methylester und

9e) (3R,6S,7aR)-6-tert-Butoxycarbonylamino-6-(tert-butyl-dimethyl-silanyloxymethyl)-5-oxo-hexahydro-pyrrolo[2,1-b] thiazol-3-carbonsäure-methylester

3,7 g (S)-2-tert-Butoxycarbonylamino-2-(tert-butyl-dimethyl-silanyloxymethyl)-pent-4-ensäure wurden in 1220 ml THF und 30 ml Wasser gelöst. Dazu wurden 131 mg Osmiumtetroxid gegeben und man ließ 5 Min. nachrühren. Dann wurden 5,5 g Natriumperiodat zugegeben. Man ließ 2 h bei Raumtemperatur rühren und filtrierte dann über Kieselgur. Der Filterkuchen wurde 2 mal mit je 30 ml THF gewaschen. Danach wurde das THF im Vakuum entfernt und der Rückstand zwischen 150 ml Diethylether und 100 ml gesättigter Natriumhydrogencarbonat Lösung verteilt. Die wäßr. Phase wurde mit 1 N HCl auf pH 1 eingestellt und 3 mal mit je 100 ml Essigester extrahiert. Die vereinigten organische Phasen wurden mit $MgSO_4$ getrocknet. Die Lösungsmittel wurden im Vakuum entfernt und der Rückstand in 40 ml Ethanol aufgenommen. Man kühlte auf-20°C und gab unter Argon 864 mg Natriumhydrogencarbonat in 40 ml Wasser gelöst hinzu. Danach wurden 1,77 g L-Cysteinmethylester Hydrochlorid zugegeben und der pH-Wert der Lösung mit 1%-iger Natriumhydrogencarbonat Lösung auf 6,5 eingestellt. Man ließ 14 h bei Raumtemperatur rühren. Anschließend wurde Lösung im Vakuum auf das halbe Volumen eingeengt. Der pH-Wert wurde mit 1 N HCl auf 6 eingestellt und die Lösuing mit 100 ml Essigester extrahiert. Das Einstellen des pH-Werts auf 6 und die Extraktion mit Essigester wurde 3 mal wiederholt. Die vereinigten organischen Phasen wurden mit MgS04 getrocknet. Die Lösungsmittel wurden im Vakuum entfernt und der Rückstand in 200 ml THF aufgenommen. Man gab 3,2 ml Triethylamin und 2,9 g 1-(3-Dimethylaminopropyl)-3-ethylcarbodiimid Hydrochlorid zu. Danach wurde für 1 h bei Raumtemperatur und 3 h bei 50°C gerührt. Das Lösungsmittel wurde im Vakuum entfernt und der Rückstand zwischen 100 ml 1 N HCl und 100 ml Essigester verteilt. Die wäßr. Phase wurde 2 mal mit je 50 ml Essigester extrahiert und die vereinigten organischen Phasen mit $MgSO_4$ getrocknet. Die Lösungsmittel wurden im Vakuum entfernt und der Rückstand chromatografisch an Kieselgel gereinigt.
Ausbeute: 340 mg 9d) (3R,6S,7aS)-6-tert-Butoxycarbonylamino-6-(tert-butyl-dimethylsilanyloxymethyl)-5-oxo-hexahydro-pyrrolo[2,1-b]thiazol-3-carbonsäure-methylester MS: 405 (M+H-tBu)
und
1,1 g 9e) (3R,6S,7aR)-6-tert-Butoxycarbonylamino-6-(tert-butyl-dimethylsilanyloxymethyl)-5-oxo-hexahydro-pyrrolo[2,1-b]thiazol-3-carbonsäure-methylester
MS: 405 (M+H-tBu)

9f) (3R,6S,7aS)-6-tert-Butoxycarbonylamino-6-hydroximethyl-5-oxo-hexahydropyrrolo[2,1-b]thiazol-3-carbonsäure-methylester

337 mg (3R,6S,7aS)-6-tert-Butoxycarbonylamino-6-(tert-butyl-dimethyl-silanyloxymethyl)-5-oxo-hexahydro-pyrrolo[2,1-b]thiazol-3-carbonsäure-methylester wurden in 10 ml THF gelöst und auf 0°C gekühlt. Man tropfte eine 1 M Lösung von Tetrabutylammoniumfluorid hinzu und ließ innerhalb von 2 h auf Raumtemperatur kommen. Danach verteilte man zwischen 120 ml gesättigter Ammoniumchlorid Lösung und 120 ml Essigester. Die wäßr. Phase wurde 2 mal mit je 40 ml Essigester extrahiert. Die vereinigten organischen Phasen wurden 1 mal mit 80 ml gesättigter Natriumchlorid Lösung gewaschen und mit $MgSO_4$ getrocknet. Die Lösungsmittel wurden im Vakuum entfernt und der Rückstand durch Chromatografie an Kieselgel gereinigt.
Ausbeute: 180 mg (3R,6S,7aS)-6-tert-Butoxycarbonylamino-6-hydroximethyl-5-oxohexahydro-pyrrolo[2,1-b]thiazol-3-carbonsäure-methylester
MS: 291 (M+H-tBu)

9g) (3R,6S,7aS)-6-tert-Butoxycarbonylamino-5-oxo-6-piperidin-1-yl-methyl)-hexahydropyrrolo[2,1-b]thiazol-3-carbonsäure-methylester

170 mg (3R,6S,7aS)-6-tert-Butoxycarbonylamino-6-hydroximethyl-5-oxo-hexahydropyrrolo[2,1-b]thiazol-3-carbonsäure-methylester wurden in 5 ml Dichlormethan gelöst. Man gab 400 µl Pyridin ind 230 mg Dess-Martin-Periodinan hinzu. Danach ließ man 2 h bei Raumtemperatur rühren. Die Lösung wurde zwischen 20 ml 0,5 M HCl un 20 ml Dichlormethan verteilt. Die wäßr. Phase wurde 2 mal mit je 10 ml Dichlormethan extrahiert und die vereinigten organischen Phasen wurden mit $MgSO_4$ getrocknet. Das Lösungsmittel wurde im Vakuum entfernt und der Rückstand in 5 ml 1,2-Dichlorethan aufgenommen. Man gab nacheinander 49 µl Piperidin, 15 µl Essigsäure und 209 mg Natriumtriacetoxyborhydrid hinzu. Danach ließ man 14 h bei Raumtemperatur rühren. Die Lösung wurde zwischen 30 ml gesättigter Natriumhydrogencarbonat Lösung und 20 ml Dichlormethan verteilt. Die wäßr. Phase wurde 2 mal mit je 10 ml Dichlormethan extrahiert und die vereinigten organischen Phasen wurden mit $MgSO_4$ getrocknet. Die Lösungsmittel wurden im Vakuum entfernt und der Rückstand an Kieselgel chromatografiert.

Ausbeute: 30 mg (3R,6S,7aS)-6-tert-Butoxycarbonylamino-5-oxo-6-piperidin-1-yl-methyl)-hexahydro-pyrrolo[2,1-b]thiazol-3-carbonsäure-methylester
MS: 414 (M+H)

9h) (3R,6S,7aS)-6-Amino-5-oxo-6-(piperidin-1-yl-methyl)-hexahydro-pyrrolo[2,1-b]thiazol-3-carbonitril-bistrifluoracetat

30 mg (3R,6S,7aS)-6-tert-Butoxycarbonylamino-5-oxo-6-(piperidin-1-yl-methyl)-hexahydro-pyrrolo[2,1-b]thiazol-3-carbonsäure-methylester wurden bei 0°C in 30 ml Ammoniak gesättigtem Methanol gelöst. Innerhalb von 4 h ließ man auf Raumtemperatur kommen und entfernte dann die Lösungsmittel im Vakuum. Der Rückstand wurde in 5 ml THF aufgenommen und nacheinander 40 µl Triethylamin sowie 20 µl Trifluoressigsäureanhydrid zugegeben. Man ließ 2 h bei Raumtemperatur rühren gab dann weitere 40 µl Triethylamin sowie 20 µl Trifluoressigsäureanhydrid zu. Nach Rühren für 2 h wurde zwischen 60 ml 0,5 M HCl und 60 ml Essigester verteilt. Die wäßr. Phase wurde 2 mal mit je 20 ml Essigester extrahiert und die vereinigten organischen Phasen 1 mal mit 30 ml gesättigter Natriumhydrogencarbonat Lösung gewaschen. Nach Trocknen mit $MgSO_4$ wurden die Lösungsmittel im Vakuum entfernt. Der Rückstand wurde in einer Mmischung aus 1 ml Trifluoressigsäure und 200 µl Thioanisol aufgenommen und für 20 Min. bei Raumtemperatur gerührt. Die Trifluoressigsäure wurde dann im Vakuum entfernt und der Rückstand an Kieselgel mit Dichlormethan/Methanol/Essigsäure/Wasser 100:10:1:1 chromatografiert.
Ausbeute: 31 mg (3R,6S,7aS)-6-Amino-5-oxo-6-piperidin-1-yl-methyl-hexahydropyrrolo[2,1-b]thiazol-3-carbonitril-bistrifluoracetat.
MS: 281 (M+H)

9i) (3R,6S,7aR)-6-Amino-5-oxo-6-(piperidin-1-yl-methyl)-hexahydro-pyrrolo[2,1-b]thiazol-3-carbonitril-bistrifluoracetat

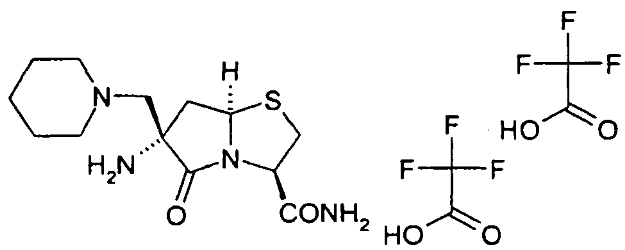

wurde analog zu den Beispielen 9f), 9g) und 9h) ausgehend vom Diastereomeren 9e) hergestellt.
MS: 281 (M+H)

**10) (3R,6S,7aS)-6-Amino-5-oxo-6-(piperidin-4-yl)-hexahydro-pyrrolo[2,1-b]thiazol-3-carbonitril-bistrifluoracetat**

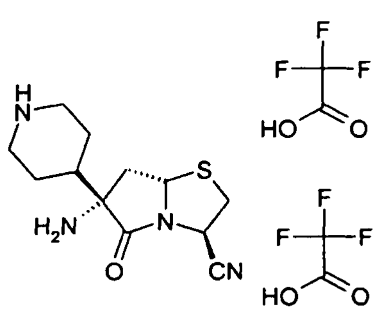

10a) 4-Hydroxi-piperidin-1-carbonsäure-2-trimethylsilanyl-ethylester

6,9 g 4-Hydroxi-piperidin wurden in 150 ml THF gelöst. Dazu wurden nacheinander 19,4 g 2-(Trimethylsilyl)-ethyl-4-nitrophenyl-carbonat und 11,9 ml N,N-Diisopropyl-ethyl-amin gegeben. Man ließ 1 h bei Raumtemperatur rühren und entfernte dann das Lösungsmittel im Vakuum. Der Rückstand wurde in 400 ml Essigester aufgenommen und 1 mal mit 200 ml 0.5 N HCl sowie 2 mal mit je 200 ml 1 N NaOH gewaschen. Die organische Phase wurde mit $MgSO_4$ getrocknet, das Lösungsmittel im Vakuum entfernt und der Rückstand chromatografisch an Kieselgel gereinigt.
Ausbeute: 15,4 g 4-Hydroxi-piperidin-1-carbonsäure-2-trimethylsilanyl-ethylester
MS: 218 (M+H-C2H4)

10b) 4-Iod-piperidin-1-carbonsäure-2-trimethylsilanyl-ethylester

12,5 g 4-Hydroxi-piperidin-1-carbonsäure-2-trimethylsilanyl-ethylester wurden in 136 ml Dichlormethan und 264 ml Tetrachlormethan gelöst. Man kühlte auf 0˚C ab und gab dann nacheinander 16 g Triphenylphosphin, 4,2 g Imidazol und 15,8 g Iod hinzu. Man ließ langsam auf Raumtemperatur kommen und 14 h nachrühren. Danach wurden zu der Lösung 300 ml einer gesättigten Natriumthiosulfat Lösung gegeben. Man ließ rühren bis die Mischung farblos war. Dann wurde die wäßr. Phase 2 mal mit je 200 ml Dichlormethan extrahiert. Die vereinigten organischen Phasen wurden 1 mal mit 200 ml einer gesättigten Natriumthiosulfat Lösung und 1 mal mit 200 ml einer gesättigten Natriumchlorid Lösung gewaschen. Nach Trocknen mit $MgSO_4$ wurden die Lösungsmittel im Vakuum entfernt und der Rückstand durch Chromatografie an Kieselgel gereinigt.
Ausbeute: 8 g 4-Iod-piperidin-1-carbonsäure-2-trimethylsilanyl-ethylester
MS: 328 (M+H-C2H4)

10c) (3R,SR,6S)-2-Oxo-5,6-diphenyl-3-[1-(2-trimethylsilanyl-ethoxycarbonyl)-piperidin-4-yl]-morpholin-4-carbonsäure-tert-butylester

4 g (5S,6R)-2-Oxo-5,6-diphenyl-morpholin-4-carbonsäure-tert-butylester wurden in 50 ml THF gelöst. Man gab 9 ml 15-Krone-5 hinzu und kühlte auf -78˚C ab. Danach tropfte man 6,8 ml einer 2 M Lösung von NaHMDS in THF zu. Man ließ 10 Min. rühren und tropfte dann 8 g 4-Iod-piperidin-1-carbonsäure-2-trimethylsilanyl-ethylester zu. Innerhalb von 6 h ließ man auf 0˚C erwärmen und beendete dann die Reaktion durch Zugabe von 200 ml gesättigter Ammoniumchlorid-Lösung. Die wäßr. Phase wurde 3 mal mit je 150 ml Essigester extrahiert und die vereinigten organischen Phasen wurden mit $MgSO_4$ getrocknet. Die Lösungsmittel wurden im Vakuum entfernt und der Rückstand an Kieselgel chromatografiert.

Ausbeute: 1,82 g (3R,5R,6S)-2-Oxo-5,6-diphenyl-3-[1-(2-trimethylsilanyl-ethoxycarbonyl)-piperidin-4-yl]-morpholin-4-carbonsäure-tert-butylester
MS: 497 (M+H-C2H4-tBu)

10d) (3S,5R,6S)-3-Allyl-2-oxo-5,6-diphenyl-3-[1-(2-trimethylsilanyl-ethoxycarbonyl)-piperidin-4-yl]-morpholin-4-carbonsäure tert-butylester

1,8 g (3R,5R,6S)-2-Oxo-5,6-diphenyl-3-[1-(2-trimethylsilanyl-ethoxycarbonyl)-piperidin-4-yl]-morpholin-4-carbonsäure-tert-butylester wurden in 20 ml THF gelöst. Man gab 2,5 ml 15-Krone-5 hinzu und kühlte auf-78˚C ab. Danach tropfte man 1,9 ml einer 2 M Lösung von NAHMDS in THF zu. Man ließ 10 Min. rühren und tropfte dann 805 μl Allylbromid zu. Innerhalb von 6 h ließ man auf 0˚C erwärmen und beendete dann die Reaktion durch Zugabe von 100 ml gesättigter Ammoniumchlorid-Lösung. Die wäßr. Phase wurde 3 mal mit je 100 ml Essigester extrahiert und die vereinigten organischen Phasen wurden mit $MgSO_4$ getrocknet. Die Lösungsmittel wurden im Vakuum entfernt und der Rückstand an Kieselgel chromatografiert.
Ausbeute: 916 mg (3S,5R,6S)-3-Allyl-2-oxo-5,6-diphenyl-3-[1-(2-trimethylsilanylethoxycarbonyl)-piperidin-4-yl]-morpholin-4-carbonsäure-tert-butylester
MS: 593 (M+H-C2H4)

10e) 4-((S)-1-tert-Butoxycarbonylamino-1-methoxycarbonyl-but-3-enyl)-piperidin-1-carbonsäure 2-trimethylsilanyl-ethylester

50 ml Ammoniak wurden bei -78˚C einkondensiert. Man gab 340 mg Natrium in 2 Portionen im Abstand von je 10 Min. zu und ließ 20 Min nachrühren. Dazu tropfte man bei - 60˚C eine Lösung von 916 mg (3S,5R,6S)-3-Allyl-2-oxo-5,6-diphenyl-3-[1-(2-trimethylsilanyl-ethoxycarbonyl)-piperidin-4-yl]-morpholine-4-carbonsäure tert-butylester und 866 μl Ethanol in 50 ml THF. Man ließ 1,5 h bei -45˚C rühren und beendete die Reaktion durch Zugabe von soviel festem Ammoniumchlorid, bis die blaue Farbe verschwand. Danach ließ man das Ammoniak abdampfen und gab zum Rückstand 150 ml Wasser. Das THF wurde im Vakuum entfernt. Die wässrige Lösung wurde mit 1 N HCl auf pH 2

eingestellt und 3 mal mit je 100 ml Essigester extrahiert. Die vereinigten organischen Phasen wurden mit $MgSO_4$ getrocknet. Die Lösungsmittel wurden im Vakuum entfernt und der Rückstand mit 4 % Methanol in Dichlormethan über Kieselgel filtriert. Das So erhaltene Produkt wurde in 5 ml Methanol aufgenommen und auf 0˚C gekühlt. Dazu wurden 5 mal im Abstand von je 1,5 Stunden 0,5 ml einer 2 N Lösung von Trimethylsilyldiazomethan in Hexan gegeben. Danach tropfte man 2 ml Essigsäure hinzu und ließ 1 h bei Raumtemperatur nachrühren. Die Lösungsmittel wurden im Vakuum entfernt und der Rückstand an Kieselgel chromatografiert.
Ausbeute: 233 mg 4-((S)-1-tert-Butoxycarbonylamino-1-methoxycarbonyl-but-3-enyl)-piperidin-1-carbonsäure 2-trimethylsilanyl-ethylester
MS: 373 (M+H-C2H4-tBu)

10f) (3R,6S,7aS)-6-tert-Butoxycarbonylamino-5-oxo-6-[1-(2-trimethylsilanylethoxycarbonyl)-piperidin-4-yl]-hexahydro-pyrrolo[2,1-b]thiazol-3-carbonsäure

230 mg 4-((S)-1-tert-Butoxycarbonylamino-1-methoxycarbonyl-but-3-enyl)-piperidine-1-carbonsäure 2-trimethylsilanyl-ethylester wurden in 25 ml THF und 5 ml Wasser gelöst. Unter Argon gab man dazu 157 µl einer 4%-igen Lösung von Osmiumtetroxid in Wasser und 269 mg Natriumperiodat. Man ließ 2 h bei Raumtemperatur rühren und filtrierte dann über Kieselgur. Der Filterkuchen wurde 2 mal mit je 10 ml THF gewaschen. Das THF wurde im Vakuum entfernt und der Rückstand zwischen 20 ml Essigester und 10 ml einer gesättigten Natriumchlorid Lösung verteilt. Die wäßr. Phase wurde 2 mal mit je 10 ml Essigester extrahiert und die vereinigten organischen Phasen wurden mit $MgSO_4$ getrocknet. Die Lösungsmittel wurden im Vakuum entfernt und der Rückstand in 15 ml Pyridin aufgenommen. Man gab 61 mg L-Cystein zu und erhitzte 24 h auf 100˚C. Danach wurde das Pyridin im Vakuum entfernt und der Rückstand an Kieselgel chromatografiert. Ausbeute: 230 mg (3R,6S,7aS)-6-tert-Butoxycarbonylamino-5-oxo-6-[1-(2-trimethylsilanylethoxycarbonyl)-piperidin-4-yl]-hexahydro-pyrrolo[2,1-b]thiazol-3-carbonsäure
MS: 446 (M+H-C2H4-tBu)

10g) (3R,6S,7aS)-6-tert-Butoxycarbonylamino-5-oxo-6-[1-(2-trimethylsilanylethoxycarbonyl)-piperidin-4-yl]-hexahydro-pyrrolo[2,1-b]thiazol-3-carbonsäuremethylester

223 mg (3R,6S,7aS)-6-tert-Butoxycarbonylamino-5-oxo-6-[1-(2-trimethylsilanylethoxycarbonyl)-piperidin-4-yl]-hexahydro-pyrrolo[2,1-b] thiazol-3-carbonsäure wurden in 10 ml Methanol und 10 ml Diethylether gelöst und auf 0˚C gekühlt. Man tropfte 2 mal im Abstand von 1,5 h je 400 µl einer 1 N Lösung von Trimethylsilyldiazomethan in Hexan zu. Danach ließ man 2 h nachrühren und tropfte dann 1 ml Essigsäure zu. Nach 1 h wurden die Lösungsmittel im Vakuum entfernt und der Rückstand durch Chromatografie an Kieselgel gereinigt.
Ausbeute: 153 mg (3R,6S,7aS)-6-tert-Butoxycarbonylamino-5-oxo-6-[1-(2-trimethylsilanyl-ethoxycarbonyl)-piperidin-4-yl]-hexahydro-pyrrolo[2,1-b]thiazol-3-carbonsäure-methylester
MS: 460 (M+H-C2H4-tBu)

10h) 4-((3R,65,7aS)-6-tert-Butoxycarbonylamino-3-cyano-5-oxo-hexahydro-pyrrolo[2,1-b]thiazol-6-yl)-piperidin-1-carbonsäure-2-trimethylsilanyl-ethylester

153 mg (3R,6S,7aS)-6-tert-Butoxycarbonylamino-5-oxo-6-[1-(2-trimethylsilanylethoxycarbonyl)-piperidin-4-yl]-hexahydro-pyrrolo[2,1-b]thiazol-3-carbonsäuremethylester wurden bei 0˚C in 30 ml Ammoniak gesättigtem Methanol gelöst. Innerhalb von 4 h ließ man auf Raumtemperatur kommen und entfernte dann die Lösungsmittel im Vakuum. Der Rückstand wurde in 5 ml THF aufgenommen und nacheinander 100 µl Triethylamin sowie 60 µl Trifluoressigsäureanhydrid zugegeben. Man ließ 2 h bei Raumtemperatur rühren. Danach wurde zwischen 60 ml 0,5 M HCl und 60 ml Essigester verteilt. Die wäßr. Phase wurde 2 mal mit je 20 ml Essigester extrahiert und die vereinigten organischen Phasen 1 mal mit 30 ml gesättigter Natriumhydrogencarbonat Lösung
gewaschen. Nach Trocknen mit $MgSO_4$ wurden die Lösungsmittel im Vakuum entfernt. Der Rückstand wurde an Kieselgel chromatografiert.
Ausbeute: 108 mg 4-((3R,6S,7aS)-6-tert-Butoxycarbonylamino-3-cyano-5-oxo-hexahydropyrrolo[2,1-b]thiazol-6-yl)-piperidin-1-carbonsäure 2-trimethylsilanyl-ethylester MS: 427 (M+H-C2H4-tBu)

10i) (3R,6S,7aS)-6-Amino-5-oxo-6-piperidin-4-yl-hexahydro-pyrrolo[2,1-b]thiazol-3-carbonitril-bistrifluoracetat

12 mg 4-((3R,6S,7aS)-6-tert-Butoxycarbonylamino-3-cyano-5-oxo-hexahydro-pyrrolo[2,1-b]thiazol-6-yl)-piperidin-1-carbonsäure 2-trimethylsilanyl-ethylester wurden bei 0˚C für 40 Min. in einer Mischung aus 1 ml Trifluoressigsäure und 200 µl Thioanisol gerührt. Die Trifluoressigsäure wurde im Vakuum entfernt und der Rückstand zwischen 6 ml Wasser und 10 ml Essigester verteilt. Die wäßr. Phase wurde 3 mal mit je 5 ml Essigester gewaschen. Danach wurde

das Wasser im Vakuum entfernt und der Rückstand mit Diethylether verrührt.
Ausbeute: 7 mg (3R,6S,7aS)-6-Amino-5-oxo-6-piperidin-4-yl-hexahydro-pyrrolo[2,1-b]thiazol-3-carbonitril-bistrifluoracetat
MS: 267 (M+H)

**11) (3R,6R,7aS)-6-Amino-5-oxo-6-(piperidin-4-yl-methyl)-hexahydropyrrolo[2,1-b]thiazol-3-carbonitril-bistrifluoracetat**

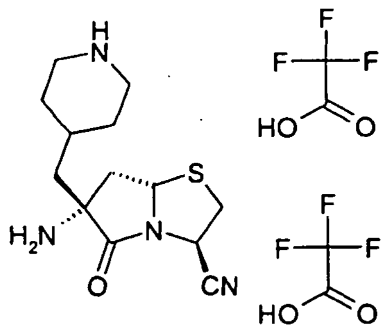

11a) 4-Hydroximethyl-piperidin-1-carbonsäure-2-trimethylsilanyl-ethylester

10,5 g 4-Piperidinyl-methanol wurden in 170 ml THF gelöst. Dazu wurden nacheinander 25,8 g 2-(Trimethylsilyl)-ethyl-4-nitrophenyl-carbonat und 15,9 ml N,N-Diisopropyl-ethylamin gegeben. Man ließ 1 h bei Raumtemperatur rühren und entfernte dann das Lösungsmittel im Vakuum. Der Rückstand wurde in 400 ml Essigester aufgenommen und 1 mal mit 300 ml 0.5 N HCl sowie 2 mal mit je 250 ml 1 N NaOH gewaschen. Die organische Phase wurde mit $MgSO_4$ getrocknet, das Lösungsmittel im Vakuum entfernt und der Rückstand chromatografisch an Kieselgel gereinigt.
Ausbeute: 19,9 g 4-Hydroximethyl-piperidin-1-carbonsäure-2-trimethylsilanyl-ethylester MS: 232 (M+H-C2H4)

11b) 4-Brommethyl-piperidin-1-carbonsäure-2-trimethylsilanyl-ethylester

19,9 g 4-Hydroximethyl-piperidin-1-carbonsäure-2-trimethylsilanyl-ethylester wurden in 600 ml THF gelöst. Man kühlte auf 0°C ab und gab dann 50,7 g Tetrabrommethan. Danach wurden 42.1 g Triphenylphosphin in 4 Portionen im Abstand von je 20 Min. zugegeben. Man ließ auf Raumtemperatur kommen und 14 h nachrühren. Anschließend wurde das Lösungsmittel im Vakuum entfernt und der Rückstand an Kieselgel chromatografiert.
Ausbeute: 22,2 g 4-Bromomethyl-piperidin-1-carbonsäure-2-trimethylsilanyl-ethylester MS: 294 (M+H-C2H4)

11c) (3S,5S,6R)-3-Allyl-2-oxo-5,6-diphenyl-3-[1-(2-trimethylsilanyl-ethoxycarbonyl)-piperidin-4-ylmethyl]-morpholin-4-carbonsäure-tert-butylester

4,9 g (3S,5S,6R)-3-Allyl-2-oxo-5,6-diphenyl-morpholin-4-carbonsäure-tert-butylester wurden in 100 ml THF gelöst. Man gab 14,9 ml 15-Krone-5 hinzu und kühlte auf-78°C ab. Danach tropfte man 6,8 ml einer 2 M Lösung von NaHMDS in THF zu. Man ließ 10 Min. rühren und tropfte dann 8 g 4-Bromomethyl-piperidine-1-carbonsäure-2-trimethylsilanyl-ethylester zu. Innerhalb von 6 h ließ man auf 0°C erwärmen und beendete dann die Reaktion durch Zugabe von 500 ml gesättigter Ammoniumchlorid-Lösung. Die wäßr. Phase wurde 3 mal mit je 400 ml Essigester extrahiert und die vereinigten organischen Phasen wurden mit $MgSO_4$ getrocknet. Die Lösungsmittel wurden im Vakuum entfernt und der Rückstand an Kieselgel chromatografiert.
Ausbeute: 2,76 g (3S,5S,6R)-3-Allyl-2-oxo-5,6-diphenyl-3-[1-(2-trimethylsilanylethoxycarbonyl)-(piperidin-4-ylmethyl)]-morpholin-4-carbonsäure-tert-butylester
MS: 551 (M+H-C2H4)

11d) (S)-4-(2-tert-Butoxycarbonylamino-2-carboxy-pent-4-en-yl)-piperidin-1-carbonsäure-2-trimethylsilanyl-ethylester

110 ml Ammoniak wurden bei -78°C einkondensiert. Man gab 1 g Natrium in 3 Portionen im Abstand von je 10 Min. zu und ließ 20 Min nachrühren. Dazu tropfte man bei - 60°C eine Lösung von 2,76 g (3S,5S,6R)-3-Allyl-2-oxo-5,6-diphenyl-3-[1-(2-trimethylsilanylethoxycarbonyl)-piperidin-4-ylmethyl]-morpholin-4-carbonsäure-tert-butylester und

2,6 ml Ethanol in 50 ml THF. Man ließ 1,5 h bei -45˚C rühren und beendete die Reaktion durch Zugabe von soviel festem Ammoniumchlorid, bis die blaue Farbe verschwand. Danach ließ man das Ammoniak abdampfen und gab zum Rückstand 300 ml Wasser. Das THF wurde im Vakuum entfernt. Die wässrige Lösung wurde mit 1 N HCl auf pH 2 eingestellt und 3 mal mit je 200 ml Essigester extrahiert. Die vereinigten organischen Phasen wurden mit $MgSO_4$ getrocknet. Die Lösungsmittel wurden im Vakuum entfernt und der Rückstand an Kieselgel chromatografiert.
Ausbeute: 1,3 g (S)-4-(-2-tert-Butoxycarbonylamino-2-carboxy-pent-4-enyl)-piperidin-1-carbonsäure-2-trimethylsilanyl-ethylester
MS: 373 (M+H-C2H4-tBu)

11e) (S)-4-(2-tert-Butoxycarbonylamino-2-methoxycarbonyl-pent-4-enyl)-piperidin-1-carbonsäure-2-trimethylsilanyl-ethylester

1,1 g (S)-(2-tert-Butoxycarbonylamino-2-methoxycarbonyl-pent-4-enyl)-piperidin-1-carbonsäure-2-trimethylsilanyl-ethylester wurden in 33 ml Methanol gelöst und auf 0˚C gekühlt. Man tropfte 2,3 ml einer 2 N Lösung von Trimethylsilyldiazomethan in Hexan zu. Innerhalb von 3 h ließ ma auf Raumtemperatur kommen und tropfte dann weitere 2,3 ml der Trimethylsilyldiazomethan Lösung zu. Man ließ 1 h nachrühren und beendete dann die Reaktion durch Zugabe von 1 ml Essigsäure. Anschließen ließ man 30 Min. nachrühren, entfernt die Lösungsmittel im Vakuum und-chromatografierte den Rückstand an Kieselgel.
Ausbeute: 755 mg (S)-(-2-tert-Butoxycarbonylamino-2-methoxycarbonyl-pent-4-enyl)-piperidin-1-carbonsäure-2-trimethylsilanyl-ethylester
MS: 387 (M+H-C2H4-tBu)

11f) (R)-4-(2-tert-Butoxycarbonylamino-2-methoxycarbonyl-4-oxo-butyl)-piperidin-1-carbonsäure-2-trimethylsilanyl-ethylester

750 mg (S)-4-(-2-tert-Butoxycarbonylamino-2-methoxycarbonyl-pent-4-enyl)-piperidin-1-carbonsäure-2-trimethylsilanyl-ethylester wurden in 30 ml THF gelöst. Man gab 852 mg Natriumperiodat und 10 ml Wasser hinzu. Danach ließ ma 3 h bei Raumtemperatur rühren und filtrierte über Kieselgur. Der Filterkuchen wurde 2 mal mit je 10 ml THF gewaschen. Das THF wurde im Vakuum entfernt und der Rückstand zwischen 30 ml Wasser und 30 ml Essigester verteilt. Der pH-Wert der wäßr. Phase wurde mit 1 N HCl Lösung auf 2 eingestellt. Die Phasen wurden getrennt und die wäßr. Phase 2 mal mit je 20 ml Essigester extrahiert.. Die vereinigten organischen Phasen wurden mit $MgSO_4$ getrocknet. Die Lösungsmittel wurden im Vakuum entfernt und der Rückstand an Kieselgel chromatografiert.
Ausbeute: 530 mg (R)-4-(2-tert-Butoxycarbonylamino-2-methoxycarbonyl-4-oxo-butyl)-piperidin-1-carbonsäure-2-trimethylsilanyl-ethylester
MS: 389 (M+H-C2H4-tBu)

11g) (3R,6R,7aS)-6-tert-Butoxycarbonylamino-5-oxo-6-[1-(2-trimethylsilanylethoxycarbonyl)-piperidin-4-ylmethyl]-hexahydro-pyrrolo[2,1-b]thiazol-3-carbonsäure

520 mg (R)-4-(2-tert-Butoxycarbonylamino-2-methoxycarbonyl-4-oxo-butyl)-piperidin-1-carbonsäure-2-trimethylsilanyl-ethylester wurden in 25 ml Pyridin gelöst. Man gab 134 mg L-Cystein hinzu und erhitzte 36 h auf 100˚C. Danach wurde das Pyridin im Vakuum entfernt und der Rückstand zwischen 50 ml Essigester und 50 ml 0,5 N HCl verteilt. Die wäßr. Phase wurde 2 mal mit je 20 ml Essigester extrahiert und die vereinigten organischen Phasen wurden mit $MgSO_4$ getrocknet. Die Lösungsmittel wurden im Vakuum entfernt und der Rückstand wurde an Kieselgel chromatografiert.
Ausbeute: 500 mg (3R,6R,7aS)-6-tert-Butoxycarbonylamino-5-oxo-6-[1-(2-trimethylsilanyl-ethoxycarbonyl)-piperidin-4-ylmethyl]-hexahydro-pyrrolo[2,1-b]thiazol-3-carbonsäure
MS. 460 (M+H-C2H4-tBu)

11h) (3R,6R,7aS)-6-tert-Butoxycarbonylamino-5-oxo-6-[1-(2-trimethylsilanylethoxycarbonyl)-piperidin-4-ylmethyl]-hexahydro-pyrrolo[2,1-b]thiazol-3-carbonsäure methylester

497 mg (3R,6R,7aS)-6-tert-Butoxycarbonylamino-5-oxo-6-[1-(2-trimethylsilanylethoxycarbonyl)-piperidin-4-ylmethyl]-hexahydro-pyrrolo[2,1-b]thiazol-3-carbonsäure wurden in 8 ml Methanol und 8 ml Diethylether gelöst und auf 0˚C gekühlt. Man tropfte 914 $\mu$l einer 1 N Lösung von Trimethylsilyldiazomethan in Hexan zu. Danach ließ man 1 h nachrühren und tropfte dann 1 ml Essigsäure zu. Nach 1 h wurden die Lösungsmittel im Vakuum entfernt und der Rückstand durch Chromatografie an Kieselgel gereinigt. Ausbeute: 345 mg (3R,6R,7aS)-6-tert-Butoxycarbonylamino-5-oxo-6-[1-(2-trimethylsilanyl-ethoxycarbonyl)-piperidin-4-ylmethyl]-hexahydro-pyrrolo[2,1-b]thiazol-3-carbonsäure-methylester
MS: 474 (M+H-C2H4-tBu)

11i) 4-((3R,6R,7aS)-6-tert-Butoxycarbonylamino-3-carbamoyl-5-oxo-hexahydropyrrolo[2,1-b]thiazol-6-ylmethyl)-piperidin-1-carbonsäure-2-trimethylsilanyl-ethylester

340 mg (3R,6R,7aS)-6-tert-Butoxycarbonylamino-5-oxo-6-[1-(2-trimethylsilanylethoxycarbonyl)-piperidin-4-ylmethyl]-hexahydro-pyrrolo[2,1-b]thiazol-3-carbonsäuremethylester wurden bei 0˚C in 35 ml einer mit Ammoniak gesättigtem Methanollösung gelöst. Innerhalb von 4 h ließ man auf Raumtemperatur kommen und entfernte dann die Lösungsmittel im Vakuum. Der Rückstand wurde ohne weitere Reinigung in der nächsten Umsetzung eingesetzt.
Ausbeute: 300 mg 4-((3R,6R,7aS)-6-tert-Butoxycarbonylamino-3-carbamoyl-5-oxohexahydro-pyrrolo[2,1-b]thiazol-6-ylmethyl)-piperidin-1-carbonsäure-2-trimethylsilanyl-ethylester
MS: 300 (M+H-C2H4-tBu)

11j) 4-((3R,6R,7aS)-6-tert-Butoxycarbonylamino-3-cyano-5-oxo-hexahydro-pyrrolo[2,1-b]thiazol-6-ylmethyl)-piperidin-1-carbonsäure-2-trimethylsilanyl-ethylester

295 mg 4-((3R,6R,7aS)-6-tert-Butoxycarbonylamino-3-carbamoyl-5-oxo-hexahydropyrrolo[2,1-b]thiazol-6-ylmethyl)-piperidin-1-carbonsäure-2-trimethylsilanyl-ethylester wurden in 20 ml THF gelöst. Dazu tropfte man nacheinander 166 μl Triethylamin und 91 μl Trifluoressigsäureanhydrid. Man ließ 1 h rühren und gab dann noch einmal die gleichen Mengen Triethylamin und Trifluoressigsäureanhydrid hinzu. Nach einer weiteren Stunde Rühren wurde zwischen 150 ml Essigester und 150 ml Wasser verteilt. Die organische Phase wurde mit $MgSO_4$ getrocknet. Das Lösungsmittel wurde im Vakuum entfernt und der Rückstand an Kieselgel chromatografiert.
Ausbeute: 254 mg 4-((3R,6R,7aS)-6-tert-Butoxycarbonylamino-3-cyano-5-oxo-hexahydropyrrolo[2,1-b]thiazol-6-ylmethyl)-piperidin-1-carbonsäure-2-trimethylsilanyl-ethylester
MS: 441 (M+H-C2H4-tBu)

11h) (3R,6R,7aS)-6-Amino-5-oxo-6-piperidin-4-yl-methyl-hexahydro-pyrrolo[2,1-b]thiazol-3-carbonitril-bistrifluoracetat

14 mg 4-((3R,6R,7aS)-6-tert-Butoxycarbonylamino-3-cyano-5-oxo-hexahydro-pyrrolo[2,1-b]thiazol-6-yl-methyl)-piperidin-1-carbonsäure-2-trimethylsilanyl-ethylester wurden bei 0˚C für 40 Min. in einer Mischung aus 1 ml Trifluoressigsäure und 200 μl Thioanisol gerührt. Die Trifluoressigsäure wurde im Vakuum entfernt und der Rückstand mit Diethylether verrührt.
Ausbeute: 10 mg (3R,6R,7aS)-6-Amino-5-oxo-6-piperidin-4-yl-methyl-hexahydropyrrolo[2,1-b]thiazol-3-carbonitril-bistrifluoracetat
MS: 281 (M+H)

## Patentansprüche

1. Verbindungen der Formel I,

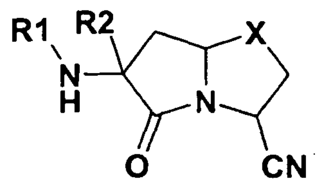

Formel I

worin bedeuten

R1 H, $(C_1-C_{10})$-Alkyl, $(C_3-C_{10})$-Cycloalkyl, $(C_2-C_6)$-Alkenyl, $(C_2-C_6)$-Alkinyl, $(C_6-C_{10})$-Aryl, Heterozyklyl, wobei die Alkyl-, Cycloalkyl-, Alkenyl-, Alkinyl-, Aryl- und Heterozyklyl Reste ein- oder mehrfach substituiert sein können mit F, Cl, Br, I, CN, $NO_2$, OH, $-CF_3$, $(C_1-C_6)$-Alkyl, $(C_2-C_6)$-Alkenyl, $(C_2-C_6)$-Alkinyl, OR3, NR3R4, COR3,

CO2R3, CONR3R4, ($C_1$-$C_6$)-Alkylen-OR3, ($C_1$-$C_6$)-Alkylen-NR3R4, ($C_1$-$C_6$)-Alkylen-SR3, Alkylen-SOR3, Alkylen-$SO_2$R3, ($C_1$-$C_6$)-Alkylen-COR3, ($C_1$-$C_6$)-Alkylen-COOR3, ($C_1$-$C_6$)-Alkylen-CONR3R4, ($C_1$-$C_6$)-Alkylen-O-P(O)$(OR3)_2$, SR3, SOR3, $SO_2$NR3R4, $SO_2$R3, ($C_1$-$C_6$)-Alkylen-($C_3$-$C_{10}$)-Cycloalkyl, ($C_1$-$C_6$)-Alkylen-($C_6$-$C_{10}$)-Aryl, ($C_1$-$C_6$)-Alkylen-Heterozyklyl, ($C_3$-$C_{10}$)-Cycloalkyl, ($C_6$-$C_{10}$)-Aryl, das mit F, Cl, Br, I, CN, $NO_2$, OH, -$CF_3$, ($C_1$-$C_6$)-Alkyl, ($C_2$-$C_6$)-Alkenyl, ($C_2$-$C_6$)-Alkinyl, OR3, NR3R4, COR3, $CO_2$R3, CONR3R4 ein oder mehrfach substituiert sein kann, oder Heterozyklyl, das mit F, Cl, Br, I, CN, $NO_2$, OH, -$CF_3$, ($C_1$-$C_6$)-Alkyl, OR3, NR3R4, COR3, $CO_2$R3, CONR3R4 ein oder mehrfach substituiert sein kann;

R2 H, ($C_1$-$C_{10}$)-Alkyl, ($C_3$-$C_{10}$)-Cycloalkyl, ($C_2$-$C_6$)-Alkenyl, ($C_2$-$C_6$)-Alkinyl, ($C_6$-$C_{10}$)-Aryl, Heterozyklyl, COR3, COOR3, CONR3R4, CN, wobei die Alkyl-, Cycloalkyl-, Alkenyl-, Alkinyl-, Aryl- und Heterozyklyl Reste ein-oder mehrfach substituiert sein können mit F, Cl, Br, I, CN, $NO_2$, SH, $SF_5$, OH, ($C_1$-$C_6$)-Alkyl, -$CF_3$, ($C_2$-$C_6$)-Alkenyl, ($C_2$-$C_6$)-Alkinyl, OR3, OP(O)$(OR3)_2$, NR3R4, NR3CONR3R4, COR3, OCOR3, OCOOR3, COOR3, CONR3R4, OCONR3R4, ($C_1$-$C_6$)-Alkylen-OR3, ($C_1$-$C_6$)-Alkylen-NR3R4, ($C_1$-$C_6$)-Alkylen-NR3$SO_2$R4, ($C_1$-$C_6$)-Alkylen-SR3, Alkylen-S(O)R3, Alkylen-$S(O)_2$R3, Alkylen-$S(O)_2$NR3R4, ($C_1$-$C_6$)-Alkylen-COR3, ($C_1$-$C_6$)-Alkylen-COOR3,($C_1$-$C_6$)-Alkylen-CONR3R4,SR3,SOR3,$SO_2$R3,$SO_2$NR3R4,NR3$SO_2$R4,($C_1$-$C_6$)-Alkylen-($C_3$-$C_{10}$)-Cycloalkyl, ($C_1$-$C_6$)-Alkylen-($C_6$-$C_{10}$)-Aryl, ($C_1$-$C_6$)-Alkylen-Heterozyklyl, ($C_3$-$C_{10}$)-Cycloalkyl, ($C_6$-$C_{10}$)-Aryl oder Heterozyklyl;

R3, R4 unabhängig voneinander H, ($C_1$-$C_6$)-Alkyl, -$CF_3$, ($C_3$-$C_{10}$)-Cycloalkyl, ($C_6$-$C_{10}$)-Aryl, Heterozyklyl, ($C_1$-$C_6$)-Alkylen-CONR5R6, CONR5R6, ($C_1$-$C_6$)-Alkylen-COOR5, COOR5, COR5, ($C_1$-$C_6$)-Alkylen-COR5, ($C_1$-$C_6$)-Alkylen-OR5, ($C_1$-$C_6$)-Alkylen-NRSR6, ($C_1$-$C_6$)-Alkylen-SR5, ($C_1$-$C_6$)-Alkylen-S(O)R5, ($C_1$-$C_6$)-Alkylen-$S(O)_2$R5, S(O)R5, $S(O)_2$R5, ($C_1$-$C_4$)-Alkylen-($C_6$-$C_{10}$)-Aryl oder ($C_1$-$C_4$)-Alkylen-Heterozyklyl;

R5, R6 unabhängig voneinander H, ($C_1$-$C_6$)-Alkyl, ($C_1$-$C_6$)-Alkylen-($C_6$-$C_{10}$)-Aryl, -($C_6$-$C_{10}$)-Aryl, Heterozyklyl, ($C_1$-$C_6$)-Alkylen-Heterozyklyl;

X S, SO, $SO_2$;

sowie deren physiologisch verträglichen Salze.

**2.** Verbindungen der Formel I, gemäß Anspruch 1, **dadurch gekennzeichnet, dass** darin bedeuten

R1 H, ($C_1$-$C_{10}$)-Alkyl, ($C_3$-$C_{10}$)-Cycloalkyl, ($C_2$-$C_6$)-Alkenyl, ($C_2$-$C_6$)-Alkinyl, ($C_6$-$C_{10}$)-Aryl, wobei die Alkyl-, Cycloalkyl-, Alkenyl-, Alkinyl- und Aryl- Reste ein- oder mehrfach substituiert sein können mit F, Cl, Br, I, CN, $NO_2$, OH, -$CF_3$, ($C_1$-$C_6$)-Alkyl, ($C_2$-$C_6$)-Alkenyl, ($C_2$-$C_6$)-Alkinyl, OR3, NR3R4, COR3, $CO_2$R3, CONR3R4, ($C_1$-$C_6$)-Alkylen-OR3, ($C_1$-$C_6$)-Alkylen-NR3R4, ($C_1$-$C_6$)-Alkylen-SR3, Alkylen-SOR3, Alkylen-$SO_2$R3, ($C_1$-$C_6$)-Alkylen-COR3, ($C_1$-$C_6$)-Alkylen- $CO_2$R3, ($C_1$-$C_6$)-Alkylen-CONR3R4, (C1-C6)-Alkylen-O-P(O)$(OR3)_2$, SR3, SOR3, $SO_2$NR3R4, $SO_2$R3, ($C_1$-$C_6$)-Alkylen-($C_3$-$C_{10}$)-Cycloalkyl, ($C_1$-$C_6$)-Alkylen-($C_6$-$C_{10}$)-Aryl, ($C_1$-$C_6$)-Alkylen-Heterozyklyl, ($C_3$-$C_{10}$)-Cycloalkyl oder ($C_6$-$C_{10}$)-Aryl, das mit F, Cl, Br, I, CN, $NO_2$, OH, -$CF_3$, ($C_1$-$C_6$)-Alkyl, ($C_2$-$C_6$)-Alkenyl, ($C_2$-$C_6$)-Alkinyl, OR3, NR3R4, COR3, $CO_2$R3, CONR3R4 ein oder mehrfach substituiert sein kann;

R2 H, ($C_1$-$C_{10}$)-Alkyl, ($C_3$-$C_{10}$)-Cycloalkyl, ($C_2$-$C_6$)-Alkenyl, ($C_2$-$C_6$)-Alkinyl, ($C_6$-$C_{10}$)-Aryl, Heterozyklyl, COR3, CO2R3, CONR3R4, CN, wobei die Alkyl-, Cycloalkyl-, Alkenyl-, Alkinyl-, Aryl- und Heterozyklyl Reste ein-oder mehrfach substituiert sein können mit F, Cl, Br, I, CN, $NO_2$, SH, $SF_5$, OH, ($C_1$-$C_6$)-Alkyl, -$CF_3$, ($C_2$-$C_6$)-Alkenyl, ($C_2$-$C_6$)-Alkinyl, OR3, OP(O)$(OR3)_2$, NR3R4, NR3CONR3R4, COR3, OCOR3, $CO_2$R3, CONR3R4, OCONR3R4, ($C_1$-$C_6$)-Alkylen-OR3, ($C_1$-$C_6$)-Alkylen-NR3R4, ($C_1$-$C_6$)-Alkylen-NR3$SO_2$R4, ($C_1$-$C_6$)-Alkylen-SR3, Alkylen-SOR3, Alkylen-$SO_2$R3, Alkylen-$SO_2$NR3R4, ($C_1$-$C_6$)-Alkylen-COR3, ($C_1$-$C_6$)-Alkylen-$CO_2$R3, ($C_1$-$C_6$)-Alkylen-CONR3R4, SR3, SOR3, $SO_2$R3, $SO_2$NR3R4, NR3$SO_2$R4, ($C_1$-$C_6$)-Alkylen-($C_3$-$C_{10}$)-Cycloalkyl, ($C_1$-$C_6$)-Alkylen-($C_6$-$C_{10}$)-Aryl, ($C_1$-$C_6$)-Alkylen-Heterozyklyl, ($C_3$-$C_{10}$)-Cycloalkyl, ($C_6$-$C_{10}$)-Aryl, das mit F, Cl, Br, I, CN, $NO_2$, OH, -$CF_3$, ($C_1$-$C_6$)-Alkyl, ($C_2$-$C_6$)-Alkenyl, ($C_2$-$C_6$)-Alkinyl, OR3, NR3R4, COR3, COzR3, CONR3R4 ein oder mehrfach substituiert sein kann, oder Heterozyklyl, das mit F, Cl, Br, I, CN, $NO_2$, OH, -$CF_3$, ($C_1$-$C_6$)-Alkyl, OR3, NR3R4, COR3, $CO_2$R3, CONR3R4 ein oder mehrfach substituiert sein kann;

R3, R4 unabhängig voneinander H, ($C_1$-$C_6$)-Alkyl, -$CF_3$, ($C_3$-$C_{10}$)-Cycloalkyl, ($C_6$-$C_{10}$)-Aryl, Heterozyklyl, ($C_1$-$C_6$)-Alkylen-CONR5R6, ($C_1$-$C_6$)-Alkylen-$CO_2$R5, ($C_1$-$C_6$)-Alkylen-COR5, ($C_1$-$C_6$)-Alkylen-OR5, ($C_1$-$C_6$)-Alkylen-NR5R6, ($C_1$-$C_6$)-Alkylen-SR5, ($C_1$-$C_6$)-Alkylen-SOR5, ($C_1$-$C_6$)-Alkylen-$SO_2$R5, ($C_1$-$C_4$)-Alkylen-($C_6$-$C_{10}$)-Aryl oder ($C_1$-$C_4$)-Alkylen-Heterozyklyl;

R5, R6 unabhängig voneinander H, ($C_1$-$C_6$)-Alkyl, ($C_3$-$C_{10}$)-Cycloalkyl, ($C_1$-$C_6$)-Alkylen-($C_6$-$C_{10}$)-Aryl, -($C_6$-$C_{10}$)-Aryl, Heterozyklyl, ($C_1$-$C_6$)-Alkylen-($C_3$-$C_{10}$)-Heterozyklyl;

X S;

sowie deren physiologisch verträglichen Salze.

**3.** Verbindungen der Formel I, gemäß Anspruch 1 oder 2, **dadurch gekennzeichnet, dass** darin bedeuten

R1 H;
R2 H, ($C_1$-$C_{10}$)-Alkyl, ($C_3$-$C_{10}$)-Cycloalkyl, ($C_2$-$C_6$)-Alkenyl, ($C_2$-$C_6$)-Alkinyl, ($C_6$-$C_{10}$)-Axyl, Heterozyklyl, COR3, $CO_2R3$, CONR3R4, CN, wobei die Alkyl-, Cycloalkyl-, Alkenyl-, Alkinyl-, Aryl- und Heterozyklyl Reste ein-oder mehrfach substituiert sein können mit F, Cl, Br, I, CN, $NO_2$, SH, $SF_5$, OH, ($C_1$-$C_6$)-Alkyl, -$CF_3$, ($C_2$-$C_6$)-Alkenyl, ($C_2$-$C_6$)-Alkinyl, OR3, OP(O)(OR3)2, NR3R4, NR3CONR3R4, COR3, OCOR3, $CO_2R3$, CONR3R4, OCONR3R4, ($C_1$-$C_6$)-Alkylen-OR3, ($C_1$-$C_6$)-Alkylen-NR3R4, ($C_1$-$C_6$)-Alkylen-NR3$SO_2$R4, ($C_1$-$C_6$)-Alkylen-SR3, Alkylen-SOR3, Alkylen-$SO_2$R3, Alkylen-$S(O)_2$NR3R4, ($C_1$-$C_6$)-Alkylen-COR3, ($C_1$-$C_6$)-Alkylen-$CO_2$R3, ($C_1$-$C_6$)-Alkylen-CONR3R4, SR3, SOR3, $SO_2$R3, $SO_2$NR3R4, NR3$SO_2$R4, ($C_1$-$C_6$)-Alkylen-($C_3$-$C_{10}$)-Cycloalkyl, ($C_1$-$C_6$)-Alkylen-($C_6$-$C_{10}$)-Aryl, ($C_1$-$C_6$)-Alkylen-Heterozyklyl, ($C_3$-$C_{10}$)-Cycloalkyl, ($C_6$-$C_{10}$)-Aryl, das mit F, Cl, Br, I, CN, $NO_2$, OH, -$CF_3$, ($C_1$-$C_6$)-Alkyl, ($C_2$-$C_6$)-Alkenyl, ($C_2$-$C_6$)-Alkinyl, OR3, NR3R4, COR3, $CO_2$R3, CONR3R4 ein oder mehrfach substituiert sein kann, oder Heterozyklyl, das mit F, Cl, Br, I, CN, $NO_2$, OH, -$CF_3$, ($C_1$-$C_6$)-Alkyl, OR3, NR3R4, COR3, $CO_2$R3, CONR3R4 ein oder mehrfach substituiert sein kann;
R3, R4 unabhängig voneinander H, ($C_1$-$C_6$)-Alkyl, -$CF_3$, ($C_3$-$C_{10}$)-Cycloalkyl, ($C_6$-$C_{10}$)-Aryl, Heterozyklyl, ($C_1$-$C_6$)-Alkylen-CONR5R6, ($C_1$-$C_6$)-Alkylen-$CO_2$R5, ($C_1$-$C_6$)-Alkylen-COR5, ($C_1$-$C_6$)-Alkylen-OR5, ($C_1$-$C_6$)-Alkylen-NR5R6, ($C_1$-$C_6$)-Alkylen-SR5, ($C_1$-$C_6$)-Alkylen-SOR5, ($C_1$-$C_6$)-Alkylen-$SO_2$R5, ($C_1$-$C_4$)-Alkylen-($C_6$-$C_{10}$)-Aryl oder ($C_1$-$C_4$)-Alkylen-Heterozyklyl;
R5, R6 unabhängig voneinander H, ($C_1$-$C_6$)-Alkyl, ($C_3$-$C_{10}$)-Cycloalkyl, ($C_1$-$C_6$)-Alkylen-($C_6$-$C_{10}$)-Aryl, -($C_6$-$C_{10}$)-Aryl, Heterozyklyl, ($C_1$-$C_6$)-Alkylen-($C_3$-$C_{10}$)-Heterozyklyl;
X S;

sowie deren physiologisch verträglichen Salze.

**4.** Verbindungen der Formel I, gemäß einem oder mehreren der Ansprüche 1 bis 3, **dadurch gekennzeichnet, dass** darin bedeuten

R1 H;
R2 H, ($C_1$-$C_{10}$)-Alkyl, ($C_3$-$C_{10}$)-Cycloalkyl, ($C_2$-$C_6$)-Alkenyl, ($C_2$-$C_6$)-Alkinyl, ($C_6$-$C_{10}$)-Aryl, ein Pyrrolidino-, Piperidino-, Hexamethylenimino-, Morpholino-, Piperazino-, Thiomorpholino- oder Homopiperazino-Rest; wobei die Alkyl-, Cycloalkyl-, Alkenyl-, Alkinyl-, Pyrrolidino-, Piperidino-, Hexamethylenimino-, Morpholino-, Piperazino-, Thiomorpholino-, Homopiperazino- und Aryl-Reste ein-oder mehrfach substituiert sein können mit F, Cl, Br, CN, $SF_5$, OH, ($C_1$-$C_6$)-Alkyl, -$CF_3$, ($C_2$-$C_6$)-Alkenyl, OR3, NR3R4, NR3CONR3R4, COR3, OCOR3, $CO_2$R3, CONR3R4, OCONR3R4, ($C_1$-$C_6$)-Alkylen-OR3, ($C_1$-$C_6$)-Alkylen-NR3R4, (C)-$C_6$)-Alkylen-NR3$SO_2$R4, ($C_1$-$C_6$)-Alkylen-SR3, Alkylen-S(O)R3, Alkylen-$S(O)_2$R3, Alkylen-$S(O)_2$NR3R4, ($C_1$-$C_6$)-Alkylen-COR3, ($C_1$-$C_6$)-Alkylen-$CO_2$R3, ($C_1$-$C_6$)-Alkylen-CONR3R4, SR3, SOR3, $SO_2$R3, $SO_2$NR3R4, NR3$SO_2$R4, ($C_1$-$C_6$)-Alkylen-($C_3C_{10}$)-Cycloalkyl, ($C_1$-$C_6$)-Alkylen-($C_6$-$C_{10}$)-Aryl, ($C_1$-$C_6$)-Alkylen-Heterozyklyl, ($C_3$-$C_{10}$)-Cycloalkyl, ($C_6$-$C_{10}$)-Aryl, das mit F, Cl, Br, I, CN, OH, -$CF_3$, ($C_1$-$C_6$)-Alkyl, OR3, NR3R4, COR3, CO2R3, CONR3R4 ein oder mehrfach substituiert sein kann, oder Heterozyklyl, das mit F, Cl, Br, CN, $NO_2$, OH, -$CF_3$, ($C_1$-$C_6$)-Alkyl, OR3, NR3R4, COR3, CO2R3, CONR3R4 ein oder mehrfach substituiert sein kann;
R3, R4 unabhängig voneinander H, ($C_1$-$C_6$)-Alkyl, -$CF_3$, ($C_3$-$C_{10}$)-Cycloalkyl, ($C_6$-$C_{10}$)-Aryl, Heterozyklyl, ($C_1$-$C_6$)-Alkylen-CONR5R6, ($C_1$-$C_6$)-Alkylen-COOR5, ($C_1$-$C_6$)-Alkylen-COR5, ($C_1$-$C_6$)-Alkylen-OR5, ($C_1$-$C_6$)-Alkylen-NR5R6, (C1-$C_6$)-Alkylen-SR5, ($C_1$-$C_6$)-Alkylen-S(O)R5, ($C_1$-$C_6$)-Alkylen-$S(O)_2$R5, ($C_1$-$C_4$)-Alkylen-($C_6$-$C_{10}$)-Aryl oder ($C_1$-$C_4$)-Alkylen-Heterozyklyl;
R5, R6 unabhängig voneinander H, ($C_1$-$C_6$)-Alkyl, ($C_3$-$C_{10}$)-Cycloalkyl, ($C_1$-$C_6$)-Alkylen-($C_6$-$C_{10}$)-Aryl, -($C_6$-$C_{10}$)-Aryl, Heterozyklyl, ($C_1$-$C_6$)-Alkylen-($C_3$-$C_{10}$)-Heterozyklyl;
X S;

sowie deren physiologisch verträglichen Salze.

**5.** Verbindungen der Formel I, gemäß einem oder mehreren der Ansprüche 1 bis 4, ladurch gekennzeichnet, dass darin bedeuten

R1 H;
R2 ($C_1$-$C_{10}$)-Alkyl, ($C_3$-$C_{10}$)-Cycloalkyl, Phenyl, ($C_1$-$C_6$)-Alkylen-Phenyl, ein Pyrrolidino-, Piperidino-, Hexamethylenimino-, Morpholino-, Piperazino-, Thiomorpholino- oder Homopiperazino-Rest;
X S;

sowie deren physiologisch verträglichen Salze.

6. Arzneimittel enthaltend eine oder mehrere der Verbindungen gemäß einem oder mehreren der Ansprüche 1 bis 5.

7. Arzneimittel enthaltend eine oder mehrere der Verbindungen gemäß einem oder mehreren der Ansprüche 1 bis 5 und mindestens einen weiteren Wirkstoff.

8. Arzneimittel, gemäß Anspruch 6, **dadurch gekennzeichnet, daß** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, Cannabinoid 1 Rezeptor Antagonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Serotonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, , Cannabinoid 1 Rezeptor Antagonisten , RXR-Modulatoren oder TR-β-Agonisten oder Amphetamine enthält.

9. Verwendung der Verbindungen einem oder mehreren der Ansprüche 1 bis 5 zur Herstellung eines Medikamentes zur Blutzuckersenkung.

10. Verwendung der Verbindungen gemäß einem oder mehreren der Ansprüche 1 bis 5 zur Herstellung eines Medikamentes zur Behandlung des Typ 2 Diabetes.

11. Verwendung der Verbindungen gemäß gemäß einem oder mehreren der Ansprüche 1 bis 5 zur Herstellung eines Medikamentes zur Behandlung von Lipid- und Kohlenhydratstoffwechselstörungen.

12. Verwendung der Verbindungen gemäß einem oder mehreren der Ansprüche 1 bis 5 zur Herstellung eines Medikaments zur Behandlung arteriosklerotischer Erscheinungen.

13. Verwendung der Verbindungen gemäß einem oder mehreren der Ansprüche 1 bis 5 zur Herstellung eines Medikaments zur Behandlung von Insulin Resistenz.

14. Verfahren zur Herstellung eines Arzneimittels enthaltend eine oder mehrere der Verbindungen gemäß einem oder mehreren der Ansprüche 1 bis 5, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. Compounds of the formula I,

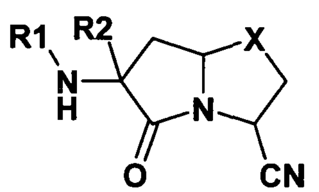

formula I in which the meanings are

R1 H, $(C_1\text{-}C_{10})$ -alkyl, $(C_3\text{-}C_{10})$ -cycloalkyl, $(C_2\text{-}C_6)$-alkenyl, $(C_2\text{-}C_6)$-alkynyl, $(C_6\text{-}C_{10})$ -aryl, heterocyclyl, where

the alkyl, cycloalkyl, alkenyl, alkynyl, aryl and heterocyclyl radicals may be substituted one or more times by F, Cl, Br, I, CN, $NO_2$, OH, $-CF_3$, $(C_1-C_6)$-alkyl, $(C_2-C_6)$-alkenyl, $(C_2-C_6)$ -alkynyl, OR3, NR3R4, COR3, CO2R3, CONR3R4, $(C_1-C_6)$-alkylene-OR3, $(C_1-C_6)$-alkylene-NR3R4, $(C_1-C_6)$-alkylene-SR3, alkylene-SOR3, alkylene-$SO_2$R3, $(C_1-C_6)$-alkylene-COR3, $(C_1-C_6)$ -alkylene-COOR3, $(C_1-C_6)$-alkylene-CONR3R4, $(C_1-C_6)$-alkylene-O-P(O) $(OR3)_2$, SR3, SOR3, $SO_2$NR3R4, $SO_2$R3, $(C_1-C_6)$ -alkylene-$(C_3-C_{10})$ -cycloalkyl, $(C_1-C_6)$-alkylene-$(C_6-C_{10})$ -aryl, $(C_1-C_6)$ -alkylene-heterocyclyl, $(C_3-C_{10})$-cycloalkyl, $(C_6-C_{10})$ -aryl which may be substituted one or more times by F, Cl, Br, I, CN, $NO_2$, OH, $-CF_3$, $(C_1-C_6)$-alkyl, $(C_2-C_6)$ -alkenyl, $(C_2-C_6)$ -alkynyl, OR3, NR3R4, COR3, $CO_2$R3, CONR3R4, or heterocyclyl which may be substituted one or more times by F, Cl, Br, I, CN, $NO_2$, OH, $-CF_3$, $(C_1-C_6)$-alkyl, OR3, NR3R4, COR3, $CO_2$R3, CONR3R4;

R2 H, $(C_1-C_{10})$-alkyl, $(C_3-C_{10})$ -cycloalkyl, $(C_2-C_6)$-alkenyl, $(C_2-C_6)$-alkynyl, $(C_6-C_{10})$-aryl, heterocyclyl, COR3, COOR3, CONR3R4, CN, where the alkyl, cycloalkyl, alkenyl, alkynyl, aryl and heterocyclyl radicals may be substituted one or more times by F, Cl, Br, I, CN, $NO_2$, SH, SF5, OH, $(C_1-C_6)$-alkyl, $-CF_3$, $(C_2-C_6)$-alkenyl, $(C_2-C_6)$-alkynyl, OR3, OP(O) $(OR3)_2$, NR3R4, NR3CONR3R4, COR3, OCOR3, OCOOR3, COOR3, CONR3R4, OCONR3R4, $(C_1-C_6)$-alkylene-OR3, $(C_1-C_6)$-alkylene-NR3R4, $(C_1-C_6)$-alkylene-NR3$SO_2$R4, $(C_1-C_6)$-alkylene-SR3, alkylene-S(O)R3, alkylene-$S(O)_2$R3, alkylene-$S(O)_2$NR3R4, $(C_1-C_6)$-alkylene-COR3, $(C_1-C_6)$-alkylene-COOR3, $(C_1-C_6)$-alkylene-CONR3R4, SR3, SOR3, $SO_2$R3, $SO_2$NR3R4, NR3$SO_2$R4, $(C_1-C_6)$-alkylene- $(C_3-C_{10})$-cycloalkyl, $(C_1-C_6)$-alkylene- $(C_6-C_{10})$ -aryl, $(C_1-C_6)$-alkylene-heterocyclyl, $(C_3-C_{10})$-cycloalkyl, $(C_6-C_{10})$-aryl or heterocyclyl;

R3, R4 independently of one another H, $(C_1-C_6)$-alkyl, $-CF_3$, $(C_3-C_{10})$ -cycloalkyl, $(C_6-C_{10})$ -aryl, heterocyclyl, $(C_1-C_6)$-alkylene-CONR5R6, CONR5R6, $(C_1-C_6)$-alkylene-COOR5, COOR5, COR5, $(C_1-C_6)$-alkylene-COR5, $(C_1-C_6)$-alkylene-OR5, $(C_1-C_6)$-alkylene-NR5R6, $(C_1-C_6)$-alkylene-SR5, $(C_1-C_6)$-alkylene-S (O) R5, $(C_1-C_6)$-alkylene-$S(O)_2$R5, S(O)R5, $S(O)_2$R5, $(C_1-C_4)$ -alkylene- $(C_6-C_{10})$-aryl or $(C_1-C_4)$-alkylene-heterocyclyl;

R5, R6 independently of one another H, $(C_1-C_6)$-alkyl, $(C_1-C_6)$ -alkylene- $(C_6-C_{10})$ -aryl, $(C_6-C_{10})$ -aryl, heterocyclyl, $(C_1-C_6)$-alkylene-heterocyclyl;

X S, SO, $SO_2$;

and the physiologically tolerated salts thereof.

**2.** Compounds of the formula I according to Claim 1, **characterized in that** the meanings therein are

R1 H, $(C_1-C_{10})$-alkyl, $(C_3-C_{10})$-cycloalkyl, $(C_2-C_6)$-alkenyl, $(C_2-C_6)$ -alkynyl, $(C_6-C_{10})$ -aryl, where the alkyl, cycloalkyl, alkenyl, alkynyl and aryl radicals may be substituted one or more times by F, Cl, Br, I, CN, $NO_2$, OH, $-CF_3$, $(C_1-C_6)$ -alkyl, $(C_2-C_6)$ -alkenyl, $(C_2-C_6)$ -alkynyl, OR3 , NR3R4, COR3 , $CO_2$R3, CONR3R4, $(C_1-C_6)$-alkylene-OR3, $(C_1-C_6)$-alkylene-NR3R4, $(C_1-C_6)$-alkylene-SR3, alkylene-SOR3, alkylene-$SO_2$R3, $(C_1-C_6)$-alkylene-COR3, $(C_1-C_6)$-alkylene-$CO_2$R3, $(C_1-C_6)$-alkylene-CONR3R4, $(C_1-C_6)$-alkylene-O-P(O) $(OR3)_2$, SR3, SOR3 , $SO_2$NR3R4, $SO_2$R3, $(C_1-C_6)$ -alkylene- $(C_3-C_{10})$ -cycloalkyl, $(C_1-C_6)$-alkylene- $(C_6-C_{10})$ -aryl, $(C_1-C_6)$ -alkylene-heterocyclyl, $(C_3-C_{10})$-cycloalkyl or $(C_6-C_{10})$-aryl which may be substituted one or more times by F, Cl, Br, I, CN, $NO_2$, OH, $-CF_3$, $(C_1-C_6)$ -alkyl, $(C_2-C_6)$ -alkenyl, $(C_2-C_6)$ -alkynyl, OR3, NR3R4, COR3, $CO_2$R3, CONR3R4;

R2 H, $(C_1-C_{10})$ -alkyl, $(C_3-C_{10})$ -cycloalkyl, $(C_2-C_6)$-alkenyl, $(C_2-C_6)$ -alkynyl, $(C_6-C_{10})$ -aryl, heterocyclyl, COR3, CO2R3, CONR3R4, CN, where the alkyl, cycloalkyl, alkenyl, alkynyl, aryl and heterocyclyl radicals may be substituted one or more times by F, Cl, Br, I, CN, $NO_2$, SH, $SF_5$, OH, $(C_1-C_6)$ -alkyl, $-CF_3$, $(C_2-C_6)$-alkenyl, $(C_2-C_6)$ -alkynyl, OR3, OP (O) $(OR3)_2$, NR3R4, NR3CONR3R4, COR3, OCOR3, $CO_2$R3 CONR3R4, OCONR3R4, $(C_1-C_6)$ -alkylene-OR3, $(C_1-C_6)$ -alkylene-NR3R4, $(C_1-C_6)$-alkylene-NR3$SO_2$R4, $(C_1-C_6)$-alkylene-SR3, alkylene-SOR3, alkylene-$SO_2$R3, alkylene-$SO_2$NR3R4, $(C_1-C_6)$ -alkylene-COR3, $(C_1-C_6)$ -alkylene-$CO_2$R3, $(C_1-C_6)$ -alkylene-CONR3R4, SR3, SOR3, $SO_2$R3, $SO_2$NR3R4, NR3$SO_2$R4, $(C_1-C_6)$ -alkylene- $(C_3-C_{10})$ -cycloalkyl, $(C_1-C_6)$-alkylene- $(C_6-C_{10})$ -aryl, $(C_1-C_6)$ -alkylene-heterocyclyl, $(C_3-C_{10})$ -cycloalkyl, $(C_6-C_{10})$-aryl, which may be substituted one or more times by F, Cl, Br, I, CN, $NO_2$, OH, $-CF_3$, $(C_1-C_6)$ -alkyl, $(C_2-C_6)$ -alkenyl, $(C_2-C_6)$ -alkynyl, OR3, NR3R4, COR3, $CO_2$R3, CONR3R4, or heterocyclyl which may be substituted one or more times by F, Cl, Br, I, CN, $NO_2$, OH, $-CF_3$, $(C_1-C_6)$-alkyl, OR3, NR3R4, COR3, $CO_2$R3, CONR3R4;

R3, R4 independently of one another H, $(C_1-C_6)$ -alkyl, $-CF_3$, $(C_3-C_{10})$ -cycloalkyl, $(C_6-C_{10})$ -aryl, heterocyclyl, $(C_1-C_6)$-alkylene-CONR5R6, $(C_1-C_6)$ -alkylene-$CO_2$R5, $(C_1-C_6)$ -alkylene-COR5, $(C_1-C_6)$ -alkylene-OR5, $(C_1-C_6)$ -alkylene-NR5R6, $(C_1-C_6)$ -alkylene-SR5, $(C_1-C_6)$ -alkylene-SORS, $(C_1-C_6)$ -alkylene-$SO_2$R5, $(C_1-C_4)$ -alkylene- $(C_6-C_{10})$-aryl or $(C_1-C_4)$-alkylene-heterocyclyl;

R5, R6 independently of one another H, $(C_1-C_6)$-alkyl, $(C_3-C_{10})$ -cycloalkyl, $(C_1-C_6)$ -alkylene-$(C_6-C_{10})$ -aryl, -$(C_6-C_{10})$-aryl, heterocyclyl, $(C_1-C_6)$ -alkylene-$(C_3-C_{10})$-heterocyclyl;

X S;

and the physiologically tolerated salts thereof.

**3.** Compounds of the formula I according to Claim 1 or 2, **characterized in that** the meanings therein are

R1 H;
R2 H, ($C_1$-$C_{10}$)-alkyl, ($C_3$-$C_{10}$)-cycloalkyl, ($C_2$-$C_6$)-alkenyl, ($C_2$-$C_6$) -alkynyl, ($C_6$-$C_{10}$) -aryl, heterocyclyl, COR3, $CO_2$R3, CONR3R4, CN, where the alkyl, cycloalkyl, alkenyl, alkynyl, aryl and heterocyclyl radicals may be substituted one or more times by F, Cl, Br, I, CN, $NO_2$, SH, $SF_5$, OH, ($C_1$-$C_6$) -alkyl, -$CF_3$, ($C_2$-$C_6$)-alkenyl, ($C_2$-$C_6$) -alkynyl, OR3, OP(O) (OR3) 2, NR3R4, NR3CONR3R4, COR3, OCOR3, $CO_2$R3, CONR3R4, OCONR3R4, ($C_1$-$C_6$)-alkylene-OR3, ($C_1$-$C_6$)-alkylene-NR3R4, ($C_1$-$C_6$)-alkylene-NR3$SO_2$R4, ($C_1$-$C_6$)-alkylene-SR3, alkylene-SOR3, alkylene-$SO_2$R3, alkylene-$S(O)_2$NR3R4, ($C_1$-$C_6$) -alkylene-COR3, ($C_1$-$C_6$)-alkylene-$CO_2$R3, ($C_1$-$C_6$) -alkylene-CONR3R4, SR3, SOR3, $SO_2$R3, $SO_2$NR3R4, NR3$SO_2$R4, ($C_1$-$C_6$) -alkylene- ($C_3$-$C_{10}$) -cycloalkyl, ($C_1$-$C_6$)-alkylene-($C_6$-$C_{10}$)-aryl, ($C_1$-$C_6$)-alkylene-heterocyclyl, ($C_3$-$C_{10}$) -cycloalkyl, ($C_6$-$C_{10}$) -aryl which may be substituted one or more times by F, Cl, Br, I, CN, $NO_2$, OH, -$CF_3$, ($C_1$-$C_6$) -alkyl, ($C_2$-$C_6$)-alkenyl, ($C_2$-$C_6$) -alkynyl, OR3, NR3R4, COR3, $CO_2$R3, CONR3R4, or heterocyclyl which may be substituted one or more times by F, Cl, Br, I, CN, $NO_2$, OH, -$CF_3$, ($C_1$-$C_6$)-alkyl, OR3, NR3R4, COR3, $CO_2$R3, CONR3R4;
R3, R4 independently of one another H, ($C_1$-$C_6$)-alkyl, -$CF_3$, ($C_3$-$C_{10}$) -cycloalkyl, ($C_6$-$C_{10}$) -aryl, heterocyclyl, ($C_1$-$C_6$)-alkylene-CONR5R6, ($C_1$-$C_6$) -alkylene-$CO_2$R5, ($C_1$-$C_6$) -alkylene-COR5, ($C_1$-$C_6$) -alkylene-OR5, ($C_1$-$C_6$) -alkylene-NR5R6, ($C_1$-$C_6$) -alkylene-SR5, ($C_1$-$C_6$) -alkylene-SOR5, ($C_1$-$C_6$)-alkylene-$SO_2$R5, ($C_1$-$C_4$) -alkylene- ($C_6$-$C_{10}$)-aryl or ($C_1$-$C_4$)-alkylene-heterocyclyl;
R5, R6 independently of one another H, ($C_1$-$C_6$)-alkyl, ($C_3$-$C_{10}$) -cycloalkyl, ($C_1$-$C_6$) -alkylene-($C_6$-$C_{10}$) -aryl, -($C_6$-$C_{10}$) -aryl, heterocyclyl, ($C_1$-$C_6$)-alkylene-($C_3$-$C_{10}$)-heterocyclyl;
X S;

and the physiologically tolerated salts thereof.

**4.** Compounds of the formula I according to one or more of Claims 1 to 3, **characterized in that** the meanings therein are

R1 H;
R2 H, ($C_1$-$C_{10}$)-alkyl, ($C_3$-$C_{10}$) -cycloalkyl, ($C_2$-$C_6$)-alkenyl, ($C_2$-$C_6$)-alkynyl, ($C_6$-$C_{10}$) -aryl, a pyrrolidino, piperidino, hexamethyleneimino, morpholino, piperazino, thiomorpholino or homopiperazino radical; where the alkyl, cycloalkyl, alkenyl, alkynyl, pyrrolidino, piperidino, hexamethyleneimino, morpholino, piperazino, thiomorpholino, homopiperazino and aryl radicals may be substituted one or more times by F, Cl, Br, CN, $SF_5$, OH, ($C_1$-$C_6$) -alkyl, -$CF_3$, ($C_2$-$C_6$) -alkenyl, OR3, NR3R4, NR3CONR3R4, COR3 , OCOR3 , $CO_2$R3, CONR3R4, OCONR3R4, ($C_1$-$C_6$)-alkylene-OR3, ($C_1$-$C_6$)-alkylene-NR3R4, ($C_1$-$C_6$)-alkylene-NR3$SO_2$R4, ($C_1$-$C_6$)-alkylene-SR3, alkylene-S(O)R3, alkylene-$S(O)_2$R3, alkylene-$S(O)_2$NR3R4, ($C_1$-$C_6$) -alkylene-COR3, ($C_1$-$C_6$)-alkylene-$CO_2$R3, ($C_1$-$C_6$)-alkylene-CONR3R4, SR3, SOR3, $SO_2$R3, $SO_2$NR3R4, NR3$SO_2$R4, ($C_1$-$C_6$)-alkylene- ($C_3$-$C_{10}$) -cycloalkyl, ($C_1$-$C_6$) -alkylene-($C_6$-$C_{10}$) -aryl, ($C_1$-$C_6$) -alkylene-heterocyclyl, ($C_3$-$C_{10}$)-cycloalkyl, ($C_6$-$C_{10}$)-aryl which may be substituted one or more times by F, Cl, Br, I, CN, OH, -$CF_3$, ($C_1$-$C_6$) -alkyl, OR3, NR3R4, COR3, CO2R3, CONR3R4, or heterocyclyl which may be substituted one or more times by F, Cl, Br, CN, $NO_2$, OH, -$CF_3$, ($C_1$-$C_6$)-alkyl, OR3, NR3R4, COR3, CO2R3, CONR3R4;
R3, R4 independently of one another H, ($C_1$-$C_6$)-alkyl, -$CF_3$, ($C_3$-$C_{10}$)-cycloalkyl, ($C_6$-$C_{10}$)-aryl, heterocyclyl, ($C_1$-$C_6$)-alkylene-CONR5R6, ($C_1$-$C_6$)-alkylene-COOR5, ($C_1$-$C_6$)-alkylene-COR5, ($C_1$-$C_6$)-alkylene-OR5, ($C_1$-$C_6$)-alkylene-NR5R6, (C1-$C_6$)-alkylene-SR5, ($C_1$-$C_6$)-alkylene-S(O)R5, ($C_1$-$C_6$) -alkylene-$S(O)_2$RS, ($C_1$-$C_4$) -alkylene- ($C_6$-$C_{10}$)-aryl ($C_1$-$C_4$)-alkylene-heterocyclyl;
R5, R6 independently of one another H, ($C_1$-$C_6$)-alkyl, ($C_3$-$C_{10}$) -cycloalkyl, ($C_1$-$C_6$) -alkylene-($C_6$-$C_{10}$)-aryl, -($C_6$-$C_{10}$)-aryl, heterocyclyl, ($C_1$-$C_6$)-alkylene-($C_3$-$C_{10}$)-heterocyclyl;
X S;

and the physiologically tolerated salts thereof.

**5.** Compounds of the formula I according to one or more of Claims 1 to 4, **characterized in that** the meanings therein are

R1 H;
R2 ($C_1$-$C_{10}$)-alkyl, ($C_3$-$C_{10}$)-cycloalkyl, phenyl, ($C_1$-$C_6$)-alkylene-phenyl, a pyrrolidino, piperidino, hexamethyleneimino, morpholino, piperazino, thiomorpholino or homopiperazino radical;
X S;

and the physiologically tolerated salts thereof.

**6.** Medicament comprising one or more of the compounds according to one or more of Claims 1 to 5.

**7.** Medicament comprising one or more of the compounds according to one or more of Claims 1 to 5 and at least one other active ingredient.

**8.** Medicament according to Claim 6, **characterized in that** it comprises as other active ingredient one or more antidiabetics, hypoglycemic active ingredients, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, CART agonists, NPY agonists, MC4 agonists, orexin agonists, cannabinoid 1 receptor antagonists, H3 agonists, TNF agonists, CRF agonists, CRF BP antagonists, urocortin agonists, β3 agonists, MSH (melanocyte-stimulating hormone) agonists, CCK agonists, serotonin reuptake inhibitors, mixed sertoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, cannabinoid 1 receptor antagonists, RXR modulators or TR-β agonists or amphetamines.

**9.** Use of the compounds according to one or more of Claims 1 to 5 for producing a medicament for reducing blood glucose.

**10.** Use of the compounds according to one or more of Claims 1 to 5 for producing a medicament for the treatment of type 2 diabetes.

**11.** Use of the compounds according to one or more of Claims 1 to 5 for producing a medicament for the treatment of disturbances of lipid and carbohydrate metabolism.

**12.** Use of the compounds according to one or more of Claims 1 to 5 for producing a medicament for the treatment of arteriosclerotic manifestations.

**13.** Use of the compounds according to one or more of Claims 1 to 5 for producing a medicament for the treatment of insulin resistance.

**14.** Process for producing a medicament comprising one or more of the compounds according to one or more of Claims 1 to 5, **characterized in that** the active ingredient is mixed with a pharmaceutically suitable carrier and this mixture is converted into a form suitable for administration.

### Revendications

**1.** Composés de formule I

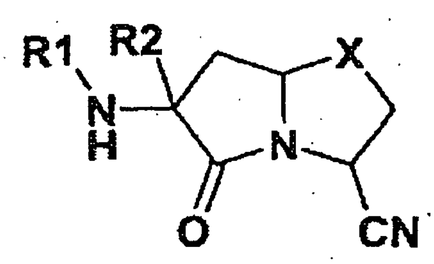

Formule I

dans laquelle

R1 est H, un groupe alkyle en $C_1$-$C_{10}$, cycloalkyle en $C_3$-$C_{10}$, alcényle en $C_2$-$C_6$, alcynyle en $C_2$-$C_6$, aryle en $C_6$-$C_{10}$, hétérocyclyle, les radicaux alkyle, cycloalkyle, alcényle, alcynyle, aryle et hétérocyclyle pouvant être une ou plusieurs fois substitués par un ou des substituants F, Cl, Br, I, CN, $NO_2$, OH, -$CF_3$, alkyle en $C_1$-$C_6$, alcényle en $C_2$-$C_6$, alcynyle en $C_2$-$C_6$, OR3, NR3R4, COR3, CO2R3, CONR3R4, (alkylène en $C_1$-$C_6$)-OR3, (alkylène en $C_1$-$C_6$)-NR3R4, (alkylène en $C_1$-$C_6$)-SR3, alkylène-SOR3, alkylène-$SO_2$R3, (alkylène en $C_1$-$C_6$)-COR3, (alkylène en $C_1$-$C_6$)-COOR3, (alkylène en $C_1$-$C_6$)-CONR3R4, (alkylène en $C_1$-$C_6$)-O-P(O)$(OR3)_2$, SR3, SOR3, $SO_2$NR3R4, $SO_2$R3, (alkylène en $C_1$-$C_6$) - (cycloalkyle en $C_3$-$C_{10}$), (alkylène en $C_1$-$C_6$)-(aryle en $C_6$-$C_{10}$), (alkylène en $C_1$-$C_6$)-hétérocyclyle, cycloalkyle en $C_3$-$C_{10}$, aryle en $C_6$-$C_{10}$, qui peut être une ou plusieurs fois substitué par un ou des substituants F, Cl, Br, I, CN, $NO_2$, OH, -$CF_3$, alkyle en $C_1$-$C_6$, alcényle en $C_2$-$C_6$, alcynyle en $C_2$-$C_6$, OR3, NR3R4, COR3, $CO_2$R3, CONR3R4 ; ou hétérocyclyle, qui peut être une ou plusieurs fois substitué par un ou des substituants F, Cl, Br, I, CN, $NO_2$, OH, -$CF_3$, alkyle en $C_1$-$C_6$, OR3, NR3R4, COR3 , $CO_2$R3, CONR3R4 ;

R2 est H, un groupe alkyle en $C_1$-$C_{10}$, cycloalkyle en $C_3$-$C_{10}$, alcényle en $C_2$-$C_6$, alcynyle en $C_2$-$C_6$, aryle en $C_6$-$C_{10}$, hétérocyclyle, COR3, COOR3, CONR3R4, CN, les radicaux alkyle, cycloalkyle, alcényle, alcynyle, aryle et hétérocyclyle pouvant être une ou plusieurs fois substitués par un ou des substituants F, Cl, Br, I, CN, $NO_2$, SH, $SF_5$, OH, alkyle en $C_1$-$C_6$, -$CF_3$, alcényle en $C_2$-$C_6$, alcynyle en $C_2$-$C_6$, OR3, OP(O)$(OR3)_2$, NR3R4, NR3CONR3R4, COR3, OCOR3, OCOOR3, COOR3, CONR3R4, OCONR3R4, (alkylène en $C_1$-$C_6$)-OR3, (alkylène en $C_1$-$C_6$)-NR3R4, (alkylène- en $C_1$-$C_6$) -NR3$SO_2$R4, (alkylène en $C_1$-$C_6$) - SR3, alkylène-S(O)R3, alkylène-S$(O)_2$R3, alkylène-S$(O)_2$NR3R4, (alkylène en $C_1$-$C_6$)-COR3, (alkylène en $C_1$-$C_6$)-COOR3, (alkylène en $C_1$-$C_6$)-CONR3R4, SR3, SOR3, $SO_2$R3, $SO_2$NR3R4, NR3$SO_2$R4, (alkylène en $C_1$-$C_6$) - (cycloalkyle en $C_3$-$C_{10}$), (alkylène en $C_1$-$C_6$)-(aryle en $C_6$-$C_{10}$), (alkylène en $C_1$-$C_6$) -hétérocyclyle, cycloalkyle en $C_3$-$C_{10}$, aryle en $C_6$-$C_{10}$ ou hétérocyclyle ;

R3 et R4 représentent chacun indépendamment de l'autre H, un groupe alkyle en $C_1$-$C_6$, -$CF_3$, cycloalkyle en $C_3$-$C_{10}$, aryle en $C_6$-$C_{10}$, hétérocyclyle, (alkylène en $C_1$-$C_6$)-CONR5R6, CONR5R6, (alkylène en $C_1$-$C_6$)-COOR5, COOR5, COR5 , (alkylène en $C_1$-$C_6$)-COR5, (alkylène en $C_1$-$C_6$)-OR5, (alkylène en $C_1$-$C_6$)-NR5R6, (alkylène en $C_1$-$C_6$)-SR5, (alkylène en $C_1$-$C_6$)-S(O)R5, (alkylène en $C_1$-$C_6$)-S$(O)_2$R5, S(O)R5, S$(O)_2$R5, (alkylène en $C_1$-$C_4$) - (aryle en $C_6$-$C_{10}$) ou (alkylène en $C_1$-$C_4$)-hétérocyclyle ;

R5 et R6 représentent chacun indépendamment de l'autre H, un groupe alkyle en $C_1$-$C_6$, (alkylène en $C_1$-$C_6$) - (aryle en $C_6$-$C_{10}$), aryle en $C_6$-$C_{10}$, hétérocyclyle, (alkylène en $C_1$-$C_6$)-hétérocyclyle ;

X est S, SO, $SO_2$ ;

ainsi que leurs sels physiologiquement tolérés.

**2.** Composés de formule I selon la revendication 1, **caractérisés en ce que**, dans cette formule R1 est H, un groupe alkyle en $C_1$-$C_{10}$, cycloalkyle en $C_3$-$C_{10}$, alcényle en $C_2$-$C_6$, alcynyle en $C_2$-$C_6$, aryle en $C_6$-$C_{10}$, les radicaux alkyle, cycloalkyle, alcényle, alcynyle et aryle pouvant être une ou plusieurs fois substitués par un ou des substituants F, Cl, Br, I, CN, $NO_2$, OH, -$CF_3$, alkyle en $C_1$-$C_6$, alcényle en $C_2$-$C_6$, alcynyle en $C_2$-$C_6$, OR3, NR3R4, COR3, $CO_2$R3, CONR3R4,

(alkylène en $C_1$-$C_6$)-OR3, (alkylène en $C_1$-$C_6$)-NR3R4, (alkylène en $C_1$-$C_6$)-SR3, alkylène-SOR3, alkylène-$SO_2$R3, (alkylène en $C_1$-$C_6$)-COR3, (alkylène en $C_1$-$C_6$)-$CO_2$R3, (alkylène en $C_1$-$C_6$)-CONR3R4, (alkylène en $C_1$-$C_6$)-O-P(O)$(OR3)_2$, SR3, SOR3, $SO_2$NR3R4, $SO_2$R3, (alkylène en $C_1$-$C_6$)-(cycloalkyle en $C_3$-$C_{10}$), (alkylène en $C_1$-$C_6$)-(aryle en $C_6$-$C_{10}$), (alkylène en $C_1$-$C_6$) -hétérocyclyle, cycloalkyle en $C_3$-$C_{10}$ ou aryle en $C_6$-$C_{10}$, qui peut être une ou plusieurs fois substitué par un ou des substituants F, Cl, Br, I, CN, $NO_2$, OH, -$CF_3$, alkyle en $C_1$-$C_6$, alcényle en $C_2$-$C_6$, alcynyle en $C_2$-$C_6$, OR3, NR3R4, COR3, $CO_2$R3, CONR3R4 ;

R2 est H, un groupe alkyle en $C_1$-$C_{10}$, cycloalkyle en $C_3$-$C_{10}$, alcényle en $C_2$-$C_6$, alcynyle en $C_2$-$C_6$, aryle en $C_6$-$C_{10}$, hétérocyclyle, COR3, CO2R3, CONR3R4, CN, les radicaux alkyle, cycloalkyle, alcényle, alcynyle, aryle et hétérocyclyle pouvant être une ou plusieurs fois substitués par un ou des substituants F, Cl, Br, I, CN, $NO_2$, SH, $SF_5$, OH, alkyle en $C_1$-$C_6$, -$CF_3$, alcényle en $C_2$-$C_6$, alcynyle en $C_2$-$C_6$, OR3, OP(O)$(OR3)_2$, NR3R4, NR3CONR3R4, COR3, OCOR3, $CO_2$R3, CONR3R4, OCONR3R4, (alkylène en $C_1$-$C_6$) -OR3, (alkylène en $C_1$-$C_6$) -NR3R4, (alkylène en $C_1$-$C_6$)-NR3$SO_2$R4, (alkylène en $C_1$-$C_6$)-SR3, alkylène-SOR3, alkylène-$SO_2$R3, alkylène-$SO_2$NR3R4, (alkylène en $C_1$-$C_6$)-COR3, (alkylène en $C_1$-$C_6$)-$CO_2$R3, (alkylène en $C_1$-$C_6$)-CONR3R4, SR3, SOR3, $SO_2$R3, $SO_2$NR3R4, NR3$SO_2$R4, (alkylène en $C_1$-$C_6$) - (cycloalkyle en $C_3$-$C_{10}$), (alkylène en $C_1$-$C_6$)-(aryle en $C_6$-$C_{10}$), (alkylène en $C_1$-$C_6$)-hétérocyclyle, cycloalkyle en $C_3$-$C_{10}$, aryle en $C_6$-$C_{10}$, qui peut être une ou plusieurs fois substitué par un ou des substituants F, Cl, Br, I, CN, $NO_2$, OH, -$CF_3$, alkyle en $C_1$-$C_6$, alcényle en $C_2$-$C_6$, alcynyle en $C_2$-$C_6$, OR3, NR3R4, COR3, $CO_2$R3, CONR3R4, ou hétérocyclyle, qui peut être une ou plusieurs fois substitué par un ou des substituants F, Cl, Br, I, CN, $NO_2$, OH, -$CF_3$, alkyle en $C_1$-$C_6$, OR3 , NR3R4, COR3, $CO_2$R3, CONR3R4 ;

R3 et R4 représentent chacun indépendamment de l'autre H, un groupe alkyle en $C_1$-$C_6$, -$CF_3$, cycloalkyle en $C_3$-$C_{10}$, aryle en $C_6$-$C_{10}$, hétérocyclyle, (alkylène en $C_1$-$C_6$)-CONR5R6, (alkylène en $C_1$-$C_6$)-$CO_2$R5, (alkylène en $C_1$-$C_6$)-COR5, (alkylène en $C_1$-$C_6$)-OR5, (alkylène en $C_1$-$C_6$)-NR5R6, (alkylène en $C_1$-$C_6$)-SR5, (alkylène en $C_1$-$C_6$)-SOR5, (alkylène en $C_1$-$C_6$)-$SO_2$R5, (alkylène en $C_1$-$C_4$)-(aryle en $C_6$-$C_{10}$) ou (alkylène en $C_1$-$C_4$)-hétérocy-

clyle;
R5 et R6 représentent chacun indépendamment de l'autre H, un groupe alkyle en $C_1$-$C_6$, cycloalkyle en $C_3$-$C_{10}$, (alkylène en $C_1$-$C_6$)-(aryle en $C_6$-$C_{10}$) -(aryle en $C_6$-$C_{10}$), hétérocyclyle, (alkylène en $C_1$-$C_6$)-(hétérocyclyle en $C_3$-$C_{10}$);
X est S ;
ainsi que leurs sels physiologiquement tolérés.

**3.** Composés de formule I selon la revendication 1 ou 2, **caractérisés en ce que**, dans cette formule
R1 est H ;
R2 est H, un groupe alkyle en $C_1$-$C_{10}$, cycloalkyle en $C_3$-$C_{10}$, alcényle en $C_2$-$C_6$, alcynyle en $C_2$-$C_6$, aryle en $C_6$-$C_{10}$, hétérocyclyle, COR3, $CO_2R3$, CONR3R4, CN, les radicaux alkyle, cycloalkyle, alcényle, alcynyle, aryle et hétérocyclyle pouvant être une ou plusieurs fois substitués par un ou des substituants F, Cl, Br, I, CN, $NO_2$, SH, $SF_5$, OH, alkyle en $C_1$-$C_6$, $-CF_3$, alcényle en $C_2$-$C_6$, alcynyle en $C_2$-$C_6$, OR3, OP(O)(OR3)2, NR3R4, NR3CONR3R4, COR3, OCOR3, $CO_2R3$, CONR3R4, OCONR3R4, (alkylène en $C_1$-$C_6$)-OR3, (alkylène en $C_1$-$C_6$)-NR3R4, (alkylène en $C_1$-$C_6$)-$NR3SO_2R4$, (alkylène en $C_1$-$C_6$)-SR3, alkylène-SOR3, alkylène-$SO_2R3$, alkylène-$S(O)_2NR3R4$, (alkylène en $C_1$-$C_6$)-COR3, (alkylène en $C_1$-$C_6$)-$CO_2R3$, (alkylène en $C_1$-$C_6$)-CONR3R4, SR3, SOR3, $SO_2R3$, $SO_2NR3R4$, $NR3SO_2R4$, (alkylène en $C_1$-$C_6$)-(cycloalkyle en $C_3$-$C_{10}$), (alkylène en $C_1$-$C_6$)-(aryle en $C_6$-$C_{10}$), (alkylène en $C_1$-$C_6$)-hétérocyclyle, cycloalkyle en $C_3$-$C_{10}$, aryle en $C_6$-$C_{10}$, qui peut être une ou plusieurs fois substitué par un ou des substituants F, Cl, Br, I, CN, $NO_2$, OH, $-CF_3$, alkyle en $C_1$-$C_6$, alcényle en $C_2$-$C_6$, alcynyle en $C_2$-$C_6$, OR3, NR3R4, COR3, $CO_2R3$, CONR3R4 ; ou hétérocyclyle, qui peut être une ou plusieurs fois substitué par un ou des substituants F, Cl, Br, I, CN, $NO_2$, OH, $-CF_3$, alkyle en $C_1$-$C_6$, OR3, NR3R4, COR3 , $CO_2R3$, CONR3R4 ;
R3 et R4 représentent chacun indépendamment de l'autre H, un groupe alkyle en $C_1$-$C_6$, $-CF_3$, cycloalkyle en $C_3$-$C_{10}$, aryle en $C_6$-$C_{10}$, hétérocyclyle, (alkylène en $C_1$-$C_6$) - CONR5R6, (alkylène en $C_1$-$C_6$)-$CO_2R5$, (alkylène en $C_1$-$C_6$)-COR5, (alkylène en $C_1$-$C_6$)-OR5, (alkylène en $C_1$-$C_6$)-NR5R6, (alkylène en $C_1$-$C_6$)-SR5, (alkylène en $C_1$-$C_6$)-SOR5, (alkylène en $C_1$-$C_6$)-$SO_2R5$, (alkylène en $C_1$-$C_4$)-(aryle en $C_6$-$C_{10}$) ou (alkylène en $C_1$-$C_4$)-hétérocyclyle ;
R5 et R6 représentent chacun indépendamment de l'autre H, un groupe alkyle en $C_1$-$C_6$, cycloalkyle en $C_3$-$C_{10}$, (alkylène en $C_1$-$C_6$)-(aryle en $C_6$-$C_{10}$), -(aryle en $C_6$-$C_{10}$), hétérocyclyle, (alkylène en $C_1$-$C_6$) - (hétérocyclyle en $C_3$-$C_{10}$) ;
X est S ;
ainsi que leurs sels physiologiquement tolérés.

**4.** Composés de formule I selon l'une ou plusieurs des revendications 1 à 3, **caractérisés en ce que**, dans ces formules,
R1 est H ;
R2 est H, un groupe alkyle en $C_1$-$C_{10}$, cycloalkyle en $C_3$-$C_{10}$, alcényle en $C_2$-$C_6$, alcynyle en $C_2$-$C_6$, aryle en $C_6$-$C_{10}$ ; un radical pyrrolidino, pipéridino, hexaméthylène-imino, morpholino, pipérazino, thiomorpholino ou homopipérazino ; les radicaux alkyle, cycloalkyle, alcényle, alcynyle, pyrrolidino, pipéridino, hexaméthylène-imino, morpholino, pipérazino, thiomorpholino, homopipérazino et aryle pouvant être une ou plusieurs fois substitués par un ou des substituants F, Cl, Br, CN, $SF_5$, OH, alkyle en $C_1$-$C_6$, $-CF_3$, alcényle en $C_2$-$C_6$, OR3, NR3R4, NR3CONR3R4, COR3, OCOR3 , $CO_2R3$, CON3R4, OCONR3R4, (alkylène en $C_1$-$C_6$)-OR3, (alkylène en $C_1$-$C_6$)-NR3R4, (alkylène en $C_1$-$C_6$)-$NR3SO_2R4$, (alkylène en $C_1$-$C_6$)-SR3, alkylène-S(O)R3, alkylène-$S(O)_2R3$, alkylène-$S(O)_2NR3R4$, (alkylène en $C_1$-$C_6$)-COR3, (alkylène en $C_1$-$C_6$)-$CO_2R3$, (alkylène en $C_1$-$C_6$)-CONR3R4, SR3, SOR3, $SO_2R3$, $SO_2NR3R4$, $NR3SO_2R4$, (alkylène en $C_1$-$C_6$) - (cycloalkyle en $C_3$-$C_{10}$), (alkylène en $C_1$-$C_6$)- (aryle en $C_6$-$C_{10}$), (alkylène en $C_1$-$C_6$) -hétérocyclyle, cycloalkyle en $C_3$-$C_{10}$, aryle en $C_6$-$C_{10}$, qui peut être une ou plusieurs fois substitué par un ou des substituants F, Cl, Br, I, CN, OH, $-CF_3$, alkyle en $C_1$-$C_6$, OR3, NR3R4, COR3, CO2R3, CONR3R4 ; ou hétérocyclyle, qui peut être une ou plusieurs fois substitué par un ou des substituants F, Cl, Br, CN, $NO_2$, OH, $-CF_3$, alkyle en $C_1$-$C_6$, OR3, NR3R4, COR3, CO2R3, CONR3R4 ;
R3 et R4 représentent chacun indépendamment de l'autre H, un groupe alkyle en $C_1$-$C_6$, $-CF_3$, cycloalkyle en $C_3$-$C_{10}$, aryle en $C_6$-$C_{10}$, hétérocyclyle, (alkylène en $C_1$-$C_6$)-CONR5R6 , (alkylène en $C_1$-$C_6$)-COOR5, (alkylène en $C_1$-$C_6$)-alkylène-COR5, (alkylène en $C_1$-$C_6$)-OR5, (alkylène en $C_1$-$C_6$)-NR5R6, (alkylène en $C_1$-$C_6$)-SR5, (alkylène en $C_1$-$C_6$)-S(O)R5, (alkylène en $C_1$-$C_6$)-$S(O)_2R5$, (alkylène en $C_1$-$C_4$)-(aryle en $C_6$-$C_{10}$) ou (alkylène en $C_1$-$C_4$) -hétérocyclyle ; R5 et R6 représentent chacun indépendamment de l'autre H, un groupe alkyle en $C_1$-$C_6$, cycloalkyle en $C_3$-$C_{10}$, (alkylène en $C_1$-$C_6$)-(aryle en $C_6$-$C_{10}$), -(aryle en $C_6$-$C_{10}$), hétérocyclyle, (alkylène en $C_1$-$C_6$)-(hétérocyclyle en $C_3$-$C_{10}$);
X est S ;
ainsi que leurs sels physiologiquement tolérés.

**5.** Composés de formule I selon l'une ou plusieurs des revendications 1 à 4, **caractérisés en ce que**, dans cette formule,

R1 et H ;
R2 est un groupe alkyle en $C_1$-$C_{10}$, cycloalkyle en $C_3$-$C_{10}$, phényle, (alkylène en $C_1$-$C_6$)-phényle, un radical pyrrolidino, pipéridino, hexaméthylène-imino, morpholino, pipérazino, thiomorpholino ou homopipérazino ;
X est S ;
ainsi que leurs sels physiologiquement tolérés.

**6.** Médicament contenant un ou plusieurs des composés selon l'une ou plusieurs des revendications 1 à 5.

**7.** Médicament contenant un ou plusieurs des composés selon l'une ou plusieurs des revendications 1 à 5 et au moins un autre principe actif.

**8.** Médicament selon la revendication 6, **caractérisé en ce qu'**il contient en tant qu'autre principe actif un ou plusieurs antibiotiques, principes actifs hypoglycémiques, inhibiteurs de la HMGCoA-réductase, inhibiteurs de la résorption du cholestérol, agonistes du PPAR gamma, agonistes du PPAR alpha, agonistes du PPAR alpha/gamma, inhibiteurs du MTP, inhibiteurs de la résorption des acides biliaires, inhibiteurs du CETP, adsorbants polymères des acides biliaires, inducteurs des récepteurs des LDL, inhibiteurs de l'ACAT, antioxydants, inhibiteurs de la lipoprotéine-lipase, inhibiteurs de l'ATP-citrate-lyase, inhibiteurs de la squalène synthétase, antagonistes de la lipoprotéine (a), inhibiteurs de la lipase, insulines, sulfonylurées, biguanides, méglitinides, thiazolidinediones, inhibiteurs de l'α-glucosidase, principes actifs agissant sur le canal calcique ATP-dépendant des cellules bêta, agonistes du CART, agonistes du NPY, agonistes du MC4, agonistes de l'orexine, antagonistes du récepteur des cannabinoïdes 1, agonistes du H3, agonistes du TNF, agonistes du CRF, antagonistes du CRF BP, agonistes de l'urocortine, agonistes des β3, agonistes de la MSH (hormone mélanostimulante), agonistes du CCK, agonistes de la recapture de la sérotonine, composés en mélange de la sérotonine et noradrénergiques, agonistes du 5HT, agonistes de la bombésine, antagonistes de la galanine, hormones de croissance, composés libérant des hormones de croissance, agonistes du TRH, modulateurs de la protéine 2 ou 3 de découplage, agonistes de la leptine, agonistes du DA (bromocriptine, doprexine), inhibiteurs de la lipase/amylase, modulateurs des PPAR, antagonistes des récepteurs du cannabinoïde 1, modulateurs du RXR ou agonistes du TR-β ou amphétamines.

**9.** Utilisation des composés selon l'une ou plusieurs des revendications 1 à 5 pour fabriquer un médicament hypoglycémiant.

**10.** Utilisation des composés selon l'une ou plusieurs des revendications 1 à 5 pour fabriquer un médicament destiné au traitement du diabète de type 2.

**11.** Utilisation des composés selon l'une ou plusieurs des revendications 1 à 5 pour fabriquer un médicament destiné au traitement des troubles du métabolisme des lipides et des glucides.

**12.** Utilisation des composés selon l'une ou plusieurs des revendications 1 à 5 pour fabriquer un médicament destiné au traitement des symptômes artériosclérotiques.

**13.** Utilisation des composés selon l'une ou plusieurs des revendications 1 à 5 pour fabriquer un médicament destiné au traitement de l'insulinorésistance.

**14.** Procédé de fabrication d'un médicament contenant un ou plusieurs des composés selon l'une ou plusieurs des revendications 1 à 5, **caractérisé en ce qu'**on mélange le principe actif à un support acceptable d'un point de vue pharmaceutique, et on met ce mélange sous une forme convenant à l'administration.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9931507 A
- US 6221633 B
- WO 9726265 A
- WO 9903861 A
- WO 0104156 A
- WO 0034331 A
- WO 0034332 A
- WO 9111457 A
- US 6380357 B
- WO 0250027 A
- WO 04007455 A
- US 0011833 W
- US 0011490 W
- DE 10142734
- US 6245744 B
- US 6221897 B
- US 6342512 B
- WO 9741097 A
- WO 0191752 A
- WO 0228346 A
- WO 0063208 A
- WO 0066585 A
- WO 0183451 A
- WO 9915525 A
- WO 0071549 A
- WO 0109111 A
- WO 0185695 A
- EP 0462884 A
- WO 0040569 A
- WO 0078312 A


### In der Beschreibung aufgeführte Nicht-Patentliteratur


- **H. OKADA et al.** *Chem. Pharm. Bull.,* 1994, vol. 42, 57-61
- *Heterocycles,* 1992, vol. 34 (5), 903-906
- *Synlett,* 1992, 249-251
- *Pharmaceutical Research,* 1986, vol. 2 (6), 318
- USP Dictionary of USAN and International Drug Names, US Pharmacopeia. 2001
- **ASAKAWA, A et al.** Cocaine-amphetamine-regulated transcript influences energy metabolism, anxiety and gastric emptying in mice. *M.:Hormone and Metabolic Research,* 2001, vol. 33 (9), 554-558
- **B. LEE ; DANIEL W. ; LEINUNG, MATTHEW C. ; ROZHAVSKAYA-ARENA ; MARINA ; GRASSO, PATRICIA.** Leptin agonists as a potential approach to the treatment of obesity. *Drugs of the Future,* 2001, vol. 26 (9), 873-881
- **SALVADOR, JAVIER ; GOMEZ-AMBROSI, JAVIER ; FRUHBECK, GEMA.** Perspectives in the therapeutic use of leptin. *Expert Opinion on Pharmacotherapy,* 2001, vol. 2 (10), 1615-1622
- **ZUNFT H J et al.** Carob pulp preparation for treatment of hypercholesterolemia. *ADVANCES IN THERAPY,* September 2001, vol. 18 (5), 230-6
- **DIXON, M. ; WEBB, E.C.** Enzymes. Academic Press, 1979, 47-206
- **LEATHERBARROW, R.J.** GraFit Version 3.0. Erithacus Software Ltd, 1992